(19) Europäisches Patentamt
European Patent Office
Office européen des brevets

(11) EP 4 335 457 A2

(12) **EUROPEAN PATENT APPLICATION**

(43) Date of publication:
**13.03.2024 Bulletin 2024/11**

(21) Application number: **23208264.4**

(22) Date of filing: **10.11.2021**

(51) International Patent Classification (IPC):
***A61K 47/64*** *(2017.01)*

(52) Cooperative Patent Classification (CPC):
**C12N 15/88; C12N 15/87;** C12N 2740/10051

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.11.2020 GB 202017725**

(62) Document number(s) of the earlier application(s) in accordance with Art. 76 EPC:
**21815257.7 / 4 244 367**

(71) Applicant: **Oxford BioMedica (UK) Limited**
**Oxford, Oxfordshire OX4 6LT (GB)**

(72) Inventors:
- **GOODYEAR, Oliver**
  **Oxford OX4 6LT (GB)**
- **DAVIES, Lee**
  **Oxford OX4 6LT (GB)**
- **KINGWOOD, Mollie**
  **Oxford OX4 6LT (GB)**
- **SANCHES, Rui**
  **Oxford OX4 6LT (GB)**
- **LAMONT, Ciaran**
  **Oxford OX4 6LT (GB)**

(74) Representative: **D Young & Co LLP**
**120 Holborn**
**London EC1N 2DY (GB)**

Remarks:
•This application was filed on 07.11.2023 as a divisional application to the application mentioned under INID code 62.
•Claims filed after the date of filing of the application / after the date of receipt of the divisional application (Rule 68(4) EPC).

(54) **PREPARATION OF A SOLUTION OF POLYMER/NUCLEIC ACID COMPLEXES**

(57) The invention relates to the preparation of a solution of polymer/nucleic acid complexes, and the use of such a solution in methods for the transfection of cells.

EP 4 335 457 A2

**Description**

FIELD OF THE INVENTION

The invention relates to the preparation of a solution of polymer/nucleic acid complexes, and the use of such a solution in methods for the transfection of cells.

BACKGROUND TO THE INVENTION

Transfection is the process of deliberately introducing nucleic acids into eukaryotic cells. There are many situations in which it may be desirable or advantageous to introduce various types of exogenous nucleic acids into eukaryotic cells. Exogenous nucleic acids commonly used are plasmid DNA, RNA, siRNA and oligonucleotides. Once delivered into cells, nucleic acids modulate gene expression by driving overexpression or silencing of a gene of interest.

Gene overexpression is an indispensable tool for several applications, from understanding the role of gene of interest (gene studies, high-throughput screening), to the production of biologics such as antibodies (protein production) and recombinant viral particles, particularly for therapeutic purposes (virus production e.g. for gene & cell therapy).

Gene silencing is a method used to prevent expression of a gene of interest. The expression of a gene can be partially reduced (gene knockdown) or completely blocked (gene knockout). Because any gene can potentially be targeted, gene silencing is a prevalent technique used to develop gene-based therapies to address monogenic pathologies, cancer and in immunotherapy strategies. It can be seen that transfection has many utilities in a number of different applications. Of particular interest is transfection of cells with expression vectors, which is widely used in the production of biological agents including recombinant proteins and viral vectors. For example, common methods of viral vector manufacture include the transfection of primary cells or mammalian/insect cell lines with vector DNA components, followed by a limited incubation period and then harvest of crude vector from culture media and/or cells (Merten, O-W. et al., 2014, Pharmaceutical Bioprocessing, 2:183-203). The efficiency of lentiviral vector manufacturing is typically affected by several factors at the 'upstream phase', including [1] viral serotype/pseudotype employed, [2] transgenic sequence composition and size, [3] media composition/gassing/pH, [4] transfection reagent/process, [5] chemical induction and vector harvest timings, [6] cell fragility/viability, [7] bioreactor shear-forces and [8] impurities. Clearly there are other factors to consider during the 'downstream' purification/concentration phase (Merten, O-W. et al., 2014, Pharmaceutical Bioprocessing, 2:237-251).

As the successes of the viral vector approaches in clinical trials begin to build towards regulatory approval and commercialisation, attention has focused on the emerging bottleneck in mass production of good manufacturing practice (GMP) grade vector material (Van der Loo JCM, Wright JF., 2016, Human Molecular Genetics, 25(R1):R42-R52). A similar bottleneck exists in the production of GMP grade recombinant proteins.

A way to overcome this challenge is to find new ways to maximise transfection efficiency during production of viral vector or recombinant protein. Thus, there is a need in the art to provide alternative and improved transfection methods for use in the production of e.g. viral vectors and other biological agents, which help to address the known issues associated with the mass production of GMP grade material, such as GMP grade vector material.

SUMMARY OF THE INVENTION

The present inventors have shown that polymer/nucleic acid complexes are only stable for a short period of time in the presence of salt before there is a negative effect on transfection efficiency. This limits the use of cationic polymers in mass production of biological agents because the optimum incubation period is too short to be practically possible at large scale GMP production.

The present invention provides an improved method for cell transfection. In this regard, the present inventors have surprisingly found that polymer/nucleic acid complexes prepared by mixing the nucleic acid and cationic polymer in a substantially salt-free aqueous solution (e.g. water), followed by the addition of salt to induce polymer/nucleic acid complex maturation, elongates the time for optimum polymer/nucleic acid complex maturation and provides high transfection efficiency. Thus, an optimum concentration of salt can be used to elongate the time for optimal polymer/nucleic acid complex maturation (i.e. the incubation time). The present inventors have further surprisingly found that the growth of the polymer/nucleic acid complexes can be curtailed and the complexes stabilised by dilution of the solution of polymer/nucleic acid complexes (thereby diluting the salt) to further extend the incubation time. Hence, the method described herein is advantageous as it provides the ability to control the initiation and/or termination of polymer/nucleic acid complex maturation. This in turn is highly advantageous for the mass production of GMP grade material, as it removes time constraints as an issue for the transfection process, i.e. improves manufacturing process flexibility.

The present inventors have surprisingly found that the ability to control the initiation and/or termination of polymer/nucleic acid complex maturation, combined with the reduced cell toxicity, is advantageous as it provides the

ability to scale the preparation of the polymer/nucleic acid complexes with the cell number during transfection. This in turn is highly advantageous for the mass production of GMP grade material, as it enables the use of higher cell densities (e.g. cell densities achieved using perfusion culture) for the transfection process, i.e. improves manufacturing process capacity and yield.

The present invention relates to such process for preparing a solution of polymer/nucleic acid complexes and use of the solution for transfection of cells, for example in the production of lentiviral vector.

In one aspect, the invention provides an *in vitro* method for producing a solution of polymer/nucleic acid complexes comprising the steps of:

a) mixing a nucleic acid and a cationic polymer in a substantially salt free aqueous solution;
b) adding a salt to the mixture produced in step a) to form a salt solution;
c) optionally incubating the salt solution produced in step b); and/or
d) optionally diluting the salt solution produced in step b) or diluting the salt solution following incubation of step c),

wherein said salt is not a valproic acid salt, isobutyric acid salt or isovaleric acid salt.

In a further aspect, the invention provides an *in vitro* method for producing a solution of polymer/nucleic acid complexes comprising the steps of:

a) mixing a nucleic acid and a cationic polymer in a substantially salt free aqueous solution;
b) adding a salt to the mixture produced in step a) to form a salt solution;
c) optionally incubating the salt solution produced in step b); and/or
d) optionally diluting the salt solution produced in step b) or diluting the salt solution following incubation of step c),

wherein said salt is added only prior to contacting the solution of polymer/nucleic acid complexes with a cell.

In a further aspect, the invention provides an *in vitro* method for producing a solution of polymer/nucleic acid complexes comprising the steps of:

a) mixing a nucleic acid and a cationic polymer in a substantially salt free aqueous solution;
b) adding a salt to the mixture produced in step a) to form a salt solution;
c) optionally incubating the salt solution produced in step b); and/or
d) optionally diluting the salt solution produced in step b) or diluting the salt solution following incubation of step c),

wherein the final salt concentration in step b) is between about 10 mM to about 100 mM.

In a further aspect, the invention provides an *in vitro* method for producing a solution of polymer/nucleic acid complexes comprising the steps of:

a) mixing a nucleic acid and a cationic polymer in a substantially salt free aqueous solution;
b) adding a salt to the mixture produced in step a) to form a salt solution;
c) optionally incubating the salt solution produced in step b); and/or
d) optionally diluting the salt solution produced in step b) or diluting the salt solution following incubation of step c),

wherein said salt has a degree of dissociation of at least 0.95 in the substantially salt free aqueous solution.

In a further aspect, the invention provides an *in vitro* method for producing a solution of polymer/nucleic acid complexes comprising the steps of:

a) mixing a nucleic acid and a cationic polymer in a substantially salt free aqueous solution;
b) adding a salt to the mixture produced in step a) to form a salt solution;
c) optionally incubating the salt solution produced in step b); and/or
d) diluting the salt solution produced in step b) or diluting the salt solution following incubation of step c).

In a further aspect, the invention provides an *in vitro* method for producing a solution of polymer/nucleic acid complexes comprising the steps of:

a) mixing a nucleic acid and a cationic polymer in a substantially salt free aqueous solution;
b) adding a salt to the mixture produced in step a) to form a salt solution;
c) incubating the salt solution produced in step b); and/or
d) optionally diluting the salt solution produced in step b) or diluting the salt solution following incubation of step c).

In a further aspect, the invention provides a solution of polymer/nucleic acid complexes obtained or obtainable by the methods of the invention.

In a further aspect, the invention provides a solution comprising polymer/nucleic acid complexes and a salt, wherein the salt concentration is between about 10 mM to about 100 mM.

In a further aspect, the invention provides the use of a solution of polymer/nucleic acid complexes of the invention for the transfection of the nucleic acid into cells.

In a further aspect, the invention provides the use of a solution of polymer/nucleic acid complexes of the invention in a method for the production of a retroviral vector.

In some embodiments, the nucleic acid is selected from DNA, RNA, oligonucleotide molecule, and mixtures thereof.

In some embodiments, the nucleic acid is DNA, preferably plasmid DNA.

In some embodiments, the cationic polymer is a polymer-based transfection reagent.

In some embodiments, the cationic polymer is selected from polyethylenimine (PEI), a dendrimer, DEAE-dextran, polypropyleneimine (PPI), chitosan [poly-(β-1/4)-2-amino-2-deoxy-D-glucopyranose], poly-L-lysine (PLL), poly(lactic-co-glycolic acid) (PLGA), poly(caprolactone) (PCL), and a derivative thereof.

In some embodiments, the cationic polymer is PEI or a derivative thereof, preferably selected from linear PEI, branched PEI, PEGylated PEI, JetPEI, PEIPro®, PEI MAX and PTG1+.

In some embodiments, the salt is selected from a sodium salt, a magnesium salt, a potassium salt, a calcium salt, a phosphate salt and a mixture thereof.

In some embodiments, the salt is phosphate buffered saline (PBS).

In some embodiments, the salt is PBS and PBS is added in step b) to a final concentration of between about 0.1X PBS to about 1.0X PBS, preferably wherein the PBS is added to a final concentration of between about 0.1X PBS to about 0.3X PBS.

In some embodiments, the salt is added in step b) to a final concentration of between about 10 mM to about 100 mM, preferably wherein the salt is added in step b) to a final concentration of between about 20 mM to about 100 mM.

In some embodiments, step a) comprises mixing a plurality of nucleic acid molecules and a plurality of cationic polymer molecules in a substantially salt free aqueous solution.

In some embodiments, the method comprises the step of incubating the salt solution produced in step b).

In some embodiments, the salt solution is incubated for about 1 minute to up to about 2 hours, preferably wherein the salt solution is incubated for about 20 minutes to about 90 minutes.

In some embodiments, the salt solution is incubated for about 60 minutes.

In some embodiments, the method comprises the step of diluting the salt solution produced in step b) or the salt solution following incubation of step c).

In some embodiments, the salt is PBS and the salt solution is diluted to a final concentration of between about 0.05X PBS to about 0.15X PBS, preferably wherein the salt solution is diluted to a final concentration of about 0.1X PBS.

In some embodiments, the nucleic acid encodes a retroviral vector component.

In some embodiments, the retroviral vector is a replication defective retroviral vector.

In some embodiments, the retroviral vector is a lentiviral vector, preferably wherein the lentiviral vector is HIV-1, HIV-2, SIV, FIV, BIV, EIAV, CAEV or Visna.

In some embodiments, the vector component is selected from:

a) the RNA genome of the lentiviral vector;
b) env or a functional substitute thereof;
c) gag-pol or a functional substitute thereof; and/or
d) rev or functional substitute thereof.

In some embodiments, the method further comprises the steps of:

e) optionally culturing a mammalian cell;
f) transfecting the mammalian cell using the solution of polymer/nucleic acid complexes obtained by the method according to any one of the preceding claims;
g) optionally introducing a nucleic acid that is different to the nucleic acid of the solution of polymer/nucleic acid complexes into the mammalian cell;
h) optionally selecting for a mammalian cell which has the nucleic acid(s) integrated within its genome;
i) optionally culturing the mammalian cell under conditions in which the nucleic acid(s) is (are) expressed;
j) optionally culturing the mammalian cell under conditions in which the retroviral vector is produced; and
k) optionally isolating the retroviral vector.

In some embodiments, the method comprises the step of culturing a mammalian cell.

In some embodiments culturing is by perfusion culture.

In some embodiments, the step of culturing a mammalian cell in a perfusion culture is performed for about 10 hours to about 96 hours.

In some embodiments, the method further comprises the step of inoculating a cell culture vessel (e.g. bioreactor) with a mammalian cell prior to the step of culturing a mammalian cell.

In some embodiments, the method further comprises the step of performing a rapid media exchange after the step of inoculating the cell and prior to the step of transfecting the cell. Suitably, the step of culturing a mammalian cell comprises at least one rapid media exchange.

In some embodiments, the method comprises the step of culturing the mammalian cell under conditions in which the nucleic acid(s) is expressed.

In some embodiments, the method comprises the step of culturing the mammalian cell under conditions in which the retroviral vector is produced.

In some embodiments, the method comprises the step of isolating the retroviral vector.

In some embodiments, the mammalian cells are HEK293T cells.

In some embodiments, the mammalian cells are suspension-adapted mammalian cells.

In some embodiments, the transfection, introduction and/or culturing step(s) is (are) performed in suspension in a serum-free medium.

In some embodiments, the transfection is a transient transfection.

In some embodiments, the transfection, introduction and/or culturing step(s) is (are) carried out in a volume of at least 50 L, preferably wherein the transfection, introduction and culturing steps are carried out in a volume of at least 50 L.

In some embodiments, the nucleic acid that is different to the nucleic acid of the solution of polymer/nucleic acid complexes is introduced into the mammalian cell by transfection or by electroporation.

In some embodiments, the nucleic acid that is different to the nucleic acid of the solution of polymer/nucleic acid complexes encodes any viral vector components selected from:

a) the RNA genome of the lentiviral vector;
b) env or a functional substitute thereof;
c) gag-pol or a functional substitute thereof; and/or
d) rev or functional substitute thereof;

that are not encoded by the nucleic acid of the solution of polymer/nucleic acid complexes.

In a further aspect, the invention provides a method for producing a retroviral vector comprising the steps of:

a) optionally culturing a mammalian cell;
b) transfecting a mammalian cell using solution of polymer/nucleic acid complexes of the invention or a solution of polymer/nucleic acid complexes of the invention;
c) optionally introducing at least one nucleic acid that is different to the nucleic acid of the solution of polymer/nucleic acid complexes into the mammalian cell;
d) optionally selecting for a mammalian cell which has the nucleic acid sequences encoding the viral vector components integrated within its genome; and
e) culturing the mammalian cell under conditions in which the retroviral vector is produced.

In some embodiments culturing is by perfusion culture

In some embodiments, the step of culturing the cell in a perfusion culture is performed for about 10 hours to about 96 hours.

In some embodiments, the method further comprises the step of isolating the retroviral vector.

In some embodiments, the retroviral vector is a replication defective retroviral vector.

In some embodiments, the retroviral vector is a lentiviral vector, preferably wherein the lentiviral vector is HIV-1, HIV-2, SIV, FIV, BIV, EIAV, CAEV or Visna.

In some embodiments, the mammalian cell is a HEK293T cell.

In some embodiments, the mammalian cell is a suspension-adapted cell.

In some embodiments, the method is performed in suspension in a serum-free medium.

In some embodiments, step a), step b), step c) and/or step e) is carried out in a volume of at least 50 L, preferably wherein step a), step b), step c) and step e) are carried out in a volume of at least 50 L.

In some embodiments, the nucleic acid is introduced into the cell by transfection or by electroporation in step c).

In some embodiments, the transfection is a transient transfection.

In some embodiments, the at least one nucleic acid optionally introduced into the mammalian cell in step b)

encodes any lentiviral vector components selected from the group consisting of:

(i) the RNA genome of the lentiviral vector;
(ii) env or a functional substitute thereof;
(iii) gag-pol or a functional substitute thereof; and/or
(iv) rev or functional substitute thereof;

that are not encoded by the nucleic acid of the solution of polymer/nucleic acid complexes.

In a further aspect, the invention provides a method for transfecting a mammalian cell comprising the steps of:

a) culturing a mammalian cell in a perfusion culture; and

b) transfecting the mammalian cell using a solution of polymer/nucleic acid complexes as described herein.

In some embodiments, the step of culturing a mammalian cell in a perfusion culture is performed for about 10 hours to about 96 hours.

Suitably, the mammalian cell is a mammalian cell as described herein.

In some embodiments, the method is performed in suspension in a serum-free medium.

In some embodiments, step a) and/or step b) is carried out in a volume of at least 50 L, preferably wherein step a) and step b) are carried out in a volume of at least 50 L.

In some embodiments, the transfection is a transient transfection.

BRIEF DESCRIPTION OF THE FIGURES

**Figure 1:** Functional titre (TU/mL) results from a shake flask time course study where individual shake flasks were transfected using PEIPro® transfection mix incubated at different time intervals. The incubation period of DNA/PEIPro® complexes significantly impacts subsequent transfection and lentiviral vector production. The data shows that the optimal incubation time that yields the highest functional titre is 3 minutes and this rapidly decreases if the incubation time is extended to 15 minutes or 30 minutes.

**Figure 2:** The figure shows that the kinetics of complex formation can be modified by preparing the transfection mix in water and spiking with varying PBS concentrations - DNA/PEIpro complexes were prepared in a salt-free aqueous solution ($H_2O$) and complex growth was initiated with a PBS spike. (A) Optimal titre was achieved after a 3-minute incubation when transfection complex was spiked with 1X PBS. (B) The incubation time can be extended if the transfection complex is spiked with a lower concentration of PBS - 0.2X PBS extends the optimal incubation time to approximately 25 minutes. N.B. as a negative control the complex was prepared in water and then used to transfect the cells without spiking with PBS. No vector was produced demonstrating that PBS is required to initiate complex growth.

**Figure 3:** Transfection efficiency is stable following dilution of the complexes to reduce the salt concentration (and arrest complex growth) for up to 12 hours and potentially up to 1 week. The transfection mix was spiked with 0.2 X PBS to initiate complex growth and then incubated for 30 minutes to allow for the optimal size of the complex to be reached. The transfection mix was then diluted using salt-free aqueous solution (e.g. $H_2O$) so that the final concentration of PBS was 0.1 X. The transfection mix was then stored at 4°C for up to 1 week. Aliquots of the transfection mix were then used to generate GFP lentivirus and the resultant functional titre was measured. The graph shows that after 12 hours' incubation, the transfection mix was still efficient for the generation of lentiviral vectors and yielded a comparable functional titre to the "fresh" transfection mix.

**Figure 4:** DLS analysis showing that when DNA:PEIpro complexes are prepared in $H_2O$, complex growth is not observed. This correlates with the data presented in figure 2B which demonstrated that complexes prepared in water and not spiked with PBS did not lead to vector production. This suggests that complex's within the size range of 80-86 nm are too small to lead to successful transfection.

**Figure 5:** DNA/PEIpro complexes were prepared in water and particle growth was initiated by addition of PBS (0.2X). Complexes were incubated for 25 min as this corresponded to the optimal time to achieve the highest functional titre (Figure 2B). Complexes were then diluted 2-fold to achieve a final PBS concentration of 0.1X. Complex size

was then measured over time for a further 45min. The DLS measurements show that dilution of the transfection mix by 2 fold was found to halt the growth of the complexes and the size remained between 600-800nm. Correlating this data with data displayed in figure 2 and 3, suggests that the optimal complex size that achieves the highest titre, is between 600-800nm.

**Figure 6:** When the transfection mix is spiked with PBS at different concentrations, the rate at which the complex grows to the preferred size for transfection varies depending on the concentration of PBS added. If the concentration of PBS is too low (≤0.1x PBS), then complex growth is not initiated.

**Figure 7**: Any salt can be used to initiate complex growth. The faster the rate of complex growth and the lower the concentration of salt required to trigger complex growth. Graph (A) and Table 2 shows the impact of spiking the transfection mix with five different concentrations of NaCl (10mM; 20mM; 50mM; 80mM and 100mM). Only concentrations of 80mM and 100mM initiated significant complex growth. Graph (B) and Table 3 shows the impact of spiking the transfection mix with five different concentrations of $MgCl_2$ (10mM; 20mM; 50mM; 80mM; 100mM).

**Figure 8**: The figure shows the functional titre of a therapeutic vector following production at the 5L bioreactor scale. This result shows that the "aqPEIPro" transfection method is scalable and demonstrates equivalent lentiviral vector titres to those achieved using a cationic lipid based transfection method. n = 2 for the "aqPEIPro" transfection method and n = 4 for the cationic lipid based transfection method.

DETAILED DESCRIPTION OF THE INVENTION

***In vitro* method for producing a solution of polymer/nucleic acid complexes**

The present invention provides an *in vitro* method for producing a solution of polymer/nucleic acid complexes comprising the steps of:

a) mixing a nucleic acid and a cationic polymer in a substantially salt-free aqueous solution;
b) adding a salt to the mixture produced in step a) to form a salt solution;
c) optionally incubating the salt solution produced in step b); and/or
d) optionally diluting the salt solution produced in step b) or diluting the salt solution following incubation of step c).

Transfection of cells with exogenous nucleic acids is well-known in the art and can be performed using various viral and non-viral transfection reagents. Transfection overcomes the inherent challenge of introducing negatively charged molecules (e.g. phosphate backbones of DNA and RNA) into cells with a negatively charged membrane. Transfection reagents can be split up into three classes: chemical reagents, cationic lipids and physical methods.

Chemical reagents include DEAE-dextran, a cationic polymer, which was one of the first chemical reagents used to transfect cultured mammalian cells (Vaheri and Pagano, 1965; McCutchan and Pagano, 1968). Other synthetic cationic polymers have been used to transfer DNA into cells, including polybrene (Kawai and Nishizawa, 1984, polyethyleneimine (Boussif et al. 1995) and dendrimers (Haensler and Szoka, 1993; Kukowska-Latallo et al. 1996). Calcium phosphate co-precipitation as a transfection method was introduced in the early 1970's (Graham and van der Eb, 1973) and represents another chemical transfection reagent.

Cationic lipids, such as Lipofectamine, represent one of the most popular methods for introducing foreign genetic material into cells. The term "liposome" refers to lipid bilayers that form colloidal particles in an aqueous medium (Sessa and Weissmann, 1968). Artificial liposomes were first used to deliver DNA into cells in 1980 (Fraley et *al.* 1980). The next advance in liposomal vehicles was the development of synthetic cationic lipids (Felgner *et* al. 1987). The cationic head group of the lipid compound associates with negatively charged phosphates on the nucleic acid. Liposome-mediated delivery offers advantages such as relatively high efficiency of gene transfer, the ability to transfect certain cell types that are resistant to calcium phosphate or DEAE-dextran, *in vitro* and *in vivo* applications, successful delivery of DNA of all sizes from oligonucleotides to yeast artificial chromosomes, delivery of RNA, and delivery of protein (reviewed in Kim and Eberwine, 2010; Stewart *et al.* 2016). Nonliposomal reagents offer an alternative to liposome-mediated transfection methods. Lipid nanoparticles represent an extension of this transfection method that is especially suited for the delivery of small-molecule drugs in clinical research and therapeutic applications (reviewed in Cullis and Hope, 2017).

Physical methods for gene transfer were developed in the early 1980's and initially involved the direct microinjection into cultured cells or nuclei (Cappechi, 1980). Recently microinjection has regained popularity in gene-editing applications and has been used to deliver CRISPR-Cas9 DNA into zygote pronuclei to create knockout pigs (Chuang et al. 2017). Electroporation represents another physical method and was first reported for gene transfer studies in

mouse cells (Wong and Neumann, 1982). The mechanism is based on the use of an electrical pulse to perturb the cell membrane and form transient pores that allow passage of nucleic acids into the cells (Shigekawa and Dower, 1988).

The most widely used non-viral transfection reagents include lipid-based reagents such as Lipofectamine and cationic polymers such as PEI. A number of transfection methods are known in the art (see, e.g. Graham et al. (1973), Virology, 52: 456; Sambrook et al. (1989) Molecular Cloning, a laboratory manual, Cold Spring Harbor Laboratories, New York; Davis et al. (1986) Basic Methods in Molecular Biology, Elsevier, and Chu et al. (1981), Gene, 13:197). Such techniques may be used to introduce one or more exogenous nucleic acids into suitable host cells.

As used herein, the terms "transfect" and "transduce" refer to the introduction of an exogenous nucleic acid (e.g. a DNA plasmid) into a host cell. Thus, the terms "transfection" and "transduction" refer to the process of introducing an exogenous nucleic acid (e.g. a DNA plasmid) into a host cell. A cell is "transduced" or "transfected" when exogenous nucleic acid has been introduced inside the cell membrane. The exogenous nucleic acid which is introduced into the host cell may stably integrate into the genome of the host cell or be extrachromosomal. The integrated nucleic acid may be maintained in that cell and inherited by progeny cells. Alternatively, the exogenous nucleic acid which is introduced into the host cell may be present in the host cell transiently.

Accordingly, as used herein, the term "transduced/transfected cell" refers to a cell into which an exogenous nucleic acid has been introduced, or a progeny thereof in which an exogenous nucleic acid is present. A transduced/transfected cell can be cultured (i.e. propagated) and the introduced exogenous nucleic acid transcribed and/or protein expressed.

As used herein, the term "transfection reagent" refers to a viral or non-viral agent that facilitates cell transduction/transfection with a nucleic acid.

As used herein, the terms "polymer/nucleic acid complexes", "polycation/nucleic acid complexes" and "complexes" refer to aggregates of cationic polymer and nucleic acids. Such complexes are also known in the art as polymer-condensed nucleic acid, and can be used to transfect a broad range of cells.

Mixing cationic polymers and nucleic acids under certain conditions and in the presence of ions results in the maturation of polymer/nucleic acid complexes due to the electrostatic interactions between the cationic polymer and nucleic acid. Hence, the present invention can employ any cationic polymer and any nucleic acid. Moreover, the present invention can be employed in the transfection of any exogenous nucleic acid to a cell during production of any biological agent, for example a recombinant protein or a lentiviral vector.

Transfection with a cationic polymer involves condensing of the nucleic acid and subsequent release of the exogenous nucleic acid into the cell. Thus, the transfection efficiency using cationic polymers is associated with nucleic acid binding and dissociation of the polymer.

Conventionally, transfection complexes (e.g. polymer/nucleic acid complexes) are generated with both the nucleic acid component and the transfection reagent (liposome/polymer) prepared by dilution in phosphate buffered saline (PBS) solution, sodium chloride (NaCl) solution or media. Once the components have been mixed there is a period of "maturation" of the transfection complexes (transfection reagent/nucleic acid complexes) which is usually associated with the physical growth of these complexes to an optimal size for transfection. Beyond this optimum complex size, the efficiency of cell transfection decreases because complexes that are too small do not deliver enough nucleic acid to cells and those that are too large impede endocytosis. The dynamic process of transfection complex maturation can be problematic in that the time taken for optimal size generation can be inappropriate for use in a large scale manufacturing environment. Often the time taken to physically mix large volumes of DNA and transfection reagent can exceed the optimal time for complex maturation and the time taken to deliver the complexes to a production bioreactor must also be factored into transfection protocols.

The present inventors have shown that polymer/nucleic acid complexes are only stable for a short period of time in the presence of salt before there is a negative effect on transfection efficiency. This has also been reported by others. For example, the manufacturer recommends that the incubation time of the cationic polymer PEIPro® with DNA should not exceed 30 minutes to ensure a good transfection efficiency (see Polyplus transfection PEIPro® DNA transfection kit for virus production protocol CPT115 vL (July 2020): https://fnkprddata.blob.core.windows.net/domestic/data/datasheet/PPU/115-01K.pdf). This limits the use of cationic polymers in mass production of biological agents, e.g. recombinant protein or lentiviral vector, because the optimum incubation period is too short to be practically possible at large scale GMP production, e.g. at the 200 L scale.

The present inventors surprisingly found that:

1. Mixing the polymer/nucleic acid complexes in a substantially salt-free solution (e.g. in water) followed by spiking with salt to induce polymer/nucleic acid complex maturation provides a solution of polymer/nucleic acid complexes that gives good transfection efficiency, and thereby good functional lentiviral vector titre. In other words, an optimum concentration of salt can be used to elongate the time for optimal polymer/nucleic acid complex maturation (i.e. the incubation time).
2. The growth of the polymer/nucleic acid complexes can be curtailed and the complexes stabilised by dilution of

the solution of polymer/nucleic acid complexes (thereby diluting the salt) to further extend the incubation time.

The method described herein is advantageous as it provides the ability to control both the initiation and termination of polymer/nucleic acid complex maturation. This in turn is highly advantageous at GMP scale, as it removes time constraints as an issue for the transfection process, i.e. improves manufacturing process flexibility.

Preparation of the polymer/nucleic acid complexes in a substantially salt-free aqueous solution (e.g. water) also offers advantages over the conventional processes. For example, it allows for preparation of polymer/nucleic acid complexes at higher concentrations than is possible when the complexes are prepared in the presence of salt (e.g. in media), which would significantly reduce the volume of the solution of polymer/nucleic acid complexes to be added to the bioreactors. This would also increase the total number of cells in the bioreactor at the transfection step which would be expected to increase productivity. Therefore, advantageously, the methods of the present invention may be used with a perfusion cell culture step prior to the transfection step. The use of perfusion cell culture may advantageously increase the cell density, i.e. the number of cells, in the bioreactor.

By way of further example, the present inventors have shown that, when mixed in a substantially salt-free aqueous solution (e.g. water), polymers and nucleic acids do not exhibit growth/aggregation of complexes and typically generate complexes of <100 nM in diameter. Hence, both the components and/or the mixture can be efficiently sterile filtered prior to complexation (i.e. the maturation of complexes) and subsequent addition during production processes. This would add an extremely valuable safety step in any manufacturing process as the sterility of the complexes could be ensured prior to addition to production processes (e.g. prior to addition to bioreactors).

By way of yet further example, the present inventors have also shown that, once matured in a substantially salt-free aqueous solution (e.g. water) and/or diluted after complex maturation, polymer/nucleic acid complexes are stable for days and indeed weeks. This allows preparation of complexes well in advance of the transfection step, greatly reducing the workload associated with this step during manufacture and increasing process flexibility. It also allows for small scale testing of the transfection efficiency of the complexes prior to use in manufacturing.

By way of yet further example, the present inventors have found that the control over the maturation of the polymer/nucleic acid complexes in a substantially salt-free aqueous solution (e.g. water) allows for the preparation of complexes proportionate to the number of cells to be transfected (i.e. scaling the concentration and/or volume of the preparation of polymer/nucleic acid complexes according to the number of cells to be transfected). In addition, the lack of toxicity associated with the polymer/nucleic acid complexes of the invention permits their use at an increased concentration compared to certain conventional transfection reagents (e.g. lipofectamine). This allows transfection of a greater total number of cells in the bioreactor which would be expected to increase productivity. This also allows preparation of the cells for transfection using perfusion culture, in order to increase the total number of cells in the bioreactor for the transfection step.

Thus, the method described herein is not only appropriate for use in large scale GMP production of material but also has advantages in such a production process. By "large scale" as used herein encompasses standard bioreactor sizes, for example 50L, 200L, 500L, 1000L and 10,000L.

The use of transfection enhancing agents to boost transfection performance and, for example, gene expression, is well-known in the art. Transfection enhancing agents include butyric acid, valproic acid, isobutyric acid and isovaleric acid, or salts thereof. The salt of butyric acid, valproic acid, isobutyric acid and isovaleric acid may be any salt, such as a sodium salt or a potassium salt (e.g. sodium butyrate). Transfection enhancing agents are typically added during culturing of the cells after the transfection step. However, such agents may be added prior to or during the transfection step, i.e. prior to or during contacting the transfection mix with the cells.

As used herein, the term "transfection enhancing agent" refers to a compound that increases cell transduction/transfection with a nucleic acid.

In some embodiments, said salt is not a transfection enhancing agent. In some embodiments, said salt is not a butyric acid salt, valproic acid salt, isobutyric acid salt or isovaleric acid salt. Preferably, said salt is not a valproic acid salt, isobutyric acid salt or isovaleric acid salt. Preferably, said salt is not sodium butyrate.

In some embodiments, the salt is not added during or after the transfection step, i.e. during or after contacting the transfection mix (for example, the solution of polymer/nucleic acid complexes) with a cell. In some preferred embodiments, the salt is added only prior to contacting the solution of polymer/nucleic acid complexes with a cell. Thus, the salt may be added to a mixture of cationic polymer and nucleic acid prepared in a substantially salt-free solution to initiate complex maturation and growth, providing a solution of polymer/nucleic acid complexes that can subsequently be used for transfection.

In some embodiments, the final concentration of salt is between about 10 mM to about 500 mM. In some preferred embodiments, the final concentration of salt is between about 50 mM to about 500 mM. In some embodiments, the salt is PBS and PBS is added to a final concentration between about 0.1X PBS to about 3X PBS.

In some embodiments, the final concentration of salt is between about 20 mM to about 100 mM. In some embodiments, the salt is PBS and PBS is added to a final concentration between about 0.15X PBS to about 0.3X PBS.

In some embodiments, the salt has a dissociation degree of at least 0.90 (suitably, at least 0.95, at least 0.96, at least 0.97, at least 0.98, at least 0.99 or 1) in the substantially salt-free solution.

As used herein, the term "dissociation degree" refers to the fraction of salt molecules that have dissociated into ions in the substantially salt-free solution. The dissociation degree can be calculated using methods known in the art (e.g. Dr Wolfgang Schärtl, Basic Physical Chemistry: A Complete Introduction on Bachelor of Science Level (1st Ed. 2014) p. 109, Bookboon, ISBN 978-87-403-0669-9).

The dissociation constant specifies the tendency of a substance $M_xN_y$ to reversibly dissociate (separate) in a solution (often aqueous) into smaller components M and N:

$$M_xN_{y(aq)} + H_2O_{(l)} \rightleftharpoons xM_{(aq)} + yN_{(aq)}$$

The dissociation constant is denoted $K_d$ and is calculated by

$$K_d = \frac{a_M^x \cdot a_N^y}{a_{M_xN_y} \cdot a_{H_2O}} \approx \frac{[M]^x[N]^y}{[M_xN_y](1)}$$

where $\alpha$ represents the activity of a species, and [M], [N], and $[M_xN_y]$ are the molar concentrations of the entities M, N, and $M_xN_y$ (https://chem.libretexts.org/Bookshelves/Physical_and_Theoretical_Chemistry_Textbook_Maps/Supplemental_Modules_(Physical_and_Theoretical_Chemistry)/Equilibria/Chemical_Equilibria/Dissociation_Constant). Because water is the solvent, and the solution is assumed to be dilute, the water is assumed to be pure, and the activity of pure water is defined as 1. The activities of the solutes are approximated with molarities. The dissociation constant is an immediate consequence of the law of mass action which describes equilibria in a more general way. The dissociation constant is also sometimes called ionization constant when applied to salts.

The dissociation degree is a fraction of original solute molecules that have dissociated. It is usually indicated by the Greek symbol $\alpha$. More accurately, degree of dissociation refers to the amount of solute dissociated into ions or radicals per mole. In case of very strong acids and bases, degree of dissociation will be close to 1. Less powerful acids and bases will have lesser degree of dissociation. There is a simple relationship between this parameter and the van't Hoff factor i. If the solute substance dissociates into *n* ions, then

$$i = 1 + \alpha(n - 1)$$

For instance, for the following dissociation

$$KCl \rightleftharpoons K^+ + Cl^-$$

as $n = 2$, we would have that $i = 1 + \alpha$ (Atkins P. and de Paula J. Physical Chemistry (8th ed. W.H.Freeman 2006) p.763, ISBN 978-0-7167-8759-4).

Accordingly, in a further aspect, the invention provides an *in vitro* method for producing a solution of polymer/nucleic acid complexes comprising the steps of:

a) mixing a nucleic acid and a cationic polymer in a substantially salt-free aqueous solution;
b) adding a salt to the mixture produced in step a) to form a salt solution;
c) optionally incubating the salt solution produced in step b); and/or
d) optionally diluting the salt solution produced in step b) or diluting the salt solution following incubation of step c),

wherein said salt is not a valproic acid salt, isobutyric acid salt or isovaleric acid salt.

In a further aspect, the invention provides an *in vitro* method for producing a solution of polymer/nucleic acid complexes comprising the steps of:

a) mixing a nucleic acid and a cationic polymer in a substantially salt-free aqueous solution;
b) adding a salt to the mixture produced in step a) to form a salt solution;

c) optionally incubating the salt solution produced in step b); and/or
d) optionally diluting the salt solution produced in step b) or diluting the salt solution following incubation of step c),

wherein said salt is added only prior to contacting the solution of polymer/nucleic acid complexes with a cell.

In a further aspect, the invention provides an *in vitro* method for producing a solution of polymer/nucleic acid complexes comprising the steps of:

a) mixing a nucleic acid and a cationic polymer in a substantially salt-free aqueous solution;
b) adding a salt to the mixture produced in step a) to form a salt solution;
c) optionally incubating the salt solution produced in step b); and/or
d) optionally diluting the salt solution produced in step b) or diluting the salt solution following incubation of step c),

wherein the final salt concentration in step b) is between about 10 mM to about 100 mM.

In a further aspect, the invention provides an *in vitro* method for producing a solution of polymer/nucleic acid complexes comprising the steps of:

a) mixing a nucleic acid and a cationic polymer in a substantially salt-free aqueous solution;
b) adding a salt to the mixture produced in step a) to form a salt solution;
c) optionally incubating the salt solution produced in step b); and/or
d) optionally diluting the salt solution produced in step b) or diluting the salt solution following incubation of step c),

wherein said salt has a degree of dissociation of at least 0.95 in the substantially salt-free aqueous solution.

In a further aspect, the invention provides an *in vitro* method for producing a solution of polymer/nucleic acid complexes comprising the steps of:

a) mixing a nucleic acid and a cationic polymer in a substantially salt-free aqueous solution;
b) adding a salt to the mixture produced in step a) to form a salt solution;
c) optionally incubating the salt solution produced in step b); and/or
d) diluting the salt solution produced in step b) or diluting the salt solution following incubation of step c).

In a further aspect, the invention provides an *in vitro* method for producing a solution of polymer/nucleic acid complexes comprising the steps of:

a) mixing a nucleic acid and a cationic polymer in a substantially salt-free aqueous solution;
b) adding a salt to the mixture produced in step a) to form a salt solution;
c) incubating the salt solution produced in step b); and/or
d) optionally diluting the salt solution produced in step b) or diluting the salt solution following incubation of step c).

In some embodiments, step a) comprises mixing a plurality of nucleic acid molecules and a plurality of cationic polymer molecules in a substantially salt-free aqueous solution.

As used herein, the term "substantially salt-free" means that the aqueous solution contains no or only trace amounts of salt. The salt content of the aqueous solution may, for example, be less than about 10 mM (suitably, less than about 7.5 mM, less than about 5 mM, less than about 2.5 mM, less than about 1 mM, less than about 0.5 mM or less than about 0.1 mM). An aqueous solution which is substantially salt-free may comprise less than about 1 mM salt, suitably, less than about 0.5 mM salt. The salt concentration of the substantially salt-free aqueous solution may be too low to initiate polymer/nucleic acid complex maturation, i.e. polymer/nucleic acid complex formation is not initiated in the aqueous solution. The salt concentration of an aqueous solution may be determined using methods known in the art, for example, using a salinity meter. Salt concentration is commonly measured using either the direct method using optical refractometer or is determined following the indirect method by means of a conductivity meter. A number of commercial options are available (https://www.pce-instruments.com/english/measuring-instruments/test-meters/salt-meter-kat_40093.htm).

Suitably, to work out the solubility of a solid in water the following procedure may be used:

1) Measure accurately 100 $cm^3$ of water and add to a beaker
2) Add small amounts of the solute until no more can dissolve
3) Record the mass of an evaporation dish
4) Filter the mixture so undissolved solid is left behind and the solution is in evaporating dish
5) Remove the water by heating or evaporation

6) Weigh the evaporating dish with the solute in it and calculate the mass of the solute that was dissolved (https://www.bbc.co.uk/bitesize/guides/z4s48min/revision/1).

In some preferred embodiments, step a) comprises mixing a nucleic acid and a cationic polymer in a salt-free aqueous solution.

In some preferred embodiments, the substantially salt-free aqueous solution is water. Thus, step a) may comprise mixing a nucleic acid and a cationic polymer in water.

As used herein, the term "nucleic acid" refers to all forms of nucleic acid, including DNA, RNA and oligonucleotides. Thus, nucleic acids include genomic DNA, cDNA, spliced or unspliced mRNA, rRNA, tRNA, inhibitory DNA or RNA (e.g. RNAi, microRNA (miRNA), short interfering (si)RNA, short hairpin (sh)RNA, trans-splicing RNA, antisense RNA, or antisense DNA), naturally occurring, synthetic, and intentionally modified or altered sequences (e.g., variant nucleic acids).

Accordingly, in some embodiments, the nucleic acid is selected from DNA, RNA, oligonucleotide molecule, and mixtures thereof. Preferably, the nucleic acid is DNA, more preferably plasmid DNA.

In some embodiments, the cationic polymer is a polymer-based transfection reagent. Cationic polymer-based transfection reagents include polyethylenimine (PEI), dendrimers, DEAE-dextran, polypropyleneimine (PPI), chitosan [poly-(β-1/4)-2-amino-2-deoxy-D-glucopyranose], poly-L-lysine (PLL), poly(lactic-co-glycolic acid) (PLGA), poly(caprolactone) (PCL), and derivatives thereof. A derivative may be a chemically modified variant of a cationic polymer, for example a PEGylated variant or a histidinylated variant.

The cationic polymer may be provided in a substantially salt-free aqueous solution, such as water, a neutral solution/buffer or an acidic solution. The cationic polymer may be prepared by dissolving the cationic polymer in an aqueous solvent, a neutral solvent/buffer or an acidic solvent. A neutral cationic polymer solution typically has a pH between about pH 6.0 to about pH 8.0 (suitably, between about pH 6.5 to about pH 7.5, between about pH 6.8 to about pH 7.2, or between about pH 7.0 to about pH 7.2). An acidic cationic polymer solution typically has a pH between about pH 0 to about pH 3.0 (suitably, between about pH 0.5 to about pH 2.0). Example neutral solvents/buffers include Tris (trizma base) and HEPES. Buffer concentrations can be in the range of about 1 mM to about 100 mM (suitably from about 2 mM to about 50 mM or from about 5 mM to about 20 mM). Example acidic solvents include mineral acids (e.g. hydrochloric acid) or organic acids (e.g. glycine-hydrochloric acid solution). Any solvent or buffer can be used for establishing or maintaining the pH of a cationic polymer solution within a suitable range without reducing the transfection activity of the cationic polymer. As described herein, cationic polymer solutions may be substantially free of salt.

PEI is a cationic polymer that provides one of the highest transfection efficiencies for the introduction of exogenous DNA into mammalian cells and is therefore one of the most widely used non-viral transfection reagents in the art. PE! can be linear PE! or branched PEI. PE! can have a molecular weight in the range of about 4 kDa to about 160 kDa (suitably, about 4 kDa to about 160 kDa, about 40 kDa or about 25 kDa). Linear PEI, branched PE! and derivatives thereof are commercially available. Specifically, 40 kDa linear PE! and 25 kDa linear PE! is commercially available. Commercially available forms of PE! include linear PEI, branched PEI, PEGylated PEI, JetPEI, PEIPro®, PEI MAX and PTG1+.

Thus, in some preferred embodiments, the cationic polymer is PE! (branched or linear PEI) or a derivative thereof. In some preferred embodiments, the cationic polymer is selected from linear PEI, branched PEI, PEGylated PEI, JetPEI, PEIPro®, PEI MAX and PTG1+.

The cationic polymer and nucleic acid may be mixed in a solution. The mixing can occur in any solution compatible with cationic polymer-based cell transduction. Non-limiting examples of suitable solutions are described herein. The molar or weight ratio of nucleic acid to cationic polymer is not limited - typical ratios include a molar ratio of about 100:1 to about 1:100 (suitably, about 1:1 to about 1:10, about 1:1 to about 1:5, about 1:2 to about 1:4) and a weight ratio of about 1:10 to about 5:1 (suitably, about 1:1 to about 5:1). The amount of nucleic acids and cationic polymer can also be expressed in terms of the molar ratio of total nitrogen (N) in the cationic polymer to the phosphate (P) in the nucleic acid, and is also not limited. Typical N:P ratios include about 1:1 to about 50:1 (suitably, about 1:1 to about 10:1, about 10:1, about 9:1, about 8:1, about 7:1, about 6:1 or about 5:1).

The salt may be suitable for use in cell culture. The skilled person would appreciate that the salt should have low toxicity. Salts that are compatible with cell culture are well-known in the art. The salt may be a simple salt or a mixture of simple salts. The salt may be selected from but not restricted to Ssodium (Na) salts, potassium (K) salts, magnesium (Mg) salts, calcium (Ca) salts, phosphate ($PO_4$) salts or combinations thereof. For example, the salt may be NaCl, $MgCl_2$, NaNOs; PBS or combinations thereof. The skilled person would appreciate that suitable salts include those having a cation of a strong base (e.g. selected from the first and second groups of the periodic table, such as K, Na, Ca, Mg, Ba) and/or an anion of a strong acid (e.g. $Cl^-$, $NO_3^-$ , $SO4^{2-}$).

In some embodiments, the salt is added to the mixture of cationic polymer and nucleic acid (i.e. in step b)) to a final concentration of between about 40 mM to about 500 mM (suitably, between about 50 mM to about 450 mM, between about 50 mM to about 400 mM, between about 60 mM to about 350 mM, between about 70 mM to about 300

mM, between about 75 mM to about 250 mM or between about 80 mM to 200 mM). In some embodiments, the salt is added to the mixture of cationic polymer and nucleic acid (i.e. in step b)) to a final concentration of between about 10 mM to about 100 mM (suitably, between about 30 mM to about 100 mM or between about 50 mM to about 100 mM). In some embodiments, the salt is added to a final concentration of less than about 500 mM (suitably, less than about 450 mM, less than about 400 mM, less than about 350 mM, less than about 300 mM, less than about 250 mM, less than about 200 mM, less than about 150 mM, less than about 100 mM, less than about 80 mM or less than about 50 mM).

In some embodiments, the salt is added to the mixture of cationic polymer and nucleic acid (i.e. in step b)) to a final concentration of between about 0.01 mM to about 49 mM (suitably, between about 0.1 mM to about 45 mM, between about 5 mM to about 45 mM, between about 10 mM to about 40 mM, between about 20 mM to about 35 mM). In some embodiments, the salt is added to the mixture of cationic polymer and nucleic acid (i.e. in step b)) to a final concentration of about 27.5 mM. In some embodiments, the salt is added to a final concentration of less than about 49 mM (suitably, less than about 45 mM, less than about 40 mM, less than about 35 mM, less than about 32 mM, less than about 30 mM, less than about 25 mM or less than about 20 mM).

In some preferred embodiments, the salt is phosphate buffered saline (PBS). In some embodiments, PBS is added in step b) to a final concentration of between about 0.2X PBS to about 10X PBS (suitably, between about 0.2X PBS to about 7.5X PBS or between about 0.2X PBS to about 5X PBS). In some embodiments, PBS is added in step b) to a final concentration of less than about 10X PBS (suitably, less than about 9X PBS, less than about 8X PBS, less than about 7X PBS, less than about 6X PBS, less than about 5X PBS or less than about 4X PBS). In some embodiments, PBS is added in step b) to a final concentration of between about 0.2X PBS to about 3X PBS (suitably, between about 0.2X PBS to about 2.5X PBS, between about 0.2X PBS to about 2X PBS, between about 0.2X PBS to about 1X PBS or between about 0.2X PBS to about 0.5X PBS). In some preferred embodiments, the PBS is added to a final concentration of between about 0.2X PBS to about 1X PBS. In some embodiments, PBS is added in step b) to a final concentration of less than about 3X PBS (suitably, less than about 2.5X PBS, less than about 2X PBS, less than about 1X PBS or less than about 0.5X PBS).

In some preferred embodiments, PBS is added in step b) to a final concentration of between about 0.15X PBS to about 0.35X PBS (suitably, between about 0.20X PBS to about 0.30X PBS). In some embodiments, the PBS is added to a final concentration of less than about 0.45X PBS (suitably, less than about 0.40X PBS, less than about 0.35X PBS, less than about 0.30X PBS or less than about 0.25X PBS). In some embodiments, PBS is added in step b) to a final concentration of about 0.2X PBS (suitably, about 0.25X PBS, about 0.3X PBS or about 0.35X PBS).

A mixture of cationic polymer and nucleic acid may be incubated in the presence of ions, e.g. in a salt solution, for the desired period of time in order to initiate polymer/nucleic acid complex formation and to grow the complexes to the optimum size for efficient transfection. In some preferred embodiments, the mixture of cationic polymer and nucleic acid is incubated in the presence of salt.

In some preferred embodiments, the method comprises the step of incubating the salt solution produced in step b). The nucleic acid and cationic polymer mixture (i.e. the salt solution produced in step b)) can be incubated for about 10 seconds to about 4 hours (suitably, for about 30 seconds to about 3 hours, for about 1 minute to about 2 hours, for about 5 minutes to about 90 minutes, for about 10 minutes to about 75 minutes, for about 20 minutes to about 60 minutes or for about 30 minutes to about 45 minutes). The nucleic acid and cationic polymer mixture (i.e. the salt solution produced in step b)) may be incubated for about 20 minutes, about 25 minutes, about 30 minutes, about 35 minutes, about 40 minutes, about 45 minutes, about 50 minutes, about 55 minutes, about 60 minutes, about 65 minutes, about 70 minutes or about 75 minutes. In some preferred embodiments, the salt solution produced in step b) is incubated for about 20 minutes to about 60 minutes, preferably for about 60 minutes. The incubation may take place at any temperature suitable for the formation and/or growth of polymer/nucleic acid complexes, e.g. room temperature.

A mixture of cationic polymer and nucleic acid in the presence of ions, e.g. in a salt solution, may be diluted, either with or without incubation of the mixture, to stabilise the polymer/nucleic acid complexes, e.g. for storage and later use. In some preferred embodiments, the mixture of cationic polymer and nucleic acid is diluted. In some preferred embodiments, the mixture of cationic polymer and nucleic acid is diluted after the incubation step. Thus, in some preferred embodiments, a solution of polymer/nucleic acid complexes is diluted following the incubation step.

In some embodiments, the method comprises the step of diluting the salt solution produced in step b) or the salt solution following the incubation of step c). In some preferred embodiments, the method comprises the step of diluting the salt solution following the incubation of step c). The diluent may be a solution which is substantially salt-free or salt-free, for example water, as described herein.

In some embodiments, said salt solution is diluted to a final concentration of between about 0.05X PBS to about 0.15X PBS, preferably wherein said salt solution is diluted to a final concentration of about 0.10X PBS.

In some embodiments, said salt solution is diluted to a final concentration of between about 5 mM to about 50 mM (suitably, between about 10 mM to about 40 mM or between about 15 mM to about 30 mM). In some embodiments, said salt solution is diluted to a final concentration of less than about 50 mM, less than about 40 mM, less than about 30 mM or less than about 20 mM.

The solution of polymer/nucleic acid complexes may be stored, e.g. at 4 °C, following dilution prior to use, e.g. for transfection.

It will be appreciated by the skilled person that the salt concentration suitable for initiating complex formation or for dilution to stabilise complex formation varies depending upon the ionic strength of the salt used. The selection of an appropriate salt concentration for use in the methods of the invention for a specific salt of known ionic strength is within the capabilities of the skilled person.

In some embodiments, the nucleic acid encodes a retroviral vector component.

In some embodiments, the retroviral vector is a replication defective retroviral vector.

In some embodiments, the retroviral vector is a lentiviral vector, preferably the lentiviral vector is HIV-1, HIV-2, SIV, FIV, BIV, EIAV, CAEV orVisna. The lentiviral vector may be a replication defective lentiviral vector.

In some embodiments, the vector component is selected from:

a) the RNA genome of the lentiviral vector;
b) env or a functional substitute thereof;
c) gag-pol or a functional substitute thereof; and/or
d) rev or functional substitute thereof.

**Vector / Expression cassette**

A vector is a tool that allows or facilitates the transfer of an entity from one environment to another. In accordance with the present invention, and by way of example, some vectors used in recombinant nucleic acid techniques allow entities, such as a segment of nucleic acid (e.g. a heterologous DNA segment, such as a heterologous cDNA segment), to be transferred into and expressed by a target cell. The vector may facilitate the integration of the nucleotide sequence encoding a viral vector component to maintain the nucleotide sequence encoding the viral vector component and its expression within the target cell.

The vector may be or may include an expression cassette (also termed an expression construct). Expression cassettes as described herein comprise regions of nucleic acid containing sequences capable of being transcribed. Thus, sequences encoding mRNA, tRNA and rRNA are included within this definition.

The vector may contain one or more selectable marker genes (e.g. a neomycin resistance gene) and/or traceable marker gene(s) (e.g. a gene encoding green fluorescent protein (GFP)). Vectors may be used, for example, to infect and/or transduce a target cell. The vector may further comprise a nucleotide sequence enabling the vector to replicate in the host cell in question, such as a conditionally replicating oncolytic vector.

The term "cassette" - which is synonymous with terms such as "conjugate", "construct" and "hybrid" - includes a polynucleotide sequence directly or indirectly attached to a promoter. The expression cassettes for use in the invention comprise a promoter for the expression of the nucleotide sequence encoding a viral vector component and optionally a regulator of the nucleotide sequence encoding the viral vector component. Preferably the cassette comprises at least a polynucleotide sequence operably linked to a promoter.

The choice of expression cassette, e.g. plasmid, cosmid, virus or phage vector, will often depend on the host cell into which it is to be introduced. The expression cassette can be a DNA plasmid (supercoiled, nicked or linearised), minicircle DNA (linear or supercoiled), plasmid DNA containing just the regions of interest by removal of the plasmid backbone by restriction enzyme digestion and purification, DNA generated using an enzymatic DNA amplification platform e.g. doggybone DNA (dbDNA™) where the final DNA used is in a closed ligated form or where it has been prepared (e.g. restriction enzyme digestion) to have open cut ends.

**Retroviral vector production systems and cells**

A retroviral vector production system (e.g. a lentiviral vector production system) comprises a set of nucleotide sequences encoding the components required for production of the retroviral vector (e.g. the lentiviral vector). Accordingly, a vector production system comprises a set of nucleotide sequences which encode the viral vector components necessary to generate retroviral vector particles (e.g. lentiviral vector particles).

"Viral vector production system" or "vector production system" or "production system" is to be understood as a system comprising the necessary components for lentiviral vector production.

In one embodiment, the viral vector production system comprises nucleotide sequences encoding Gag and Gag/Pol proteins, and Env protein and the vector genome sequence. The production system may optionally comprise a nucleotide sequence encoding the Rev protein, or functional substitute thereof.

In one embodiment of the invention at least one transgene component may be inverted or in the reverse orientation.

In one embodiment, at least one transgene component may be inverted or in the reverse orientation relative

to the 5'-3' directionality of the vector genome RNA. Lentiviral vector genomes wherein the transgene cassette is inverted, i.e. the transcription unit is opposed to the promoter driving the vector genome cassette, may be utilised. In addition, there may be instances where one component of the transgene cassette may be in reverse and another in the forward orientation, for example use of bi-directional transgene cassettes, or multiple separate cassettes.

In one embodiment, the viral vector production system comprises modular nucleic acid constructs (modular constructs). A modular construct is a DNA expression construct comprising two or more nucleic acids used in the production of lentiviral vectors. A modular construct can be a DNA plasmid comprising two or more nucleic acids used in the production of lentiviral vectors. The plasmid may be a bacterial plasmid. The nucleic acids can encode for example, gag-pol, rev, env, vector genome. In addition, modular constructs designed for generation of packaging and producer cell lines may additionally need to encode transcriptional regulatory proteins (e.g. TetR, CymR) and/or translational repression proteins (e.g. TRAP) and selectable markers (e.g Zeocin™, hygromycin, blasticidin, puromycin, neomycin resistance genes). Suitable modular constructs for use in the present invention are described in EP 3502260, which is hereby incorporated by reference in its entirety.

As the modular constructs for use in accordance with the present invention contain nucleic acid sequences encoding two or more of the retroviral components on one construct, the safety profile of these modular constructs has been considered and additional safety features directly engineered into the constructs. These features include the use of insulators for multiple open reading frames of retroviral vector components and/or the specific orientation and arrangement of the retroviral genes in the modular constructs. It is believed that by using these features the direct read-through to generate replication-competent viral particles will be prevented.

The nucleic acid sequences encoding the viral vector components may be in reverse and/or alternating transcriptional orientations in the modular construct. Thus, the nucleic acid sequences encoding the viral vector components are not presented in the same 5' to 3' orientation, such that the viral vector components cannot be produced from the same mRNA molecule. The reverse orientation may mean that at least two coding sequences for different vector components are presented in the 'head-to-head' and 'tail-to-tail' transcriptional orientations. This may be achieved by providing the coding sequence for one vector component, e.g. env, on one strand and the coding sequence for another vector component, e.g. rev, on the opposing strand of the modular construct. Preferably, when coding sequences for more than two vector components are present in the modular construct, at least two of the coding sequences are present in the reverse transcriptional orientation. Accordingly, when coding sequences for more than two vector components are present in the modular construct, each component may be orientated such that it is present in the opposite 5' to 3' orientation to all of the adjacent coding sequence(s) for other vector components to which it is adjacent, i.e. alternating 5' to 3' (or transcriptional) orientations for each coding sequence may be employed.

The modular construct for use according to the present invention may comprise nucleic acid sequences encoding two or more of the following vector components: gag-pol, rev, env, vector genome. The modular construct may comprise nucleic acid sequences encoding any combination of the vector components. In one embodiment, the modular construct may comprise nucleic acid sequences encoding:

i) the RNA genome of the lentiviral vector and rev, or a functional substitute thereof;
ii) the RNA genome of the lentiviral vector and gag-pol;
iii) the RNA genome of the lentiviral vector and env;
iv) gag-pol and rev, or a functional substitute thereof;
v) gag-pol and env;
vi) env and rev, or a functional substitute thereof;
vii) the RNA genome of the lentiviral vector, rev, or a functional substitute thereof, and gag-pol;
viii) the RNA genome of the lentiviral vector, rev, or a functional substitute thereof, and env;
ix) the RNA genome of the lentiviral vector, gag-pol and env; or
x) gag-pol, rev, or a functional substitute thereof, and env,

wherein the nucleic acid sequences are in reverse and/or alternating orientations.

In one embodiment, a cell for producing lentiviral vectors may comprise nucleic acid sequences encoding any one of the combinations i) to x) above, wherein the nucleic acid sequences are located at the same genetic locus and are in reverse and/or alternating orientations. The same genetic locus may refer to a single extrachromosomal locus in the cell, e.g. a single plasmid, or a single locus (i.e. a single insertion site) in the genome of the cell. The cell may be a stable or transient cell for producing retroviral vectors, e.g. lentiviral vectors.

The DNA expression construct can be a DNA plasmid (supercoiled, nicked or linearised), minicircle DNA (linear or supercoiled), plasmid DNA containing just the regions of interest by removal of the plasmid backbone by restriction enzyme digestion and purification, DNA generated using an enzymatic DNA amplification platform e.g. doggybone DNA (dbDNA™) where the final DNA used is in a closed ligated form or where it has been prepared (e.g restriction enzyme digestion) to have open cut ends.

In one embodiment, the lentiviral vector is derived from HIV-1, HIV-2, SIV, FIV, BIV, EIAV, CAEV or Visna lentivirus.

A "viral vector production cell", "vector production cell", or "production cell" is to be understood as a cell that is capable of producing a lentiviral vector or lentiviral vector particle. Lentiviral vector production cells may be "producer cells" or "packaging cells". One or more DNA constructs of the viral vector system may be either stably integrated or episomally maintained within the viral vector production cell. Alternatively, all the DNA components of the viral vector system may be transiently transfected into the viral vector production cell. In yet another alternative, a production cell stably expressing some of the components may be transiently transfected with the remaining components required for vector production.

As used herein, the term "packaging cell" refers to a cell which contains the elements necessary for production of lentiviral vector particles but which lacks the vector genome. Optionally, such packaging cells contain one or more expression cassettes which are capable of expressing viral structural proteins (such as *gag, gag/pol* and *env*) and typically rev.

Producer cells/packaging cells can be of any suitable cell type. Producer cells are generally mammalian cells but can be, for example, insect cells.

As used herein, the term "producer cell" or "vector producing/producer cell" refers to a cell which contains all the elements necessary for production of lentiviral vector particles. The producer cell may be either a stable producer cell line or derived transiently or may be a stable packaging cell wherein the retroviral genome is transiently expressed.

The vector production cells may be cells cultured *in vitro* such as a tissue culture cell line. Suitable cell lines include, but are not limited to, mammalian cells such as murine fibroblast derived cell lines or human cell lines. Preferably the vector production cells are derived from a human cell line.

**Cells and Production Methods**

The solution of polymer/nucleic acid complexes obtained or obtainable by the *in vitro* method as described herein or the solution of polymer nucleic acid complexes as described herein may be used for the transfection of a cell.

Accordingly, in some embodiments, the *in vitro* method for producing a solution of polymer/nucleic acid complexes as described herein further comprises the steps of:

e) optionally culturing a mammalian cell;
f) transfecting the mammalian cell using the solution of polymer/nucleic acid complexes obtained by the method as described herein;
g) optionally introducing a nucleic acid that is different to the nucleic acid of the solution of polymer/nucleic acid complexes into the mammalian cell;
h) optionally selecting for a mammalian cell which has the nucleic acid(s) integrated within its genome;
i) optionally culturing the mammalian cell under conditions in which the nucleic acid(s) is (are) expressed;
j) optionally culturing the mammalian cell under conditions in which the retroviral vector is produced; and
k) optionally isolating the retroviral vector.

Suitably, step e) is performed prior to steps a), b), c) and/or d), such as prior to steps a) to d).

Suitably, step e) is performed simultaneously to steps a), b), c) and/or d), such as simultaneously to steps a) to d).

Suitably, step e) is performed following steps a), b), c) and/or d), such as following steps a) to d).

In a further aspect, the invention provides the use of a solution of polymer/nucleic acid complexes as described herein for the transfection of the nucleic acid into cells.

In a further aspect, the invention provides the use of a solution of polymer/nucleic acid complexes as described herein in a method for the production of a retroviral vector.

In a further aspect, the invention provides a method for producing a retroviral vector comprising the steps of:

a) optionally culturing a mammalian cell;
b) transfecting the mammalian cell using a solution of polymer/nucleic acid complexes as described herein;
c) optionally introducing at least one nucleic acid that is different to the nucleic acid of the solution of polymer/nucleic acid complexes into the mammalian cell;
d) optionally selecting for a mammalian cell which has the nucleic acid sequences encoding the viral vector components integrated within its genome; and
e) further culturing the mammalian cell under conditions in which the retroviral vector is produced.

In a further aspect, the present invention provides a lentiviral vector produced by any method of the invention.

In a further aspect the present invention provides a method for generating a production cell for producing

lentiviral vectors, comprising the steps of:

a) optionally culturing a mammalian cell;
b) transfecting the mammalian cell using a solution of polymer/nucleic acid complexes as described herein;
c) optionally introducing at least one nucleic acid that is different to the nucleic acid of the solution of polymer/nucleic acid complexes into the mammalian cell; and
d) optionally selecting for a mammalian cell which comprises the nucleic acid sequences encoding the viral vector components.

In a further aspect the present invention provides a method for generating a stable production cell for producing lentiviral vectors, comprising the steps of:

a) optionally culturing a mammalian cell;
b) transfecting the mammalian cell using a solution of polymer/nucleic acid complexes as described herein;
c) optionally introducing at least one nucleic acid that is different to the nucleic acid of the solution of polymer/nucleic acid complexes into the mammalian cell; and
d) selecting for a cell which comprises said nucleic acids encoding vector components integrated within its genome.

In a further aspect, the invention provides a method for generating a transient production cell for producing lentiviral vectors, comprising optionally culturing a mammalian cell, transfecting the mammalian cell using a solution of polymer/nucleic acid complexes as described herein and optionally introducing at least one nucleic acid that is different to the nucleic acid of the solution of polymer/nucleic acid complexes into the mammalian cell.

In a further aspect, the invention provides a cell for producing lentiviral vectors produced by any method of the invention.

In a further aspect, the invention provides a stable production cell for producing lentiviral vectors produced by any method of the invention.

In a further aspect, the invention provides a transient production cell for producing lentiviral vectors produced by any method of the invention.

In some embodiments, the nucleic acid of the polymer/nucleic acid complex encodes a retroviral vector component. In some embodiments, the vector component is selected from:

a) the genome of the viral vector;
b) env or a functional substitute thereof;
c) gag-pol or a functional substitute thereof; and/or
d) rev or functional substitute thereof.

In some embodiments, the retroviral vector is a replication defective retroviral vector.

In some embodiments, the retroviral vector is a lentiviral vector, preferably the lentiviral vector is HIV-1, HIV-2, SIV, FIV, BIV, EIAV, CAEV or Visna.

In some embodiments, the vector component is selected from:

a) the RNA genome of the lentiviral vector;
b) env or a functional substitute thereof;
c) gag-pol or a functional substitute thereof; and/or
d) rev or functional substitute thereof.

In some embodiments, the method comprises the step of culturing a mammalian cell. Suitably, the step of culturing a mammalian cell may be performed in batch culture, fed-batch culture, concentrated fed-batch culture or perfusion culture.

As used herein, the term "batch culture" refers to an operational technique for biotechnological processes, including viral vector production, in which a limited supply of medium containing nutrients is provided for the cells, i.e. the cells are cultured in the same medium, without the addition of fresh medium and/or supplements and without the removal of nutrient-depleted medium containing impurities. Thus, when the nutrients are used up, or another factor becomes limiting, such as the production of waste product(s) or by-products(s), the culture declines. Typically, the viral vector product is harvested at the end of the process. Batch culture is an upstream processing technique in the production of a biomaterial or cell product, such as a viral vector.

As used herein, the term "fed-batch culture" refers to an operational technique for biotechnological processes, including viral vector production, in which one or more nutrients necessary for cell growth and viral vector production

(for example, nutrients which would otherwise become limiting) are supplied to the cells in the culture vessel, e.g. bioreactor, during cultivation. The viral vector product remains in the culture vessel, e.g. bioreactor, until the end of the process run, when the viral vector is harvested. The one or more nutrients may be supplied either continuously or periodically via at least one fed stream without the removal of nutrient-depleted medium containing impurities, i.e. the one or more nutrients are added to an otherwise batch process. The process may be repeated if the cells remain viable at the end of a process run. Fed-batch culture is an upstream processing technique in the production of a biomaterial or cell product, such as a viral vector.

As used herein, the term "concentrated fed-batch culture" refers to an operational technique for biotechnological processes, including viral vector production, in which a perfusion culture system, which can be alternating tangential flow (ATF) or tangential flow filtration (TFF), is used with an ultrafiltration membrane. In concentrated fed-batch culture, cells are retained inside the culture vessel, e.g. bioreactor, whilst nutrient-depleted medium containing impurities is removed and fresh media is supplied to the cells. This process retains the product in the culture vessel, e.g. bioreactor, until the end of the process run like conventional fed-batch culture, but obtains higher cell and product concentration in the culture vessel.

As used herein, the term "perfusion culture" (also known as "continuous perfusion culture" or "perfusion") refers to an operational technique for biotechnological processes, including viral vector production, in which cells are retained inside the culture vessel, e.g. bioreactor, whilst nutrient-depleted medium containing impurities is continuously removed and fresh media is continuously provided to the cells. Fresh media is typically provided to the cells at the same rate as the nutrient-depleted media containing impurities is removed. Therefore, continuous perfusion involves the continuous removal of small quantities of bioreactor working volume and introduction of fresh medium to the bioreactor, typically of an equivalent volume to the removed media. Perfusion culture is an upstream continuous processing technique in the production of a biomaterial or cell product, such as a viral vector. The viral vector product may remain in the culture vessel, e.g. bioreactor, until the end of the process run, when the viral vector is harvested. For example, this may be the case for viral vectors which are produced intracellularly, such as adenoviral vectors and adeno-associated viral vectors. Alternatively, the viral vector product may be perfused out of the culture vessel, e.g. bioreactor, followed by collection of the viral vector product from the perfusate. For example, this may be the case for secreted viral vectors, such as lentiviral vectors, if perfusion is performed after viral vector production has commenced.

In one embodiment, the method comprises the step of culturing a mammalian cell in a perfusion culture (e.g. in a perfusion culture system). Continuous perfusion culture is advantageous over batch or fed-batch culture in the production of viral vector as the build-up of impurities that limit output titre and purity is reduced by the media exchange.

The production process of the present invention is preferably a large scale-process for producing clinical grade formulations that are suitable for administration to humans as therapeutics. The viral vector compositions described herein are preferably suitable for administration to humans as therapeutics.

The method according to the invention as described herein may additionally comprise the step of inoculating a cell culture vessel with a mammalian cell. Suitably, the step of inoculating a cell culture vessel with a mammalian cell is carried out prior to the step of culturing the mammalian cell (e.g. culturing the mammalian cell in a perfusion culture). By "inoculating" is meant introducing the cell into culture medium, for example within the culture vessel.

Suitably, the step of culturing the mammalian cell (e.g. culturing the mammalian cell in a perfusion culture) is performed immediately following the step of inoculating a cell culture vessel with a mammalian cell.

In some embodiments, the method further comprises the step of performing a rapid media exchange after the step of inoculating the cell and prior to the step of transfecting the cell preferably wherein the medium is exchanged between about 20 and about 28 hours post-inoculation. Suitably, the medium may be exchanged between about 21 (suitably 22, 23, 24, 25, 26, or 27) and about 28 hours post-inoculation. Suitably, the medium may be exchanged between about 21 and about 27, 22 and about 26, or 23 and about 25 hours prior to transfection. Suitably, the medium may be exchanged at about 24 hours prior to transfection. Suitably, the medium may be exchanged less than about 21 (suitably less than about 22, about 23, about 24, about 25, about 26, about 27 or about 28) hours post-inoculation. Suitably, the step of culturing a mammalian cell comprises at least one rapid media exchange. Cell culture using rapid media exchange is advantageous over batch or fed-batch culture in the production of viral vector for the same reasons as perfusion culture, i.e. as the build-up of impurities that limit output titre and purity is reduced by the media exchange.

As used herein, the term "rapid media exchange" refers to an operational technique for biotechnological processes, including viral vector production, in which cells are retained inside a culture vessel, e.g. a bioreactor as described herein, whilst removing a defined amount of the cell culture vessel working volume and introducing a defined volume of fresh medium to the cell culture vessel in a short, discrete time period. Thus, media depleted of nutrients by cell metabolism, and impurities and other agents which may have a negative impact on viral vector production, such as transfection reagents, are removed and fresh media is provided to the cells during rapid media exchange. Rapid media exchange is a sequential process, i.e. spent media is first removed from the cell culture vessel (e.g. bioreactor) and then fresh media is introduced into the cell culture vessel (e.g. bioreactor). The defined volume of fresh medium which is introduced to the cell culture vessel may be higher than, lower than, equivalent to or equal to the defined amount of the cell culture

vessel working volume which is removed. The volume of nutrient-depleted media containing impurities removed is typically equivalent to the volume of fresh medium which is introduced. The defined amount of the cell culture vessel working volume is typically removed from the cell culture vessel followed by the introduction of the defined volume of fresh medium to the cell culture vessel, i.e. the working volume of the cell culture vessel is temporarily reduced during the rapid media exchange prior to the addition of fresh media. This differs from traditional perfusion processes, in which the removal of cell culture vessel working volume and introduction of fresh media typically occurs simultaneously. Rapid media exchange is an upstream continuous processing technique in the production of a biomaterial or cell product, such as a viral vector.

Suitably, the culture vessel may be a bioreactor, wave bag, roller bottle or flask. Preferably, the culture vessel is a bioreactor.

In one embodiment, the suspension culture may be in a culture vessel such as a bioreactor. The bioreactor may have a volume (e.g. a working volume) of, for example, from about 0.1 litres to about 1000 litres, such as about 0.1 litres to about 500 litres.

For example, the volume, such as working volume, may be from about 0.1 litres to about 0.25 litres, about 0.5 litres to about 250 litres, about 1 litre to about 200 litres, from about 5 to about 180 litres, from about 10 to about 150 litres, from about 15 to about 100 litres, from about 20 to about 80 litres, or from about 30 to about 50 litres.

In one embodiment, the volume, such as working volume, may be about 0.1, 0.25, 0.5, 0.75, 1, 2, 3, 4, 5, 10, 15, 20, 25, 30, 35, 40, 45, 50, 55, 60, 65, 70, 75, 80, 85, 90, 95, 100, 150, 200, 250, 300, 350, 400, 450, 500, 1000 or 2000 litres.

In one embodiment, the working volume may be about 2 litres to about 100 litres.

In one embodiment, the working volume may be about 5 litres.

In one embodiment, the working volume may be about 50 litres.

In one embodiment, the working volume may be about 200 litres.

In one embodiment, the working volume may be about 1000 litres.

In some embodiments, the method comprises the step of culturing the mammalian cell under conditions in which the nucleic acid(s) is expressed.

In some embodiments, the method comprises the step of culturing the mammalian cell under conditions in which the retroviral vector is produced.

In some embodiments, the method comprises the step of isolating the retroviral vector.

In some embodiments, the mammalian cells are HEK293T cells.

In some embodiments, the mammalian cells are suspension-adapted mammalian cells.

As used herein, the term "suspension-adapted cells" refers to cells that typically grow in adherent mode which have been adapted to suspension growth. Methods for the adaptation of cells to suspension are known in the art. For example, adaptation to suspension growth can be carried out by sequential serum reduction during passage of the cells. This yields, suspension-adapted cells that grow in suspension in a serum-free medium, i.e. in the absence of serum such as fetal bovine serum (FBS).

Thus, in some embodiments, the transfection, introduction and/or culturing step(s) is (are) performed in suspension in a serum-free medium.

The methods of the invention may be performed on adherent cells cultured in suspension systems. For example, adherent cells may be cultivated on microcarriers in suspension systems.

In some embodiments, the transfection is a transient transfection.

The methods and solution of polymer/nucleic acid complexes described herein are suitable for use in large scale production of material. Non-limiting examples of large volume culture vessels suitable for use in the present invention include spinner flasks (up to about 36 L volume), wave bags (up to about 100 L volume) and bioreactors (up to about 10 000 L volume). In some embodiments, the transfection, introduction and/or culturing step(s) is (are) carried out in a volume of at least 50 L (suitably at least 60 L, at least 75 L, at least 100 L, at least 200 L), preferably the transfection, introduction and culturing steps are carried out in a volume of at least 50 L (suitably at least 60 L, at least 75 L, at least 100 L, at least 200 L).

In some embodiments, the nucleic acid that is different to the nucleic acid of the solution of polymer/nucleic acid complexes is introduced into the mammalian cell by transfection or by electroporation.

In some embodiments, the nucleic acid that is different to the nucleic acid of the solution of polymer/nucleic acid complexes encodes any viral vector components selected from:

a) the RNA genome of the lentiviral vector;
b) env or a functional substitute thereof;
c) gag-pol or a functional substitute thereof; and/or
d) rev or functional substitute thereof;

that are not encoded by the nucleic acid of the solution of polymer/nucleic acid complexes.

In some embodiments, the method further comprises the step of repeating steps (i), (j) and/or (k). In some embodiments, the method further comprises the step of repeating steps (i), (j) and (k). In some embodiments, the method further comprises the step of repeating steps (i) and (k). In some embodiments, the method further comprises the step of repeating steps (j) and (k). Steps (i), (j) and/or (k) may be repeated multiple times.

As described above, perfusion culture is advantageous over batch or fed-batch culture in the production of viral vector as the build-up of impurities that limit output titre and purity is reduced by the media exchange. This enables a higher cell density to be reached. Thus, the length of the perfusion step can be varied according to the target cell density for transfection. Suitably, the cell can be continuously cultured in a perfusion cell culture system for a long period of time. Suitably, the cell may be continuously cultured in a perfusion cell culture system from inoculation until the transfection step or until shortly before the transfection step.

In some embodiments, the cell is cultured in a perfusion culture system for about 0.5 hours to about 168 hours (suitably, about 0.5 hours to about 156 hours, about 0.5 hours to about 144 hours, about 0.5 hours to about 132 hours, about 0.5 hours to about 120 hours, about 0.5 hours to about 108 hours, about 0.5 hours to about 96 hours, about 0.5 hours to about 84 hours, about 0.5 hours to about 72 hours, about 0.5 hours to about 60 hours, about 0.5 hours to about 48 hours, about 0.5 hours to about 36 hours, about 0.5 hours to about 32 hours, about 0.5 hours to about 28 hours, about 0.5 hours to about 24 hours, about 0.5 hours to about 22 hours, about 0.5 hours to about 20 hours, about 0.5 hours to about 18 hours, or about 0.5 hours to about 16 hours) prior to the step of transfecting the mammalian cell using a solution of polymer/nucleic acid complexes as described herein. Preferably, the cell is cultured in a perfusion culture system for about 0.5 hours to about 24 hours, about 0.5 hours to about 22 hours, about 0.5 hours to about 20 hours, or about 0.5 hours to about 18 hours, such as about 0.5 hours to about 20 hours.

In some embodiments, the cell is cultured in a perfusion culture system for about 1 hour to about 168 hours (suitably, about 1 hour to about 156 hours, about 1 hour to about 144 hours, about 1 hour to about 132 hours, about 1 hour to about 120 hours, about 1 hour to about 108 hours, about 1 hour to about 96 hours, about 1 hour to about 84 hours, about 1 hour to about 72 hours, about 1 hour to about 60 hours, about 1 hour to about 48 hours, about 1 hour to about 36 hours, about 1 hour to about 32 hours, about 1 hour to about 28 hours, about 1 hour to about 24 hours, about 1 hour to about 22 hours, about 1 hour to about 20 hours, about 1 hour to about 18 hours, or about 1 hour to about 16 hours) prior to the step of transfecting the mammalian cell using a solution of polymer/nucleic acid complexes as described herein. Preferably, the cell is cultured in a perfusion culture system for about 1 hour to about 24 hours, about 1 hour to about 22 hours, about 1 hour to about 20 hours, or about 1 hour to about 18 hours, such as about 1 hour to about 20 hours.

In some embodiments, the cell is cultured in a perfusion culture system for about 12 hours to about 168 hours (suitably, about 12 hours to about 156 hours, about 12 hours to about 144 hours, about 12 hours to about 132 hours, about 12 hours to about 120 hours, about 12 hours to about 108 hours, about 12 hours to about 96 hours, about 12 hours to about 84 hours, about 12 hours to about 72 hours, about 12 hours to about 60 hours, about 12 hours to about 48 hours, about 12 hours to about 36 hours, about 12 hours to about 32 hours, about 12 hours to about 28 hours, about 12 hours to about 24 hours, about 12 hours to about 22 hours, about 12 hours to about 20 hours, about 12 hours to about 18 hours, or about 12 hours to about 16 hours) prior to the step of transfecting the mammalian cell using a solution of polymer/nucleic acid complexes as described herein. Preferably, the cell is cultured in a perfusion culture system for about 12 hours to about 24 hours, about 12 hours to about 22 hours, about 12 hours to about 20 hours, or about 12 hours to about 18 hours, such as about 12 hours to about 20 hours.

In some embodiments, the transfection step is carried out immediately following the step of culturing the mammalian cell in a perfusion culture.

In some embodiments, the transfection step is carried out about 0.5 hours to about 24 hours (suitably, about 0.5 hours, about 1 hour, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours or about 24 hours, preferably about 1 hour) following the step of culturing the mammalian cell in a perfusion culture.

In some embodiments, the transfection step is carried out at least about 0.5 hours to at least about 24 hours (suitably, at least about 0.5 hours, at least about 1 hour, at least about 2 hours, at least about 3 hours, at least about 4 hours, at least about 5 hours, at least about 6 hours, at least about 7 hours, at least about 8 hours, at least about 9 hours, at least about 10 hours, at least about 11 hours, at least about 12 hours, at least about 13 hours, at least about 14 hours, at least about 15 hours, at least about 16 hours, at least about 17 hours, at least about 18 hours, at least about 19 hours, at least about 20 hours, at least about 21 hours, at least about 22 hours, at least about 23 hours or at least about 24 hours, preferably at least about 1 hour) following the step of culturing the mammalian cell in a perfusion culture.

In some embodiments, the perfusion culture is performed by removing about 50% to about 1000% (suitably, about 50% to about 900%, about 50% to about 800%, about 50% to about 700%, about 50% to about 600%, about 50% to about 500%, about 50% to about 400% about 50% to about 300%, about 50% to about 250%, about 50% to about

200%, about 50% to about 150%, about 50% to about 100%, about 100%, about 150%, about 200%, about 300%, about 400%, about 500%, about 600%, about 700%, about 800%, about 900% or about 1000%) of the suspension cell culture volume and introducing a suitable volume of fresh medium to the suspension cell culture over the entire duration of the perfusion culture. Thus, in some embodiments, the perfusion culture is performed by removing about 50% to about 1000% (suitably, about 50% to about 900%, about 50% to about 800%, about 50% to about 700%, about 50% to about 600%, about 50% to about 500%, about 50% to about 400% about 50% to about 300%, about 50% to about 250%, about 50% to about 200%, about 50% to about 150%, about 50% to about 100%, about 100%, about 150%, about 200%, about 300%, about 400%, about 500%, about 600%, about 700%, about 800%, about 900% or about 1000%) of the cell culture vessel working volume, e.g. bioreactor working volume, and introducing a suitable volume of fresh medium to the cell culture vessel, e.g. bioreactor. Suitably, this volume is removed over the entire duration of the perfusion culture. The volume of fresh medium which is introduced to the cell culture vessel may be higher than, lower than, equivalent to or equal to the cell culture vessel working volume which is removed during the perfusion culture. In a preferred embodiment, the volume of fresh media which is introduced to the suspension cell culture is equivalent to the suspension cell culture volume which is removed during the perfusion culture.

Suitably, about 50% to about 1000% (suitably, about 50% to about 900%, about 50% to about 800%, about 50% to about 700%, about 50% to about 600%, about 50% to about 500%, about 50% to about 400% about 50% to about 300%, about 50% to about 250%, about 50% to about 200%, about 50% to about 150%, about 50% to 100%, about 100%, about 150%, about 200%) of the suspension cell culture volume may be removed and the volume of fresh media which is introduced to the suspension cell culture may be equivalent to the suspension cell culture volume which is removed.

The suspension cell culture volume removed in the perfusion culture step may be at least about 50% (suitably at least about 100%, at least about 150%, at least about 200%, at least about 250%, at least about 300%, at least about 400%, at least about 500%, at least about 600%, at least about 700%, at least about 800%, at least about 900% or at least about 1000%) of the suspension cell culture volume.

The suspension cell culture volume removed in the perfusion culture step may be less than about 50% (suitably less than about 100%, less than about 150%, less than about 200%, less than about 250%, less than about 300%%, less than about 400%, less than about 500%, less than about 600%, less than about 700%, less than about 800%, less than about 900% or less than about 1000%) of the suspension cell culture volume.

In a preferred embodiment, about 100% to about 200% (suitably about 150%) of the suspension cell culture volume is removed in the perfusion culture step and the volume of fresh media which is introduced to the suspension cell culture may be equivalent to the suspension cell culture volume which is removed.

In some embodiments, the perfusion culture is performed using a flow rate of about 50% to about 500% (suitably, about 50% to about 450%, about 50% to about 400%, about 50% to about 350%, about 50% to about 300%, about 50% to about 250%, about 50% to about 200%, about 50% to about 150%, about 50% to 100%, about 100%, about 150%, about 200%) of the suspension cell culture volume per day. Thus, in some embodiments, the perfusion culture is performed using a flow rate of about 50% to about 500% (suitably, about 50% to about 450%, about 50% to about 400%, about 50% to about 350%, about 50% to about 300%, about 50% to about 250%, about 50% to about 200%, about 50% to about 150%, about 50% to 100%, about 100%, about 150%, about 200%) of the cell culture vessel working volume, e.g. bioreactor working volume, per day. Preferably, the perfusion culture is performed using a flow rate of about 50% to about 200% (suitably, about 50% to about 150% or about 50% to 100%) of the suspension cell culture volume per day. Preferably, the perfusion culture is performed using a flow rate of about 200%, about 150% or about 100% of the suspension cell culture volume per day. Preferably, the perfusion culture is performed using a flow rate of about 150% of the suspension cell culture volume per day.

As described above, the use of rapid media exchange is advantageous over batch or fed-batch culture in the production of viral vector as the build-up of impurities that limit output titre and purity is reduced by the media exchange. This enables a higher cell density to be reached.

In some embodiments, the rapid media exchange is completed within about 0.1 to about 8 hours (suitably about 0.2 to about 8 hours, about 0.3 to about 8 hours, about 0.4 to about 8 hours, about 0.5 to about 8 hours, about 1 to about 8 hours, about 1.5 to about 8 hours, about 2 to about 8 hours, about 2.5 to about 8 hours, about 3 to about 8 hours, about 4 to about 8 hours, about 5 to about 8 hours).

The rapid media exchange may be completed within about 0.1 to about 5 hours (suitably about 0.2 to about 5 hours, about 0.3 to about 5 hours, about 0.4 to about 5 hours, about 0.5 to about 5 hours, about 1 to about 5 hours, about 1.5 to about 5 hours, about 2 to about 5 hours, about 2.5 to about 5 hours, about 3 to about 5 hours, about 4 to about 5 hours). The rapid media exchange may be completed within about 0.1 to about 3 hours (suitably about 0.2 to about 3 hours, about 0.3 to about 3 hours, about 0.4 to about 3 hours, about 0.5 to about 3 hours, about 1 to about 3 hours, about 1.5 to about 3 hours, about 2 to about 3 hours, about 2.5 to about 3 hours).

The rapid media exchange may be completed in less than about 0.5 hours (suitably less than about 1, about 1.5, about 2, about 2.5, about 3, about 3.5, about 4, about 4.5, about 5, about 5.5, about 6, about 6.5, about 7, about

7.5, about 8 hours).

In some embodiments, the rapid media exchange is performed by removing about 50% to about 99% of the suspension cell culture volume and introducing a suitable volume of fresh medium to the suspension cell culture. Thus, in some embodiments, the rapid media exchange is performed by removing about 50% to about 99% of the cell culture vessel working volume, e.g. bioreactor working volume, and introducing a suitable volume of fresh medium to the cell culture vessel, e.g. bioreactor. The volume of fresh medium which is introduced to the cell culture vessel may be higher than, lower than, equivalent to or equal to the cell culture vessel working volume which is removed during the rapid media exchange. In a preferred embodiment, the volume of fresh media which is introduced to the suspension cell culture is equivalent to the suspension cell culture volume which is removed during the rapid media exchange.

Suitably, about 55% to about 99% (suitably about 60% to about 99%, about 65% to about 99%, about 70% to about 99%, about 75% to about 99%, about 80% to about 99%, about 85% to about 99%, about 90% to about 99%) of the suspension cell culture volume may be removed during the rapid media exchange p and the volume of fresh media which is introduced to the suspension cell culture may be equivalent to the suspension cell culture volume which is removed.

Suitably, about 55% to about 95% (suitably about 60% to about 90%, about 65% to about 85%, about 70% to about 80%, about 70% to about 85%, about 75% to about 85%) of the suspension cell culture volume may be removed during the rapid media exchange and the volume of fresh media which is introduced to the suspension cell culture may be equivalent to the suspension cell culture volume which is removed.

In a preferred embodiment, about 70% to about 85% (suitably about 75% or about 80%) of the suspension cell culture volume is removed and the volume of fresh media which is introduced to the suspension cell culture may be equivalent to the suspension cell culture volume which is removed.

The suspension cell culture volume removed may be at least about 50% (suitably at least about 55%, at least about 60%, at least about 65%, at least about 70%, at least about 75%, at least about 80%, at least about 85%, at least about 90%, at least about 95%, at least about 99%).

The suspension cell culture volume removed may be less than about 55%, less than about 60%, less than about 65%, less than about 70%, less than about 75%, less than about 80%, less than about 85%, less than about 90%, less than about 95%, less than about 99%).

In some embodiments, the rapid media exchange is performed between 0 to about 24 hours (suitably about 0.1, about 0.2, about 0.3, about 0.4, about 0.5, about 1, about 1.5, about 2, about 2.5, about 3, about 3.5, about 4, about 4.5, about 5, about 5.5, about 6, about 6.5, about 7, about 7.5, about 8, about 8.5, about 9, about 9.5, about 10, about 11, about 12, about 13, about 14, about 15, about 16, about 17, about 18, about 19, about 20, about 21, about 22, about 23 to about 24 hours) prior to transfection. In some embodiments, the rapid media exchange is performed between 0 to about 3 hours (suitably about 0.1, about 0.2, about 0.3, about 0.4, about 0.5, about 1, about 1.5, about 2 or about 2.5 to about 3 hours) prior to transfection. The rapid media exchange may be performed between 0 to about 2 hours (suitably about 0.1, about 0.2, about 0.3, about 0.4, about 0.5, about 1 or about 1.5 to about 2 hours) prior to transfection. The rapid media exchange may be performed less than about 0.1, about 0.2, about 0.3, about 0.4, about 0.5, about 1, about 1.5, about 2, about 2.5, about 3, about 3.5, about 4, about 4.5, about 5, about 5.5, about 6, about 6.5, about 7, about 7.5, about 8, about 8.5, about 9, about 9.5, about 10, about 11, about 12, about 13, about 14, about 15, about 16, about 17, about 18, about 19, about 20, about 21, about 22, about 23, or about 24 hours prior to transfection. In a preferred embodiment, the rapid media exchange is performed about 1 hour prior to transfection.

In some embodiments, the cells may be in the exponential growth phase at the time of perfusion culture. The cells may have a final cell density of between about $1\times10^5$ cells/mL to about $1\times10^9$ cells/mL (suitably, between about $2\times10^5$ cells/mL to about $5\times10^8$ cells/mL, between about $3\times10^5$ cells/mL to about $1\times10^8$ cells/mL, between about $4\times10^5$ cells/mL to about $5\times10^7$ cells/mL, between about $5\times10^5$ cells/mL to about $1\times10^7$ cells/mL, between about $6\times10^5$ cells/mL to about $5\times10^6$ cells/mL, between about $7\times10^5$ cells/mL to about $1\times10^6$ cells/mL) following the perfusion culture step. The cells may have a final cell density of between about $1\times10^5$ cells/mL to about $5\times10^7$ cells/mL (suitably, between about $2\times10^5$ cells/mL to about $1\times10^7$ cells/mL, between about $3\times10^5$ cells/mL to about $5\times10^6$ cells/mL, between about $4\times10^5$ cells/mL to about $1\times10^6$ cells/mL, between about $5\times10^5$ cells/mL to about $1\times10^6$ cells/mL) following the perfusion culture step. The cell number may increase by at least about 25% (suitably, at least about 50%, at least about 70%, at least about 100%, at least about 150%, at least about 200%, at least about 250%, at least about 300%, at least about 350%, at least about 400%, at least about 450%, at least about 500%, at least about 600%, at least about 700%, at least about 800%, at least about 900% or at least about 1000%) during the perfusion culture step. The cell number may increase by about 25% (suitably, about 50%, about 70%, about 100%, about 150%, about 200%, about 250%, about 300%, about 350%, about 400%, about 450%, about 500%, about 600%, about 700%, about 800%, about 900% or about 1000%) during the perfusion culture step. The cell number may increase by about 0.5-fold (suitably, about 1-fold, about 1.5-fold, about 2-fold, about 2.5-fold, about 3-fold, about 3.5-fold, about 4-fold, about 4.5-fold, about 5-fold, about 6-fold, about 7-fold, about 8-fold, about 9-fold, about 10-fold, about 15-fold, about 20-fold, about 25-fold) during the perfusion culture step.

The solution of polymer/nucleic acid complexes may be contacted with (i.e. incubated with) the cells for transfection for a time period which is not limited. For example, the time period may be from about 20 minutes to about 24 hours (suitably, about 30 minutes to about 20 hours, about 1 hour to about 16 hours, about 2 hours to about 12 hours, about 3 hours to about 8 hours). The culture medium may be replaced with fresh medium post-transfection to reduce the cytotoxic effects of the cationic polymer on the cells.

The cells may be in the exponential growth phase prior to or at the time of contact with the solution of polymer/nucleic acid complexes. The cells may have a cell density of between about $1\times10^5$ cells/mL to about $1\times10^8$ cells/mL (suitably, between about $2\times10^5$ cells/mL to about $5\times10^6$ cells/mL, between about $3\times10^5$ cells/mL to about $4\times10^6$ cells/mL, between about $4\times10^5$ cells/mL to about $3\times10^6$ cells/mL, between about $5\times10^5$ cells/mL to about $2\times10^6$ cells/mL, between about $6\times10^5$ cells/mL to about $1\times 10^6$ cells/mL) prior to or at the time of contact with the solution of polymer/nucleic acid complexes.

In some embodiments, the ratio of the polymer/nucleic acid complex : cells used in the transfection step is about 0.1 μg DNA per $1\times10^6$ cells (suitably, about 0.2 μg DNA per $1\times10^6$ cells, about 0.3 μg DNA per $1\times10^6$ cells, about 0.4 μg DNA per $1\times10^6$ cells, about 0.5 μg DNA per $1\times10^6$ cells, about 0.6 μg DNA per $1\times10^6$ cells, about 0.7 μg DNA per $1\times10^6$ cells, about 0.8 μg DNA per $1\times10^6$ cells, about 0.9 μg DNA per $1\times10^6$ cells, about 1 μg DNA per $1\times10^6$ cells, 1.5 μg DNA per $1\times10^6$ cells or 2 μg DNA per $1\times10^6$ cells). Preferably, the ratio of the polymer/nucleic acid complex : cells used in the transfection step is about 0.6 μg DNA per $1\times10^6$ cells.

In some embodiments, the ratio of the polymer/nucleic acid complex : cells used in the transfection step is about 0.3 μg DNA per $1\times10^6$ cells (suitably, about 0.6 μg DNA per $1\times10^6$ cells, about 1 μg DNA per $1\times10^6$ cells, 1.5 μg DNA per $1\times10^6$ cells or 2 μg DNA per $1\times10^6$ cells). Preferably, the ratio of the polymer/nucleic acid complex : cells used in the transfection step is about 0.6 μg DNA per $1\times10^6$cells.

In the methods of the invention, the vector components may include gag, env, rev and/or the RNA genome of the viral vector. The nucleotide sequences encoding vector components may be introduced into the cell either simultaneously or sequentially in any order.

In some embodiments of the methods and uses of the invention, suitable production cells or cells for producing a lentiviral vector are those cells which are capable of producing viral vectors or viral vector particles when cultured under appropriate conditions. Thus, the cells typically comprise nucleotide sequences encoding vector components, which may include gag, env, rev and the RNA genome of the lentiviral vector. Suitable cell lines include, but are not limited to, mammalian cells such as murine fibroblast derived cell lines or human cell lines. They are generally mammalian, including human cells, for example HEK293T, HEK293, CAP, CAP-T or CHO cells, but can be, for example, insect cells such as SF9 cells. Preferably, the vector production cells are derived from a human cell line. Accordingly, such suitable production cells may be employed in any of the methods or uses of the present invention.

Methods for introducing nucleotide sequences into cells are well known in the art and have been described previously. Thus, the introduction into a cell of nucleotide sequences encoding vector components including gag, env, rev and the RNA genome of the lentiviral vector using conventional techniques in molecular and cell biology is within the capabilities of a person skilled in the art.

Stable production cells may be packaging or producer cells. To generate producer cells from packaging cells the vector genome DNA construct may be introduced stably or transiently. Packaging/producer cells can be generated by transducing a suitable cell line with a retroviral vector which expresses one of the components of the vector, i.e. a genome, the *gag-pol* components and an envelope as described in WO 2004/022761.

Alternatively, the nucleotide sequence can be transfected into cells and then integration into the production cell genome occurs infrequently and randomly. The transfection methods using a solution of polymer/nucleic acid complexes as described herein may be performed using methods well known in the art. For example, a stable transfection process may employ constructs which have been engineered to aid concatemerisation. The skilled person will be aware of methods to encourage integration of the nucleotide sequences into production cells. For example, linearising a nucleic acid construct can help if it is naturally circular. Less random integration methodologies may involve the nucleic acid construct comprising of areas of shared homology with the endogenous chromosomes of the mammalian host cell to guide integration to a selected site within the endogenous genome. Furthermore, if recombination sites are present on the construct then these can be used for targeted recombination. For example, the nucleic acid construct may contain a *loxP* site which allows for targeted integration when combined with Cre recombinase (i.e. using the Cre/lox system derived from P1 bacteriophage). Alternatively, or additionally, the recombination site is an *att* site (e.g. from λ phage), wherein the *att* site permits site-directed integration in the presence of a lambda integrase. This would allow the lentiviral genes to be targeted to a locus within the host cellular genome which allows for high and/or stable expression.

Other methods of targeted integration are well known in the art. For example, methods of inducing targeted cleavage of genomic DNA can be used to encourage targeted recombination at a selected chromosomal locus. These methods often involve the use of methods or systems to induce a double strand break (DSB) e.g. a nick in the endogenous genome to induce repair of the break by physiological mechanisms such as non-homologous end joining (NHEJ). Cleavage can occur through the use of specific nucleases such as engineered zinc finger nucleases (ZFN), transcription-activator like effector nucleases (TALENs), using CRISPR/Cas9 systems with an engineered crRNA/tracr RNA ('single guide RNA') to guide specific cleavage, and/or using nucleases based on the Argonaute system (e.g., from T. *thermophilus*).

Packaging/producer cell lines can be generated by integration of nucleotide sequences using methods of just lentiviral transduction or just nucleic acid transfection, or a combination of both can be used.

Methods for generating retroviral vectors from production cells and in particular the processing of retroviral vectors are described in WO 2009/153563.

In one embodiment, the production cell may comprise the RNA-binding protein (e.g. tryptophan RNA-binding attenuation protein, TRAP) and/or the Tet Repressor (TetR) protein or alternative regulatory proteins (e.g. CymR).

Production of lentiviral vector from production cells can be via transfection methods, from production from stable cell lines which can include induction steps (e.g. doxycycline induction) or via a combination of both. The transfection methods may be performed using methods well known in the art, and examples have been described previously.

Production cells, either packaging or producer cell lines or those transiently transfected with the lentiviral vector encoding components are cultured to increase cell and virus numbers and/or virus titres. Culturing a cell is performed to enable it to metabolize, and/or grow and/or divide and/or produce viral vectors of interest according to the invention. This can be accomplished by methods well known to persons skilled in the art, and includes but is not limited to providing nutrients for the cell, for instance in the appropriate culture media. The methods may comprise growth adhering to surfaces, growth in suspension, or combinations thereof. Culturing can be done for instance in tissue culture flasks, tissue culture multiwell plates, dishes, roller bottles, wave bags or in bioreactors, using batch, fed-batch, continuous systems and the like. In order to achieve large scale production of viral vector through cell culture it is preferred in the art to have cells capable of growing in suspension. Suitable conditions for culturing cells are known (see e.g. Tissue Culture, Academic Press, Kruse and Paterson, editors (1973), and R.I. Freshney, Culture of animal cells: A manual of basic technique, fourth edition (Wiley- Liss Inc., 2000, ISBN 0-471-34889-9).

Preferably, cells are initially 'bulked up' in tissue culture flasks or bioreactors and subsequently grown in multilayered culture vessels or large bioreactors (greater than 50 L) to generate the vector producing cells of the present invention.

Preferably, cells are grown in an adherent mode to generate the vector producing cells of the present invention.

Preferably, cells are grown in a suspension mode to generate the vector producing cells of the present invention.

**Solution of polymer/nucleic acid complexes**

In a further aspect, the invention provides a solution of polymer/nucleic acid complexes obtained or obtainable by any of the *in vitro* methods as described herein.

Accordingly, in a further aspect, the invention provides a solution comprising polymer/nucleic acid complexes and a salt, wherein the salt concentration is between about 10 mM to about 500 mM. In some embodiments, the salt concentration is between about 50 mM to about 500 mM.

In a further aspect, the invention provides a solution comprising polymer/nucleic acid complexes and a salt, wherein the salt concentration is between about 0.1 mM to about 49 mM. In some embodiments, the salt concentration is between about 10 mM to about 49 mM.

In some embodiments, the salt is PBS and the PBS concentration is between about 0.15X PBS to about 3X PBS.

In some embodiments, the concentration of PBS is less than about 0.3X PBS.

**Method for transfecting a mammalian cell**

In a further aspect, the invention provides a method for transfecting a mammalian cell comprising the steps of:

a) culturing a mammalian cell; and

b) transfecting the mammalian cell using solution of polymer/nucleic acid complexes as described herein.

In some embodiments, the step of culturing a mammalian cell is performed in a perfusion culture.

Accordingly, in a further aspect, the invention provides a method for transfecting a mammalian cell comprising the steps of:

a) culturing a mammalian cell in a perfusion culture; and

b) transfecting the mammalian cell using solution of polymer/nucleic acid complexes as described herein.

Suitably, the mammalian cell is a mammalian cell as described herein.

In some embodiments, the method is performed in suspension in a serum-free medium.

In some embodiments, step a) and/or step b) is carried out in a volume of at least 50 L, preferably wherein step a) and step b) are carried out in a volume of at least 50 L.

In some embodiments, the transfection is a transient transfection.

Suitably, the steps of culturing a mammalian cell (e.g. in a perfusion culture) and of transfecting the mammalian cell are performed as described herein.

**Retroviral vectors and lentiviral vectors**

A viral vector may also be called a vector, vector virion or vector particle.

The retroviral vector may be derived from or may be derivable from any suitable retrovirus. A large number of different retroviruses have been identified. Examples include: murine leukemia virus (MLV), human T-cell leukemia virus (HTLV), mouse mammary tumour virus (MMTV), Rous sarcoma virus (RSV), Fujinami sarcoma virus (FuSV), Moloney murine leukemia virus (Mo MLV), FBR murine osteosarcoma virus (FBR MSV), Moloney murine sarcoma virus (Mo-MSV), Abelson murine leukemia virus (A-MLV), Avian myelocytomatosis virus-29 (MC29) and Avian erythroblastosis virus (AEV). A detailed list of retroviruses may be found in Coffin et al. (1997) "Retroviruses", Cold Spring Harbour Laboratory Press Eds: JM Coffin, SM Hughes, HE Varmus pp 758-763.

Retroviruses may be broadly divided into two categories, namely "simple" and "complex". Retroviruses may even be further divided into seven groups. Five of these groups represent retroviruses with oncogenic potential. The remaining two groups are the lentiviruses and the spumaviruses. A review of these retroviruses is presented in Coffin et al (1997) ibid.

Lentiviruses are part of a larger group of retroviruses. A detailed list of lentiviruses may be found in Coffin et al (1997) "Retroviruses" Cold Spring Harbour Laboratory Press Eds: JM Coffin, SM Hughes, HE Varmus pp 758-763). In brief, lentiviruses can be divided into primate and non-primate groups. Examples of primate lentiviruses include but are not limited to: the human immunodeficiency virus (HIV), the causative agent of human auto-immunodeficiency syndrome (AIDS), and the simian immunodeficiency virus (SIV). The non-primate lentiviral group includes the prototype "slow virus" visna/maedi virus (VMV), as well as the related caprine arthritis-encephalitis virus (CAEV), equine infectious anaemia virus (EIAV), feline immunodeficiency virus (FIV), Maedi visna virus (MW) and bovine immunodeficiency virus (BIV). In one embodiment, the lentiviral vector is derived from HIV-1, HIV-2, SIV, FIV, BIV, EIAV, CAEV or Visna lentivirus.

The lentivirus family differs from retroviruses in that lentiviruses have the capability to infect both dividing and non-dividing cells (Lewis et al (1992) EMBO J 11(8):3053-3058 and Lewis and Emerman (1994) J Virol 68 (1):510-516). In contrast, other retroviruses, such as MLV, are unable to infect non-dividing or slowly dividing cells such as those that make up, for example, muscle, brain, lung and liver tissue.

A lentiviral vector, as used herein, is a vector which comprises at least one component part derivable from a retrovirus. Preferably, that component part is involved in the biological mechanisms by which the vector infects or transduces target cells and expresses NOI.

The retroviral vector may be used to replicate the NOI in a compatible target cell *in vitro.* Thus, described herein is a method of making proteins *in vitro* by introducing a vector of the invention into a compatible target cell *in vitro* and growing the target cell under conditions which result in expression of the NOI. Protein and NOI may be recovered from the target cell by methods well known in the art. Suitable target cells include mammalian cell lines and other eukaryotic cell lines.

In some aspects the vectors may have "insulators" - genetic sequences that block the interaction between promoters and enhancers, and act as a barrier reducing read-through from an adjacent gene.

In one embodiment the insulator is present between one or more of the retroviral nucleic acid sequences to prevent promoter interference and read-thorough from adjacent genes. If the insulators are present in the vector between one or more of the retroviral nucleic acid sequences, then each of these insulated genes may be arranged as individual expression units.

The basic structure of retroviral and lentiviral genomes share many common features such as a 5' LTR and a 3' LTR, between or within which are located a packaging signal to enable the genome to be packaged, a primer binding site, integration sites to enable integration into a target cell genome and *gag*/*pol* and *env* genes encoding the packaging components - these are polypeptides required for the assembly of viral particles. Lentiviruses have additional features, such as the *rev* gene and RRE sequences in HIV, which enable the efficient export of RNA transcripts of the integrated provirus from the nucleus to the cytoplasm of an infected target cell.

In the provirus, these genes are flanked at both ends by regions called long terminal repeats (LTRs). The LTRs are responsible for proviral integration, and transcription. LTRs also serve as enhancer-promoter sequences and can control the expression of the viral genes.

The LTRs themselves are identical sequences that can be divided into three elements, which are called U3, R

and U5. U3 is derived from the sequence unique to the 3' end of the RNA. R is derived from a sequence repeated at both ends of the RNA and U5 is derived from the sequence unique to the 5' end of the RNA. The sizes of the three elements can vary considerably among different retroviruses.

In a typical retroviral vector as described herein, at least part of one or more protein coding regions essential for replication may be removed from the virus; for example, *gag*/*pol* and *env* may be absent or not functional. This makes the viral vector replication-defective.

The lentiviral vector may be derived from either a primate lentivirus (e.g. HIV-1) or a non-primate lentivirus (e.g. EIAV).

In general terms, a typical retroviral vector production system involves the separation of the viral genome from the essential viral packaging functions. These components are normally provided to the production cells on separate DNA expression cassettes (alternatively known as plasmids, expression plasmids, DNA constructs or expression constructs).

The vector genome comprises the NOI. Vector genomes typically require a packaging signal ($\psi$), the internal expression cassette harbouring the NOI, (optionally) a post-transcriptional element (PRE), typically a central polypurine tract (cppt), the 3'-ppu and a self-inactivating (SIN) LTR. The R-U5 regions are required for correct polyadenylation of both the vector genome RNA and NOI mRNA, as well as the process of reverse transcription. The vector genome may optionally include an open reading frame, as described in WO 2003/064665, which allows for vector production in the absence of rev.

The packaging functions include the *gag*/*pol* and *env* genes. These are required for the production of vector particles by the production cell. Providing these functions *in trans* to the genome facilitates the production of replication-defective viral vectors.

Production systems for gamma-retroviral vectors are typically 3-component systems requiring genome, *gag*/*pol* and *env* expression constructs. Production systems for HIV-1-based lentiviral vectors may additionally require the accessory gene *rev* to be provided and for the vector genome to include the rev-responsive element (RRE). EIAV-based lentiviral vectors do not require rev to be provided in *trans* if an open-reading frame (ORF) is present within the genome (see WO 2003/064665).

Usually both the "external" promoter (which drives the vector genome cassette) and "internal" promoter (which drives the NOI cassette) encoded within the vector genome cassette are strong eukaryotic or virus promoters, as are those driving the other vector system components.

Examples of such promoters include CMV, EF1$\alpha$, PGK, CAG, TK, SV40 and Ubiquitin promoters. Strong 'synthetic' promoters, such as those generated by DNA libraries (e.g. JeT promoter) may also be used to drive transcription. Alternatively, tissue-specific promoters such as rhodopsin (Rho), rhodopsin kinase (RhoK), cone-rod homeobox containing gene (CRX), neural retina-specific leucine zipper protein (NRL), Vitelliform Macular Dystrophy 2 (VMD2), Tyrosine hydroxylase, neuronal-specific neuronal-specific enolase (NSE) promoter, astrocyte-specific glial fibrillary acidic protein (GFAP) promoter, human $\alpha$1-antitrypsin (hAAT) promoter, phosphoenolpyruvate carboxykinase (PEPCK), liver fatty acid binding protein promoter, Flt-1 promoter, INF-$\beta$ promoter, Mb promoter, SP-B promoter, SYN1 promoter, WASP promoter, SV40 / hAlb promoter, SV40 / CD43, SV40 / CD45, NSE / RU5' promoter, ICAM-2 promoter, GPIIb promoter, GFAP promoter, Fibronectin promoter, Endoglin promoter, Elastase-1 promoter, Desmin promoter, CD68 promoter, CD14 promoter and B29 promoter may be used to drive transcription.

Production of retroviral vectors involves either the transient co-transfection of the production cells with these DNA components or use of stable production cell lines wherein all the components are stably integrated within the production cell genome (e.g. Stewart HJ, Fong-Wong L, Strickland I, Chipchase D, Kelleher M, Stevenson L, Thoree V, McCarthy J, Ralph GS, Mitrophanous KA and Radcliffe PA. (2011). Hum Gene Ther. Mar; 22 (3):357-69). An alternative approach is to use a stable packaging cell (into which the packaging components are stably integrated) and then transiently transfect in the vector genome plasmid as required (e.g. Stewart, H. J., M. A. Leroux-Carlucci, C. J. Sion, K. A. Mitrophanous and P. A. Radcliffe (2009). Gene Ther. Jun; 16 (6):805-14). It is also feasible that alternative, not complete, packaging cell lines could be generated (just one or two packaging components are stably integrated into the cell lines) and to generate vector the missing components are transiently transfected. The production cell may also express regulatory proteins such as a member of the tet repressor (TetR) protein group of transcription regulators (e.g.T-Rex, Tet-On, and Tet-Off), a member of the cumate inducible switch system group of transcription regulators (e.g. cumate repressor (CymR) protein), or an RNA-binding protein (e.g. TRAP - tryptophan-activated RNA-binding protein).

In one embodiment of the present invention, the viral vector is derived from EIAV. EIAV has the simplest genomic structure of the lentiviruses and is particularly preferred for use in the present invention. In addition to the *gag*/*pol* and *env genes,* EIAV encodes three other genes: *tat, rev,* and S2. *Tat* acts as a transcriptional activator of the viral LTR (Derse and Newbold (1993) Virology 194(2):530-536 and Maury et al (1994) Virology 200(2):632-642) and *rev* regulates and coordinates the expression of viral genes through rev-response elements (RRE) (Martarano et al. (1994) J Virol 68(5):3102-3111). The mechanisms of action of these two proteins are thought to be broadly similar to the analogous mechanisms in the primate viruses (Martarano et al. (1994) J Virol 68(5):3102-3111). The function of S2 is

unknown. In addition, an EIAV protein, Ttm, has been identified that is encoded by the first exon of *tat* spliced to the *env* coding sequence at the start of the transmembrane protein. In an alternative embodiment of the present invention the viral vector is derived from HIV: HIV differs from EIAV in that it does not encode S2 but unlike EIAV it encodes vif, vpr, vpu and nef.

The term "recombinant retroviral or lentiviral vector" (RRV) refers to a vector with sufficient retroviral genetic information to allow packaging of an RNA genome, in the presence of packaging components, into a viral particle capable of transducing a target cell. Transduction of the target cell may include reverse transcription and integration into the target cell genome. The RRV carries non-viral coding sequences which are to be delivered by the vector to the target cell. A RRV is incapable of independent replication to produce infectious retroviral particles within the target cell. Usually the RRV lacks a functional *gag*/*pol* and/or *env* gene, and/or other genes essential for replication.

Preferably the RRV vector of the present invention has a minimal viral genome.

As used herein, the term "minimal viral genome" means that the viral vector has been manipulated so as to remove the non-essential elements whilst retaining the elements essential to provide the required functionality to infect, transduce and deliver a NOI to a target cell. Further details of this strategy can be found in WO 1998/17815 and WO 99/32646. A minimal EIAV vector lacks *tat,* S2 genes, and optionally rev, and neither are these genes provided in *trans* in the production system. A minimal HIV vector lacks vif, vpr, vpu, tat and nef.

The expression plasmid used to produce the vector genome within a production cell may include transcriptional regulatory control sequences operably linked to the retroviral genome to direct transcription of the genome in a production cell/packaging cell. All 3rd generation lentiviral vectors are deleted in the 5' U3 enhancer-promoter region, and transcription of the vector genome RNA is driven by heterologous promoter such as another viral promoter, for example the CMV promoter, as discussed below. This feature enables vector production independently of tat. Some lentiviral vector genomes require additional sequences for efficient virus production. For example, particularly in the case of HIV, RRE sequences may be included. However, the requirement for RRE on the (separate) GagPol cassette (and dependence on *rev* which is provided in *trans)* may be reduced or eliminated by codon optimisation of the GagPol ORF. Further details of this strategy can be found in WO 2001/79518.

Alternative sequences which perform the same function as the *rev*/RRE system are also known. For example, a functional analogue of the *rev*/RRE system is found in the Mason Pfizer monkey virus. This is known as the constitutive transport element (CTE) and comprises an RRE-type sequence in the genome which is believed to interact with a factor in the infected cell. The cellular factor can be thought of as a rev analogue. Thus, CTE may be used as an alternative to the *rev*/RRE system. Any other functional equivalents of the Rev protein which are known or become available may be relevant to the invention. For example, it is also known that the Rex protein of HTLV-I can functionally replace the Rev protein of HIV-1. *Rev* and RRE may be absent or non-functional in the vector for use in the methods of the present invention; in the alternative *rev* and RRE, or functionally equivalent system, may be present.

As used herein, the term "functional substitute" means a protein or sequence having an alternative sequence which performs the same function as another protein or sequence. The term "functional substitute" is used interchangeably with "functional equivalent" and "functional analogue" herein with the same meaning.

***SIN vectors***

The viral vectors as described herein may be used in a self-inactivating (SIN) configuration in which the viral enhancer and promoter sequences have been deleted. SIN vectors can be generated and transduce non-dividing target cells *in vivo, ex vivo* or *in vitro* with an efficacy similar to that of non-SIN vectors. The transcriptional inactivation of the long terminal repeat (LTR) in the SIN provirus should prevent mobilisation of vRNA, and is a feature that further diminishes the likelihood of formation of replication-competent virus. This should also enable the regulated expression of genes from internal promoters by eliminating any *cis*-acting effects of the LTR.

By way of example, self-inactivating retroviral vector systems have been constructed by deleting the transcriptional enhancers or the enhancers and promoter in the U3 region of the 3' LTR. After a round of vector reverse transcription and integration, these changes are copied into both the 5' and the 3' LTRs producing a transcriptionally inactive 'provirus'. However, any promoter(s) internal to the LTRs in such vectors will still be transcriptionally active. This strategy has been employed to eliminate effects of the enhancers and promoters in the viral LTRs on transcription from internally placed genes. Such effects include increased transcription or suppression of transcription. This strategy can also be used to eliminate downstream transcription from the 3' LTR into genomic DNA. This is of particular concern in human gene therapy where it is important to prevent the adventitious activation of any endogenous oncogene. Yu et al., (1986) PNAS 83: 3194-98; Marty et al., (1990) Biochimie 72: 885-7; Naviaux et al., (1996) J. Virol. 70: 5701-5; Iwakuma et al., (1999) Virol. 261: 120-32; Deglon et al., (2000) Human Gene Therapy 11: 179-90. SIN lentiviral vectors are described in US 6,924,123 and US 7,056,699.

### *Replication-defective lentiviral vectors*

In the genome of a replication-defective lentiviral vector the sequences of *gag*/*pol* and/or *env* may be mutated and/or not functional.

In a typical lentiviral vector as described herein, at least part of one or more coding regions for proteins essential for virus replication may be removed from the vector. This makes the viral vector replication-defective. Portions of the viral genome may also be replaced by a NOI in order to generate a vector comprising an NOI which is capable of transducing a non-dividing target cell and/or integrating its genome into the target cell genome.

In one embodiment the lentiviral vectors are non-integrating vectors as described in WO 2006/010834 and WO 2007/071994.

In a further embodiment the vectors have the ability to deliver a sequence which is devoid of or lacking viral RNA. In a further embodiment a heterologous binding domain (heterologous to gag) located on the RNA to be delivered and a cognate binding domain on Gag or GagPol can be used to ensure packaging of the RNA to be delivered. Both of these vectors are described in WO 2007/072056.

### *Vector titre*

The skilled person will understand that there are a number of different methods of determining the titre of viral vectors. Titre is often described as transducing units/mL (TU/mL). Titre may be increased by increasing the number of infectious particles and by increasing the specific activity of a vector preparation.

### NOI and Polynucleotides

Polynucleotides of the invention may comprise DNA or RNA. They may be single-stranded or double-stranded. It will be understood by a skilled person that numerous different polynucleotides can encode the same polypeptide as a result of the degeneracy of the genetic code. In addition, it is to be understood that skilled persons may, using routine techniques, make nucleotide substitutions that do not affect the polypeptide sequence encoded by the polynucleotides of the invention to reflect the codon usage of any particular host organism in which the polypeptides of the invention are to be expressed.

The polynucleotides may be modified by any method available in the art. Such modifications may be carried out in order to enhance the *in vivo* activity or lifespan of the polynucleotides of the invention.

Polynucleotides such as DNA polynucleotides may be produced recombinantly, synthetically or by any means available to those of skill in the art. They may also be cloned by standard techniques.

Longer polynucleotides will generally be produced using recombinant means, for example using polymerase chain reaction (PCR) cloning techniques. This will involve making a pair of primers (e.g. of about 15 to 30 nucleotides) flanking the target sequence which it is desired to clone, bringing the primers into contact with mRNA or cDNA obtained from an animal or human cell, performing PCR under conditions which bring about amplification of the desired region, isolating the amplified fragment (e.g. by purifying the reaction mixture with an agarose gel) and recovering the amplified DNA. The primers may be designed to contain suitable restriction enzyme recognition sites so that the amplified DNA can be cloned into a suitable vector.

### Common Retroviral Vector Elements

#### *Promoters and Enhancers*

Expression of a NOI and polynucleotide may be controlled using control sequences for example transcription regulation elements or translation repression elements, which include promoters, enhancers and other expression regulation signals (e.g. tet repressor (TetR) system) or the Transgene Repression In vector Production cell system (TRIP) or other regulators of NOIs described herein.

Prokaryotic promoters and promoters functional in eukaryotic cells may be used. Tissue-specific or stimuli-specific promoters may be used. Chimeric promoters may also be used comprising sequence elements from two or more different promoters.

Suitable promoting sequences are strong promoters including those derived from the genomes of viruses, such as polyoma virus, adenovirus, fowlpox virus, bovine papilloma virus, avian sarcoma virus, cytomegalovirus (CMV), retrovirus and Simian Virus 40 (SV40), or from heterologous mammalian promoters, such as the actin promoter, EF1$\alpha$, CAG, TK, SV40, ubiquitin, PGK or ribosomal protein promoter. Alternatively, tissue-specific promoters such as rhodopsin (Rho), rhodopsin kinase (RhoK), cone-rod homeobox containing gene (CRX), neural retina-specific leucine zipper protein (NRL), Vitelliform Macular Dystrophy 2 (VMD2), Tyrosine hydroxylase, neuronal-specific neuronal-specific enolase (NSE)

promoter, astrocyte-specific glial fibrillary acidic protein (GFAP) promoter, human $\alpha$1-antitrypsin (hAAT) promoter, phosphoenolpyruvate carboxykinase (PEPCK), liver fatty acid binding protein promoter, Flt-1 promoter, INF-$\beta$ promoter, Mb promoter, SP-B promoter, SYN1 promoter, WASP promoter, SV40 / hAlb promoter, SV40 / CD43, SV40 / CD45, NSE / RU5' promoter, ICAM-2 promoter, GPIIb promoter, GFAP promoter, Fibronectin promoter, Endoglin promoter, Elastase-1 promoter, Desmin promoter, CD68 promoter, CD14 promoter and B29 promoter may be used to drive transcription.

Transcription of a NOI may be increased further by inserting an enhancer sequence into the vector. Enhancers are relatively orientation- and position-independent; however, one may employ an enhancer from a eukaryotic cell virus, such as the SV40 enhancer and the CMV early promoter enhancer. The enhancer may be spliced into the vector at a position 5' or 3' to the promoter, but is preferably located at a site 5' from the promoter.

The promoter can additionally include features to ensure or to increase expression in a suitable target cell. For example, the features can be conserved regions e.g. a Pribnow Box or a TATA box. The promoter may contain other sequences to affect (such as to maintain, enhance or decrease) the levels of expression of a nucleotide sequence. Suitable other sequences include the Sh1-intron or an ADH intron. Other sequences include inducible elements, such as temperature, chemical, light or stress inducible elements. Also, suitable elements to enhance transcription or translation may be present.

### *Regulators of NOIs*

A complicating factor in the generation of retroviral packaging/producer cell lines and retroviral vector production is that constitutive expression of certain retroviral vector components and NOIs are cytotoxic leading to death of cells expressing these components and therefore inability to produce vector. Therefore, the expression of these components (e.g. gag-pol and envelope proteins such as VSV-G) can be regulated. The expression of other non-cytotoxic vector components, e.g. rev, can also be regulated to minimise the metabolic burden on the cell. Thus the modular constructs or nucleotide sequences encoding vector components and/or cells as described herein may comprise cytotoxic and/or non-cytotoxic vector components associated with at least one regulatory element. As used herein, the term "regulatory element" refers to any element capable of affecting, either increasing or decreasing, the expression of an associated gene or protein. A regulatory element includes a gene switch system, transcription regulation element and translation repression element

A number of prokaryotic regulator systems have been adapted to generate gene switches in mammalian cells. Many retroviral packaging and producer cell lines have been controlled using gene switch systems (e.g. tetracycline and cumate inducible switch systems) thus enabling expression of one or more of the retroviral vector components to be switched on at the time of vector production. Gene switch systems include those of the (TetR) protein group of transcription regulators (e.g.T-Rex, Tet-On, and Tet-Off), those of the cumate inducible switch system group of transcription regulators (e.g. CymR protein) and those involving an RNA-binding protein (e.g. TRAP).

One such tetracycline-inducible system is the tetracycline repressor (TetR) system based on the T-REx™ system. By way of example, in such a system tetracycline operators ($TetO_2$) are placed in a position such that the first nucleotide is 10bp from the 3' end of the last nucleotide of the TATATAA element of the human cytomegalovirus major immediate early promoter (hCMVp) then TetR alone is capable of acting as a repressor (Yao F, Svensjo T, Winkler T, Lu M, Eriksson C, Eriksson E., 1998, Hum Gene Then, 9: 1939-1950). In such a system the expression of the NOI can be controlled by a CMV promoter into which two copies of the $TetO_2$ sequence have been inserted in tandem. TetR homodimers, in the absence of an inducing agent (tetracycline or its analogue doxycycline [dox]), bind to the $TetO_2$ sequences and physically block transcription from the upstream CMV promoter. When present, the inducing agent binds to the TetR homodimers, causing allosteric changes such that it can no longer bind to the $TetO_2$ sequences, resulting in gene expression. The TetR gene may be codon optimised as this was found to improve translation efficiency resulting in tighter control of $TetO_2$ controlled gene expression.

The TRIP system is described in WO 2015/092440 and provides another way of repressing expression of the NOI in the production cells during vector production. The TRAP-binding sequence (e.g. TRAP-tbs) interaction forms the basis for a transgene protein repression system for the production of retroviral vectors, when a constitutive and/or strong promoter, including a tissue-specific promoter, driving the transgene is desirable and particularly when expression of the transgene protein in production cells leads to reduction in vector titres and/or elicits an immune response *in vivo* due to viral vector delivery of transgene-derived protein (Maunder et al, Nat Commun. (2017) Mar 27; 8).

Briefly, the TRAP-tbs interaction forms a translational block, repressing translation of the transgene protein (Maunder et al, Nat Commun. (2017) Mar 27; 8). The translational block is only effective in production cells and as such does not impede the DNA- or RNA- based vector systems. The TRiP system is able to repress translation when the transgene protein is expressed from a constitutive and/or strong promoter, including a tissue-specific promoter from single- or multi cistronic mRNA. It has been demonstrated that unregulated expression of transgene protein can reduce vector titres and affect vector product quality. Repression of transgene protein for both transient and stable PaCL/PCL vector production systems is beneficial for production cells to prevent a reduction in vector titres: where toxicity or

molecular burden issues may lead to cellular stress; where transgene protein elicits an immune response *in vivo* due to viral vector delivery of transgene-derived protein; where the use of gene-editing transgenes may result in on/off target affects; where the transgene protein may affect vector and/or envelope glycoprotein exclusion.

***Envelope and Pseudotyping***

In one preferred aspect, the lentiviral vector as described herein has been pseudotyped. In this regard, pseudotyping can confer one or more advantages. For example, the *env* gene product of the HIV based vectors would restrict these vectors to infecting only cells that express a protein called CD4. But if the *env* gene in these vectors has been substituted with *env* sequences from other enveloped viruses, then they may have a broader infectious spectrum (Verma and Somia (1997) Nature 389(6648):239-242). By way of example, workers have pseudotyped an HIV based vector with the glycoprotein from VSV (Verma and Somia (1997) Nature 389(6648):239-242).

In another alternative, the Env protein may be a modified Env protein such as a mutant or engineered Env protein. Modifications may be made or selected to introduce targeting ability or to reduce toxicity or for another purpose (Valsesia-Wittman et al 1996 J Virol 70: 2056-64; Nilson et al (1996) Gene Ther 3(4):280-286; and Fielding et al (1998) Blood 91(5): 1802-1809 and references cited therein).

The vector may be pseudotyped with any molecule of choice.

As used herein, "env" shall mean an endogenous lentiviral envelope or a heterologous envelope, as described herein.

***VSV-G***

The envelope glycoprotein (G) of Vesicular stomatitis virus (VSV), a rhabdovirus, is an envelope protein that has been shown to be capable of pseudotyping certain enveloped viruses and viral vector virions.

Its ability to pseudotype MoMLV-based retroviral vectors in the absence of any retroviral envelope proteins was first shown by Emi et al. (1991) Journal of Virology 65:1202-1207. WO 1994/294440 teaches that retroviral vectors may be successfully pseudotyped with VSV-G. These pseudotyped VSV-G vectors may be used to transduce a wide range of mammalian cells. More recently, Abe et al. (1998) J Virol 72(8) 6356-6361 teach that non-infectious retroviral particles can be made infectious by the addition of VSV-G.

Burns et al. (1993) Proc. Natl. Acad. Sci. USA 90:8033-7 successfully pseudotyped the retrovirus MLV with VSV-G and this resulted in a vector having an altered host range compared to MLV in its native form. VSV-G pseudotyped vectors have been shown to infect not only mammalian cells, but also cell lines derived from fish, reptiles and insects (Burns *et* al. (1993) ibid). They have also been shown to be more efficient than traditional amphotropic envelopes for a variety of cell lines (Yee et al., (1994) Proc. Natl. Acad. Sci. USA 91:9564-9568, Emi et al. (1991) Journal of Virology 65:1202-1207). VSV-G protein can be used to pseudotype certain retroviruses because its cytoplasmic tail is capable of interacting with the retroviral cores.

The provision of a non-retroviral pseudotyping envelope such as VSV-G protein gives the advantage that vector particles can be concentrated to a high titre without loss of infectivity (Akkina et al. (1996) J. Virol. 70:2581-5). Retrovirus envelope proteins are apparently unable to withstand the shearing forces during ultracentrifugation, probably because they consist of two non-covalently linked subunits. The interaction between the subunits may be disrupted by the centrifugation. In comparison the VSV glycoprotein is composed of a single unit. VSV-G protein pseudotyping can therefore offer potential advantages for both efficient target cell infection/transduction and during manufacturing processes.

WO 2000/52188 describes the generation of pseudotyped retroviral vectors, from stable producer cell lines, having vesicular stomatitis virus-G protein (VSV-G) as the membrane-associated viral envelope protein, and provides a gene sequence for the VSV-G protein.

***Ross River Virus***

The Ross River viral envelope has been used to pseudotype a non-primate lentiviral vector (FIV) and following systemic administration predominantly transduced the liver (Kang et al., 2002, J. Virol., 76:9378-9388). Efficiency was reported to be 20-fold greater than obtained with VSV-G pseudotyped vector, and caused less cytotoxicity as measured by serum levels of liver enzymes suggestive of hepatotoxicity.

***Baculovirus GP64***

The baculovirus GP64 protein has been shown to be an alternative to VSV-G for viral vectors used in the large-scale production of high-titre virus required for clinical and commercial applications (Kumar M, Bradow BP, Zimmerberg J (2003) Hum Gene Ther. 14(1):67-77). Compared with VSV-G-pseudotyped vectors, GP64-pseudotyped vectors have

a similar broad tropism and similar native titres. Because, GP64 expression does not kill cells, HEK293T-based cell lines constitutively expressing GP64 can be generated.

### *Alternative envelopes*

Other envelopes which give reasonable titre when used to pseudotype EIAV include Mokola, Rabies, Ebola and LCMV (lymphocytic choriomeningitis virus). Intravenous infusion into mice of lentivirus pseudotyped with 4070A led to maximal gene expression in the liver.

### *Packaging sequence*

As utilized within the context of the present invention the term "packaging signal", which is referred to interchangeably as "packaging sequence" or "psi", is used in reference to the noncoding, cis-acting sequence required for encapsidation of retroviral RNA strands during viral particle formation. In HIV-1, this sequence has been mapped to loci extending from upstream of the major splice donor site (SD) to at least the gag start codon (some or all of the 5' sequence of gag to nucleotide 688 may be included). In EIAV the packaging signal comprises the R region into the 5' coding region of Gag.

As used herein, the term "extended packaging signal" or "extended packaging sequence" refers to the use of sequences around the psi sequence with further extension into the gag gene. The inclusion of these additional packaging sequences may increase the efficiency of insertion of vector RNA into viral particles.

Feline immunodeficiency virus (FIV) RNA encapsidation determinants have been shown to be discrete and non-continuous, comprising one region at the 5' end of the genomic mRNA (R-U5) and another region that mapped within the proximal 311 nt of gag (Kaye et al., J Virol. Oct;69(10):6588-92 (1995).

### *Internal ribosome entry site (IRES)*

Insertion of IRES elements allows expression of multiple coding regions from a single promoter (Adam et al (as above); Koo et al (1992) Virology 186:669-675; Chen et al 1993 J. Virol 67:2142-2148). IRES elements were first found in the non-translated 5' ends of picornaviruses where they promote cap-independent translation of viral proteins (Jang et al (1990) Enzyme 44: 292-309). When located between open reading frames in an RNA, IRES elements allow efficient translation of the downstream open reading frame by promoting entry of the ribosome at the IRES element followed by downstream initiation of translation.

A review on IRES is presented by Mountford and Smith (TIG May 1995 vol 11, No 5:179-184). A number of different IRES sequences are known including those from encephalomyocarditis virus (EMCV) (Ghattas, I.R., et al., Mol. Cell. Biol., 11 :5848-5859 (1991); BiP protein [Macejak and Sarnow, Nature 353:91 (1991)]; the Antennapedia gene of Drosophila (exons d and e) [Oh, et al., Genes & Development, 6:1643-1653 (1992)] as well as those in polio virus (PV) [Pelletier and Sonenberg, Nature 334: 320-325 (1988); see also Mountford and Smith, TIG 11, 179-184 (1985)].

IRES elements from PV, EMCV and swine vesicular disease virus have previously been used in retroviral vectors (Coffin et al, as above).

The term "IRES" includes any sequence or combination of sequences which work as or improve the function of an IRES. The IRES(s) may be of viral origin (such as EMCV IRES, PV IRES, or FMDV 2A-like sequences) or cellular origin (such as FGF2 IRES, NRF IRES, Notch 2 IRES or EIF4 IRES).

In order for the IRES to be capable of initiating translation of each polynucleotide it should be located between or prior to the polynucleotides in the modular construct.

The nucleotide sequences utilised for development of stable cell lines require the addition of selectable markers for selection of cells where stable integration has occurred. These selectable markers can be expressed as a single transcription unit within the nucleotide sequence or it may be preferable to use IRES elements to initiate translation of the selectable marker in a polycistronic message (Adam et al 1991 J.Virol. 65, 4985).

### *Genetic Orientation and Insulators*

It is well known that nucleic acids are directional and this ultimately affects mechanisms such as transcription and replication in the cell. Thus genes can have relative orientations with respect to one another when part of the same nucleic acid construct.

In certain embodiments of the present invention, at least two nucleic acid sequences present at the same locus in the cell or construct can be in a reverse and/or alternating orientations. In other words, in certain embodiments of the invention at this particular locus, the pair of sequential genes will not have the same orientation. This can help prevent both transcriptional and translational read-through when the region is expressed within the same physical location of

the host cell.

Having the alternating orientations benefits retroviral vector production when the nucleic acids required for vector production are based at the same genetic locus within the cell. This in turn can also improve the safety of the resulting constructs in preventing the generation of replication-competent retroviral vectors.

When nucleic acid sequences are in reverse and/or alternating orientations the use of insulators can prevent inappropriate expression or silencing of a NOI from its genetic surroundings.

The term "insulator" refers to a class of DNA sequence elements that when bound to insulator-binding proteins possess an ability to protect genes from surrounding regulator signals. There are two types of insulators: an enhancer blocking function and a chromatin barrier function. When an insulator is situated between a promoter and an enhancer, the enhancer-blocking function of the insulator shields the promoter from the transcription-enhancing influence of the enhancer (Geyer and Corces 1992; Kellum and Schedl 1992). The chromatin barrier insulators function by preventing the advance of nearby condensed chromatin which would lead to a transcriptionally active chromatin region turning into a transcriptionally inactive chromatin region and resulting in silencing of gene expression. Insulators which inhibit the spread of heterochromatin, and thus gene silencing, recruit enzymes involved in histone modifications to prevent this process (Yang J, Corces VG. 2011;110:43-76; Huang, Li et al. 2007; Dhillon, Raab et al. 2009). An insulator can have one or both of these functions and the chicken β-globin insulator (cHS4) is one such example. This insulator is the most extensively studied vertebrate insulator, is highly rich in G+C and has both enhancer-blocking and heterochromatic barrier functions (Chung J H, Whitely M, Felsenfeld G. Cell. 1993;74:505-514). Other such insulators with enhancer blocking functions are not limited to but include the following: human β-globin insulator 5 (HS5), human β-globin insulator 1 (HS1), and chicken β-globin insulator (cHS3) (Farrell CM1, West AG, Felsenfeld G., Mol Cell Biol. 2002 Jun;22(11):3820-31; J Ellis et al. EMBO J. 1996 Feb 1; 15(3): 562-568). In addition to reducing unwanted distal interactions the insulators also help to prevent promoter interference (i.e. where the promoter from one transcription unit impairs expression of an adjacent transcription unit) between adjacent retroviral nucleic acid sequences. If the insulators are used between each of the retroviral vector nucleic acid sequences, then the reduction of direct read-through will help prevent the formation of replication-competent retroviral vector particles.

The insulator may be present between each of the retroviral nucleic acid sequences. In one embodiment, the use of insulators prevents promoter-enhancer interactions from one NOI expression cassette interacting with another NOI expression cassette in a nucleotide sequence encoding vector components.

An insulator may be present between the vector genome and *gag-pol* sequences. This therefore limits the likelihood of the production of a replication-competent retroviral vector and 'wild-type' like RNA transcripts, improving the safety profile of the construct. The use of insulator elements to improve the expression of stably integrated multigene vectors is cited in Moriarity et al, Nucleic Acids Res. 2013 Apr;41 (8):e92.

### *Vector titre*

The skilled person will understand that there are a number of different methods of determining the titre of lentiviral vectors. Titre is often described as transducing units/mL (TU/mL). Titre may be increased by increasing the number of vector particles and by increasing the specific activity of a vector preparation.

## Therapeutic Use

The lentiviral vector as described herein or a cell or tissue transduced with the lentiviral vector as described herein may be used in medicine.

In addition, the lentiviral vector as described herein, a production cell of the invention or a cell or tissue transduced with the lentiviral vector as described herein may be used for the preparation of a medicament to deliver a nucleotide of interest to a target site in need of the same. Such uses of the lentiviral vector or transduced cell of the invention may be for therapeutic or diagnostic purposes, as described previously.

Accordingly, there is provided a cell transduced by the lentiviral vector as described herein.

A "cell transduced by a viral vector particle" is to be understood as a cell, in particular a target cell, into which the nucleic acid carried by the viral vector particle has been transferred.

In a preferred embodiment, the nucleotide of interest gives rise to a therapeutic effect.

"Target cell" is to be understood as a cell in which it is desired to express the NOI. The NOI may be introduced into the target cell using a viral vector of the present invention. Delivery to the target cell may be performed *in vivo, ex vivo* or *in vitro.*

The NOI may have a therapeutic or diagnostic application. Suitable NOIs include, but are not limited to sequences encoding enzymes, co-factors, cytokines, chemokines, hormones, antibodies, anti-oxidant molecules, engineered immunoglobulin-like molecules, single chain antibodies, fusion proteins, immune co-stimulatory molecules, immunomodulatory molecules, chimeric antigen receptors a transdomain negative mutant of a target protein, toxins, conditional

toxins, antigens, transcription factors, structural proteins, reporter proteins, subcellular localization signals, tumour suppressor proteins, growth factors, membrane proteins, receptors, vasoactive proteins and peptides, anti-viral proteins and ribozymes, and derivatives thereof (such as derivatives with an associated reporter group). The NOIs may also encode micro-RNA. Without wishing to be bound by theory, it is believed that the processing of micro-RNA will be inhibited by TRAP.

In one embodiment, the NOI may be useful in the treatment of a neurodegenerative disorder.

In another embodiment, the NOI may be useful in the treatment of Parkinson's disease.

In another embodiment, the NOI may encode an enzyme or enzymes involved in dopamine synthesis. For example, the enzyme may be one or more of the following: tyrosine hydroxylase, GTP-cyclohydrolase I and/or aromatic amino acid dopa decarboxylase. The sequences of all three genes are available (GenBank® Accession Nos. X05290, U19523 and M76180, respectively).

In another embodiment, the NOI may encode the vesicular monoamine transporter 2 (VMAT2). In an alternative embodiment the viral genome may comprise a NOI encoding aromatic amino acid dopa decarboxylase and a NOI encoding VMAT2. Such a genome may be used in the treatment of Parkinson's disease, in particular in conjunction with peripheral administration of L-DOPA.

In another embodiment the NOI may encode a therapeutic protein or combination of therapeutic proteins.

In another embodiment, the NOI may encode a protein or proteins selected from the group consisting of glial cell derived neurotophic factor (GDNF), brain derived neurotrophic factor (BDNF), ciliary neurotrophic factor (CNTF), neurotrophin-3 (NT-3), acidic fibroblast growth factor (aFGF), basic fibroblast growth factor (bFGF), interleukin-1 beta (IL-1$\beta$), tumor necrosis factor alpha (TNF-$\alpha$), insulin growth factor-2, VEGF-A, VEGF-B, VEGF-C/VEGF-2, VEGF-D, VEGF-E, PDGF-A, PDGF-B, hetero- and homo-dimers of PDFG-A and PDFG-B.

In another embodiment, the NOI may encode an anti-angiogenic protein or anti-angiogenic proteins selected from the group consisting of angiostatin, endostatin, platelet factor 4, pigment epithelium derived factor (PEDF), placental growth factor, restin, interferon-$\alpha$, interferon-inducible protein, gro-beta and tubedown-1, interleukin(IL)-1, IL-12, retinoic acid, anti-VEGF antibodies or fragments /variants thereof such as aflibercept, thrombospondin, VEGF receptor proteins such as those described in US 5,952,199 and US 6,100,071, and anti-VEGF receptor antibodies.

In another embodiment, the NOI may encode anti-inflammatory proteins, antibodies or fragment/variants of proteins or antibodies selected from the group consisting of NF-kB inhibitors, IL1beta inhibitors, TGFbeta inhibitors, IL-6 inhibitors, IL-23 inhibitors, IL-18 inhibitors, Tumour necrosis factor alpha and Tumour necrosis factor beta, Lymphotoxin alpha and Lymphotoxin beta, LIGHT inhibitors, alpha synuclein inhibitors, Tau inhibitors, beta amyloid inhibitors, IL-17 inhibitors,

In another embodiment the NOI may encode cystic fibrosis transmembrane conductance regulator (CFTR).

In another embodiment the NOI may encode a protein normally expressed in an ocular cell.

In another embodiment, the NOI may encode a protein normally expressed in a photoreceptor cell and/or retinal pigment epithelium cell.

In another embodiment, the NOI may encode a protein selected from the group comprising RPE65, arylhydrocarbon-interacting receptor protein like 1 (AIPL1), CRB1, lecithin retinal acetyltransferace (LRAT), photoreceptor-specific homeo box (CRX), retinal guanylate cyclise (GUCY2D), RPGR interacting protein 1 (RPGRIP1), LCA2, LCA3, LCA5, dystrophin, PRPH2, CNTF, ABCR/ABCA4, EMP1, TIMP3, MERTK, ELOVL4, MYO7A, USH2A, VMD2, RLBP1, COX-2, FPR, harmonin, Rab escort protein 1, CNGB2, CNGA3, CEP 290, RPGR, RS1, RP1, PRELP, glutathione pathway enzymes and opticin.

In other embodiments, the NOI may encode the human clotting Factor VIII or Factor IX.

In other embodiments, the NOI may encode protein or proteins involved in metabolism selected from the group comprising phenylalanine hydroxylase (PAH), Methylmalonyl CoA mutase, Propionyl CoA carboxylase, Isovaleryl CoA dehydrogenase, Branched chain ketoacid dehydrogenase complex, Glutaryl CoA dehydrogenase, Acetyl CoA carboxylase, propionyl CoA carboxylase, 3 methyl crotonyl CoA carboxylase, pyruvate carboxylase, carbamoyl-phophate synthase ammonia, ornithine transcarbamylase, glucosylceramidase beta, alpha galactosidase A, glucosylceramidase beta, cystinosin, glucosamine(N-acetyl)-6-sulfatase, N-acetyl-alpha-glucosaminidase, N-sulfoglucosamine sulfohydrolase, Galactosamine-6 sulfatase, arylsulfatase A, cytochrome B-245 beta, *ABCD1,* ornithine carbamoyltransferase, argininosuccinate synthase, argininosuccinate lysase, arginase 1, alanine glycoxhylate amino transferase, ATP-binding cassette, sub-family B members.

In other embodiments, the NOI may encode a chimeric antigen receptor (CAR) or a T cell receptor (TCR). In one embodiment, the CAR is an anti-5T4 CAR. In other embodiments, the NOI may encode B-cell maturation antigen (BCMA), CD19, CD22, CD20, CD47, CD138, CD30, CD33, CD123, CD70, prostate specific membrane antigen (PSMA), Lewis Y antigen (LeY), Tyrosine-protein kinase transmembrane receptor (ROR1), Mucin 1, cell surface associated (Muc1), Epithelial cell adhesion molecule (EpCAM), endothelial growth factor receptor (EGFR), insulin, protein tyrosine phosphatase, non-receptor type 22, interleukin 2 receptor alpha, interferon induced with helicase C domain 1, human epidermal growth factor receptor (HER2), glypican 3 (GPC3), disialoganglioside (GD2), mesiothelin, vesicular endothelial

growth factor receptor 2 (VEGFR2).

In other embodiments, the NOI may encode a chimeric antigen receptor (CAR) against NKG2D ligands selected from the group comprising ULBP1, 2 and 3, H60, Rae-1a, b, g, d, MICA, MICB.

In further embodiments the NOI may encode SGSH, SUMF1, GAA, the common gamma chain (CD132), adenosine deaminase, WAS protein, globins, alpha galactosidase A, δ-aminolevulinate (ALA) synthase, δ-aminolevulinate dehydratase (ALAD), Hydroxymethylbilane (HMB) synthase, Uroporphyrinogen (URO) synthase, Uroporphyrinogen (URO) decarboxylase, Coproporphyrinogen (COPRO) oxidase, Protoporphyrinogen (PROTO) oxidase, Ferrochelatase, α-L-iduronidase, Iduronate sulfatase, Heparan sulfamidase, N-acetylglucosaminidase, Heparan-α-glucosaminide N-acetyltransferase, 3 N-acetylglucosamine 6-sulfatase, Galactose-6-sulfate sulfatase, β-galactosidase, N-acetylgalactosamine-4-sulfatase, β-glucuronidase and Hyaluronidase.

In addition to the NOI the vector may also comprise or encode a siRNA, shRNA, or regulated shRNA. (Dickins et al. (2005) Nature Genetics 37: 1289-1295, Silva et al. (2005) Nature Genetics 37:1281-1288).

*Indications*

The vectors, including retroviral and AAV vectors, according to the present invention may be used to deliver one or more NOI(s) useful in the treatment of the disorders listed in WO 1998/05635, WO 1998/07859, WO 1998/09985. The nucleotide of interest may be DNA or RNA. Examples of such diseases are given below:

A disorder which responds to cytokine and cell proliferation/differentiation activity; immunosuppressant or immunostimulant activity (e.g. for treating immune deficiency, including infection with human immunodeficiency virus, regulation of lymphocyte growth; treating cancer and many autoimmune diseases, and to prevent transplant rejection or induce tumour immunity); regulation of haematopoiesis (e.g. treatment of myeloid or lymphoid diseases); promoting growth of bone, cartilage, tendon, ligament and nerve tissue (e.g. for healing wounds, treatment of burns, ulcers and periodontal disease and neurodegeneration); inhibition or activation of follicle-stimulating hormone (modulation of fertility); chemotactic/chemokinetic activity (e.g. for mobilising specific cell types to sites of injury or infection); haemostatic and thrombolytic activity (e.g. for treating haemophilia and stroke); anti-inflammatory activity (for treating, for example, septic shock or Crohn's disease); macrophage inhibitory and/or T cell inhibitory activity and thus, anti-inflammatory activity; anti-immune activity (i.e. inhibitory effects against a cellular and/or humoral immune response, including a response not associated with inflammation); inhibition of the ability of macrophages and T cells to adhere to extracellular matrix components and fibronectin, as well as up-regulated fas receptor expression in T cells.

Malignancy disorders, including cancer, leukaemia, benign and malignant tumour growth, invasion and spread, angiogenesis, metastases, ascites and malignant pleural effusion.

Autoimmune diseases including arthritis, including rheumatoid arthritis, hypersensitivity, allergic reactions, asthma, systemic lupus erythematosus, collagen diseases and other diseases.

Vascular diseases including arteriosclerosis, atherosclerotic heart disease, reperfusion injury, cardiac arrest, myocardial infarction, vascular inflammatory disorders, respiratory distress syndrome, cardiovascular effects, peripheral vascular disease, migraine and aspirin-dependent anti-thrombosis, stroke, cerebral ischaemia, ischaemic heart disease or other diseases.

Diseases of the gastrointestinal tract including peptic ulcer, ulcerative colitis, Crohn's disease and other diseases.

Hepatic diseases including hepatic fibrosis, liver cirrhosis.

Inherited metabolic disorders including phenylketonuria PKU, Wilson disease, organic acidemias, urea cycle disorders, cholestasis, and other diseases.

Renal and urologic diseases including thyroiditis or other glandular diseases, glomerulonephritis or other diseases.

Ear, nose and throat disorders including otitis or other oto-rhino-laryngological diseases, dermatitis or other dermal diseases.

Dental and oral disorders including periodontal diseases, periodontitis, gingivitis or other dental/oral diseases.

Testicular diseases including orchitis or epididimo-orchitis, infertility, orchidal trauma or other testicular diseases.

Gynaecological diseases including placental dysfunction, placental insufficiency, habitual abortion, eclampsia, pre-eclampsia, endometriosis and other gynaecological diseases.

Ophthalmologic disorders such as Leber Congenital Amaurosis (LCA) including LCA10, posterior uveitis, intermediate uveitis, anterior uveitis, conjunctivitis, chorioretinitis, uveoretinitis, optic neuritis, glaucoma, including open angle glaucoma and juvenile congenital glaucoma, intraocular inflammation, e.g. retinitis or cystoid macular oedema, sympathetic ophthalmia, scleritis, retinitis pigmentosa, macular degeneration including age related macular degeneration (AMD) and juvenile macular degeneration including Best Disease, Best vitelliform macular degeneration, Stargardt's Disease, Usher's syndrome, Doyne's honeycomb retinal dystrophy, Sorby's Macular Dystrophy, Juvenile retinoschisis, Cone-Rod Dystrophy, Corneal Dystrophy, Fuch's Dystrophy, Leber's congenital amaurosis, Leber's hereditary optic neuropathy (LHON), Adie syndrome, Oguchi disease, degenerative fondus disease, ocular trauma, ocular inflammation caused by infection, proliferative vitreo-retinopathies, acute ischaemic optic neuropathy, excessive scarring, e.g. following glaucoma filtration operation, reaction against ocular implants, corneal transplant graft rejection, and other ophthalmic diseases, such as diabetic macular oedema, retinal vein occlusion, RLBP1-associated retinal dystrophy, choroideremia and achromatopsia.

Neurological and neurodegenerative disorders including Parkinson's disease, complication and/or side effects from treatment of Parkinson's disease, AIDS-related dementia complex HIV-related encephalopathy, Devic's disease, Sydenham chorea, Alzheimer's disease and other degenerative diseases, conditions or disorders of the CNS, strokes, post-polio syndrome, psychiatric disorders, myelitis, encephalitis, subacute sclerosing pan-encephalitis, encephalomyelitis, acute neuropathy, subacute neuropathy, chronic neuropathy, Fabry disease, Gaucher disease, Cystinosis, Pompe disease, metachromatic leukodystrophy, Wiscott Aldrich Syndrome, adrenoleukodystrophy, beta-thalassemia, sickle cell disease, Guillaim-Barre syndrome, Sydenham chorea, myasthenia gravis, pseudo-tumour cerebri, Down's Syndrome, Huntington's disease, CNS compression or CNS trauma or infections of the CNS, muscular atrophies and dystrophies, diseases, conditions or disorders of the central and peripheral nervous systems, motor neuron disease including amyotropic lateral sclerosis, spinal muscular atropy, spinal cord and avulsion injury.

Other diseases and conditions such as cystic fibrosis, mucopolysaccharidosis including Sanfilipo syndrome A, Sanfilipo syndrome B, Sanfilipo syndrome C, Sanfilipo syndrome D, Hunter syndrome, Hurler-Scheie syndrome, Morquio syndrome, ADA-SCID, X-linked SCID, X-linked chronic granulomatous disease, porphyria, haemophilia A, haemophilia B, post-traumatic inflammation, haemorrhage, coagulation and acute phase response, cachexia, anorexia, acute infection, septic shock, infectious diseases, diabetes mellitus, complications or side effects of surgery, bone marrow transplantation or other transplantation complications and/or side effects, complications and side effects of gene therapy, e.g. due to infection with a viral carrier, or AIDS, to suppress or inhibit a humoral and/or cellular immune response, for the prevention and/or treatment of graft rejection in cases of transplantation of natural or artificial cells, tissue and organs such as cornea, bone marrow, organs, lenses, pacemakers, natural or artificial skin tissue.

***siRNA, micro-RNA and shRNA***

In certain other embodiments, the NOI comprises a micro-RNA. Micro-RNAs are a very large group of small RNAs produced naturally in organisms, at least some of which regulate the expression of target genes. Founding members of the micro-RNA family are *let-7* and *lin-4.* The *let-7* gene encodes a small, highly conserved RNA species that regulates the expression of endogenous protein-coding genes during worm development. The active RNA species is transcribed initially as an ~70 nt precursor, which is post-transcriptionally processed into a mature ~21 nt form. Both *let-7* and *lin-4* are transcribed as hairpin RNA precursors which are processed to their mature forms by Dicer enzyme.

In addition to the NOI the vector may also comprise or encode a siRNA, shRNA, or regulated shRNA (Dickins et al. (2005) Nature Genetics 37: 1289-1295, Silva et al. (2005) Nature Genetics 37:1281-1288).

Post-transcriptional gene silencing (PTGS) mediated by double-stranded RNA (dsRNA) is a conserved cellular defence mechanism for controlling the expression of foreign genes. It is thought that the random integration of elements such as transposons or viruses causes the expression of dsRNA which activates sequence-specific degradation of homologous single-stranded mRNA or viral genomic RNA. The silencing effect is known as RNA interference (RNAi) (Ralph et al. (2005) Nature Medicine 11:429-433). The mechanism of RNAi involves the processing of long dsRNAs into duplexes of about 21-25 nucleotide (nt) RNAs. These products are called small interfering or silencing RNAs (siRNAs) which are the sequence-specific mediators of mRNA degradation. In differentiated mammalian cells, dsRNA >30 bp has been found to activate the interferon response leading to shut-down of protein synthesis and non-specific mRNA degradation (Stark et al., Annu Rev Biochem 67:227-64 (1998)). However this response can be bypassed by using 21 nt siRNA duplexes (Elbashir et al., EMBO J. Dec 3;20(23):6877-88 (2001), Hutvagner et al., Science.Aug 3,

293(5531):834-8. Eupub Jul 12 (2001)) allowing gene function to be analysed in cultured mammalian cells.

## Pharmaceutical compositions

The present disclosure provides a pharmaceutical composition comprising the lentiviral vector as described herein or a cell or tissue transduced with the viral vector as described herein, in combination with a pharmaceutically acceptable carrier, diluent or excipient.

The present disclosure provides a pharmaceutical composition for treating an individual by gene therapy, wherein the composition comprises a therapeutically effective amount of a lentiviral vector. The pharmaceutical composition may be for human or animal usage.

The composition may comprise a pharmaceutically acceptable carrier, diluent, excipient or adjuvant. The choice of pharmaceutical carrier, excipient or diluent can be made with regard to the intended route of administration and standard pharmaceutical practice. The pharmaceutical compositions may comprise, or be in addition to, the carrier, excipient or diluent any suitable binder(s), lubricant(s), suspending agent(s), coating agent(s), solubilising agent(s) and other carrier agents that may aid or increase vector entry into the target site (such as for example a lipid delivery system).

Where appropriate, the composition can be administered by any one or more of inhalation; in the form of a suppository or pessary; topically in the form of a lotion, solution, cream, ointment or dusting powder; by use of a skin patch; orally in the form of tablets containing excipients such as starch or lactose, or in capsules or ovules either alone or in admixture with excipients, or in the form of elixirs, solutions or suspensions containing flavouring or colouring agents; or they can be injected parenterally, for example intracavernosally, intravenously, intramuscularly, intracranially, intraoccularly intraperitoneally, or subcutaneously. For parenteral administration, the compositions may be best used in the form of a sterile aqueous solution which may contain other substances, for example enough salts or monosaccharides to make the solution isotonic with blood. For buccal or sublingual administration, the compositions may be administered in the form of tablets or lozenges which can be formulated in a conventional manner.

The lentiviral vector as described herein may also be used to transduce target cells or target tissue *ex vivo* prior to transfer of said target cell or tissue into a patient in need of the same. An example of such cell may be autologous T cells and an example of such tissue may be a donor cornea.

## Variants, derivatives, analogues, homologues and fragments

In addition to the specific proteins and nucleotides mentioned herein, the present invention also encompasses the use of variants, derivatives, analogues, homologues and fragments thereof.

In the context of the present invention, a variant of any given sequence is a sequence in which the specific sequence of residues (whether amino acid or nucleic acid residues) has been modified in such a manner that the polypeptide or polynucleotide in question retains at least one of its endogenous functions. A variant sequence can be obtained by addition, deletion, substitution, modification, replacement and/or variation of at least one residue present in the naturally-occurring protein.

The term "derivative" as used herein, in relation to proteins or polypeptides of the present invention includes any substitution of, variation of, modification of, replacement of, deletion of and/or addition of one (or more) amino acid residues from or to the sequence providing that the resultant protein or polypeptide retains at least one of its endogenous functions.

The term "analogue" as used herein, in relation to polypeptides or polynucleotides includes any mimetic, that is, a chemical compound that possesses at least one of the endogenous functions of the polypeptides or polynucleotides which it mimics.

Typically, amino acid substitutions may be made, for example from 1, 2 or 3 to 10 or 20 substitutions provided that the modified sequence retains the required activity or ability. Amino acid substitutions may include the use of non-naturally occurring analogues.

Proteins used in the present invention may also have deletions, insertions or substitutions of amino acid residues which produce a silent change and result in a functionally equivalent protein. Deliberate amino acid substitutions may be made on the basis of similarity in polarity, charge, solubility, hydrophobicity, hydrophilicity and/or the amphipathic nature of the residues as long as the endogenous function is retained. For example, negatively charged amino acids include aspartic acid and glutamic acid; positively charged amino acids include lysine and arginine; and amino acids with uncharged polar head groups having similar hydrophilicity values include asparagine, glutamine, serine, threonine and tyrosine.

Conservative substitutions may be made, for example according to the table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R H |
| AROMATIC | | F W Y |

The term "homologue" means an entity having a certain homology with the wild type amino acid sequence and the wild type nucleotide sequence. The term "homology" can be equated with "identity".

In the present context, a homologous sequence is taken to include an amino acid sequence which may be at least 50%, 55%, 65%, 75%, 85% or 90% identical, preferably at least 95%, 97 or 99% identical to the subject sequence. Typically, the homologues will comprise the same active sites etc. as the subject amino acid sequence. Although homology can also be considered in terms of similarity (i.e. amino acid residues having similar chemical properties/functions), in the context of the present invention it is preferred to express homology in terms of sequence identity.

In the present context, a homologous sequence is taken to include a nucleotide sequence which may be at least 50%, 55%, 65%, 75%, 85% or 90% identical, preferably at least 95%, 97%, 98% or 99% identical to the subject sequence. Although homology can also be considered in terms of similarity, in the context of the present invention it is preferred to express homology in terms of sequence identity.

Homology comparisons can be conducted by eye, or more usually, with the aid of readily available sequence comparison programs. These commercially available computer programs can calculate percentage homology or identity between two or more sequences.

Percentage homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion in the nucleotide sequence may cause the following codons to be put out of alignment, thus potentially resulting in a large reduction in percent homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible, reflecting higher relatedness between the two compared sequences, will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example, when using the GCG Wisconsin Bestfit package the default gap penalty for amino acid sequences is -12 for a gap and -4 for each extension.

Calculation of maximum percentage homology therefore firstly requires the production of an optimal alignment, taking into consideration gap penalties. A suitable computer program for carrying out such an alignment is the GCG Wisconsin Bestfit package (University of Wisconsin, U.S.A.; Devereux et al. (1984) Nucleic Acids Research 12:387). Examples of other software that can perform sequence comparisons include, but are not limited to, the BLAST package (see Ausubel et al. (1999) ibid - Ch. 18), FASTA (Atschul et al. (1990) J. Mol. Biol. 403-410) and the GENEWORKS suite of comparison tools. Both BLAST and FASTA are available for offline and online searching (see Ausubel et al. (1999) ibid, pages 7-58 to 7-60). However, for some applications, it is preferred to use the GCG Bestfit program. Another tool, called BLAST 2 Sequences is also available for comparing protein and nucleotide sequences (see FEMS Microbiol Lett (1999) 174(2):247-50; FEMS Microbiol Lett (1999) 177(1):187-8).

Although the final percentage homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such

a matrix commonly used is the BLOSUM62 matrix - the default matrix for the BLAST suite of programs. GCG Wisconsin programs generally use either the public default values or a custom symbol comparison table if supplied (see user manual for further details). For some applications, it is preferred to use the public default values for the GCG package, or in the case of other software, the default matrix, such as BLOSUM62.

Once the software has produced an optimal alignment, it is possible to calculate percentage homology, preferably percentage sequence identity. The software usually does this as part of the sequence comparison and generates a numerical result.

"Fragments" are also variants and the term typically refers to a selected region of the polypeptide or polynucleotide that is of interest either functionally or, for example, in an assay. "Fragment" thus refers to an amino acid or nucleic acid sequence that is a portion of a full-length polypeptide or polynucleotide.

Such variants may be prepared using standard recombinant DNA techniques such as site-directed mutagenesis. Where insertions are to be made, synthetic DNA encoding the insertion together with 5' and 3' flanking regions corresponding to the naturally-occurring sequence either side of the insertion site may be made. The flanking regions will contain convenient restriction sites corresponding to sites in the naturally-occurring sequence so that the sequence may be cut with the appropriate enzyme(s) and the synthetic DNA ligated into the break. The DNA is then expressed in accordance with the invention to make the encoded protein. These methods are only illustrative of the numerous standard techniques known in the art for manipulation of DNA sequences and other known techniques may also be used.

All variants, fragments or homologues of the regulatory protein suitable for use in the cells and/or modular constructs of the invention will retain the ability to bind the cognate binding site of the NOI such that translation of the NOI is repressed or prevented in a viral vector production cell.

All variant fragments or homologues of the binding site will retain the ability to bind the cognate RNA-binding protein, such that translation of the NOI is repressed or prevented in a viral vector production cell.

### *Codon optimisation*

The polynucleotides used in the present invention (including the NOI and/or components of the vector production system) may be codon-optimised. Codon optimisation has previously been described in WO 1999/41397 and WO 2001/79518. Different cells differ in their usage of particular codons. This codon bias corresponds to a bias in the relative abundance of particular tRNAs in the cell type. By altering the codons in the sequence so that they are tailored to match with the relative abundance of corresponding tRNAs, it is possible to increase expression. By the same token, it is possible to decrease expression by deliberately choosing codons for which the corresponding tRNAs are known to be rare in the particular cell type. Thus, an additional degree of translational control is available.

Many viruses, including retroviruses, use a large number of rare codons and changing these to correspond to commonly used mammalian codons, increases expression of a gene of interest, e.g. a NOI or packaging components in mammalian production cells, can be achieved. Codon usage tables are known in the art for mammalian cells, as well as for a variety of other organisms.

Codon optimisation of viral vector components has a number of other advantages. By virtue of alterations in their sequences, the nucleotide sequences encoding the packaging components of the viral particles required for assembly of viral particles in the producer cells/packaging cells have RNA instability sequences (INS) eliminated from them. At the same time, the amino acid sequence coding sequence for the packaging components is retained so that the viral components encoded by the sequences remain the same, or at least sufficiently similar that the function of the packaging components is not compromised. In lentiviral vectors codon optimisation also overcomes the Rev/RRE requirement for export, rendering optimised sequences Rev-independent. Codon optimisation also reduces homologous recombination between different constructs within the vector system (for example between the regions of overlap in the *gag-pol* and *env* open reading frames). The overall effect of codon optimisation is therefore a notable increase in viral titre and improved safety.

In one embodiment only codons relating to INS are codon optimised. However, in a much more preferred and practical embodiment, the sequences are codon optimised in their entirety, with some exceptions, for example the sequence encompassing the frameshift site of *gag-pol* (see below).

The *gag-pol* gene of lentiviral vectors comprises two overlapping reading frames encoding the *gag-pol* proteins. The expression of both proteins depends on a frameshift during translation. This frameshift occurs as a result of ribosome "slippage" during translation. This slippage is thought to be caused at least in part by ribosome-stalling RNA secondary structures. Such secondary structures exist downstream of the frameshift site in the *gag-pol* gene. For HIV, the region of overlap extends from nucleotide 1222 downstream of the beginning of *gag* (wherein nucleotide 1 is the A of the gag ATG) to the end of *gag* (nt 1503). Consequently, a 281 bp fragment spanning the frameshift site and the overlapping region of the two reading frames is preferably not codon optimised. Retaining this fragment will enable more efficient expression of the Gag-Pol proteins. For EIAV the beginning of the overlap has been taken to be nt 1262 (where nucleotide 1 is the A of the gag ATG) and the end of the overlap to be nt 1461. In order to ensure that the frameshift site and the

*gag-pol* overlap are preserved, the wild type sequence has been retained from nt 1156 to 1465.

Derivations from optimal codon usage may be made, for example, in order to accommodate convenient restriction sites, and conservative amino acid changes may be introduced into the Gag-Pol proteins.

In one embodiment, codon optimisation is based on lightly expressed mammalian genes. The third and sometimes the second and third base may be changed.

Due to the degenerate nature of the genetic code, it will be appreciated that numerous *gag-pol* sequences can be achieved by a skilled worker. Also there are many retroviral variants described which can be used as a starting point for generating a codon-optimised *gag-pol* sequence. Lentiviral genomes can be quite variable. For example, there are many quasi-species of HIV-1 which are still functional. This is also the case for EIAV. These variants may be used to enhance particular parts of the transduction process. Examples of HIV-1 variants may be found at the HIV Databases operated by Los Alamos National Security, LLC at http://hiv-web.lanl.gov. Details of EIAV clones may be found at the National Center for Biotechnology Information (NCBI) database located at http://www.ncbi.nlm.nih.gov.

The strategy for codon-optimised *gag-pol* sequences can be used in relation to any retrovirus. This would apply to all lentiviruses, including EIAV, FIV, BIV, CAEV, VMR, SIV, HIV-1 and HIV-2. In addition, this method could be used to increase expression of genes from HTLV-1, HTLV-2, HFV, HSRV and human endogenous retroviruses (HERV), MLV and other retroviruses.

Codon optimisation can render *gag-pol* expression Rev-independent. In order to enable the use of anti-rev or RRE factors in the lentiviral vector, however, it would be necessary to render the viral vector generation system totally Rev/RRE-independent. Thus, the genome also needs to be modified. This is achieved by optimising vector genome components. Advantageously, these modifications also lead to the production of a safer system absent of all additional proteins both in the producer and in the transduced cell.

This disclosure is not limited by the exemplary methods and materials disclosed herein, and any methods and materials similar or equivalent to those described herein can be used in the practice or testing of embodiments of this disclosure. Numeric ranges are inclusive of the numbers defining the range. Unless otherwise indicated, any nucleic acid sequences are written left to right in 5' to 3' orientation; amino acid sequences are written left to right in amino to carboxy orientation, respectively.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within this disclosure. The upper and lower limits of these smaller ranges may independently be included or excluded in the range, and each range where either, neither or both limits are included in the smaller ranges is also encompassed within this disclosure, subject to any specifically excluded limit in the stated range. Where the stated range includes one or both of the limits, ranges excluding either or both of those included limits are also included in this disclosure.

It must be noted that as used herein and in the appended claims, the singular forms "a", "an", and "the" include plural referents unless the context clearly dictates otherwise.

The terms "comprising", "comprises" and "comprised of' as used herein are synonymous with "including", "includes" or "containing", "contains", and are inclusive or open-ended and do not exclude additional, non-recited members, elements or method steps. The terms "comprising", "comprises" and "comprised of' also include the term "consisting of.

The publications discussed herein are provided solely for their disclosure prior to the filing date of the present application. Nothing herein is to be construed as an admission that such publications constitute prior art to the claims appended hereto.

The invention will now be further described by way of Examples, which are meant to serve to assist one of ordinary skill in the art in carrying out the invention and are not intended in any way to limit the scope of the invention.

EXAMPLES

**Materials and methods**

***Preparation of PEIProIDNA complexes***

The standard PEIpro preparation method is as follows:

1. **Make "PEI mix".** Add PEIPro to **culture media** at approximately 1:20 ratio. Briefly mix by swirling after addition is complete.
2. **Make "DNA mix".** Add DNA plasmids to **media** at approximately 1:20 ratio.
3. **Combine PEI and DNA mixes.** The PEI mixture is then added to the DNA mixture at a 1:1 ratio, by either pouring

or pumping. The transfection mixture is then incubated at room temperature to allow complex formation.

The aqueous PEIpro method is as follows:

1. **Make "PEI mix".** Add PEIPro to **water** at approximately 1:20 ratio. Briefly mix by swirling after addition is complete. Neither the addition time, nor incubation time after addition is time-dependent.
2. **Make "DNA mix".** Add DNA plasmids to **water** at approximately 1:20 ratio. Neither the addition time, nor incubation time after addition is time-dependent.
3. **Combine PEI and DNA mixes.** The PEI mixture is then added to the DNA mixture at a 1:1 ratio, by either pouring or pumping. Briefly swirl mixture after completion. Neither the addition time, nor incubation time after addition is time-dependent.
4. **Spike PBS.** Add 10X PBS at approximately a 1:50 ratio of 10X PBS to the final transfection volume so that the final concentration of PBS is equal to 0.2X. The mixture is briefly swirled immediately after addition and timer started. The mixture is incubated at room temperature to allow complex formation.

The above steps may be followed by transfection. For the bioreactor experiments, the transfection mix was added to the bioreactors via hand pump after 30 minutes of incubation. The incubation period varied for the shake flask studies where time course experiments were executed.

***Shake flask experimental conditions***

In order to investigate the effects of transfection complex incubation time on GFP vector production using DNA/PEIPro complexes prepared in culture media, a single transfection mix was prepared as described above and allowed to stand for up to 60 minutes at room temperature following complexation. At defined intervals, approximately 2 mL aliquots were removed from the bulk transfection mix and used to transfect individual E125 Erlenmeyer flasks seeded with HEK293T cells. The final volume in the shake flasks equalled 40mL so the transfection volume represented 1:20 ratio.

**Example 1 - When the transfection mix is prepared in media, the optimal incubation time that yields the highest titre is too short to be applicable to GMP manufacturing**

The data demonstrates that the incubation period of DNA/PEIPro® complexes significantly impacts subsequent transfection and lentiviral vector production (Figure 1). The data shows that the optimal incubation time that yields the highest titre is approximately 3 minutes and this rapidly decreases if the incubation time is extended to 15 minutes or 30 minutes. This represents a significant issue in terms of developing a PEIPro® process for large scale GMP manufacturing purposes. Compared to the standard Lipofectamine 2000CD-based process where complex stability of up to 6 hours allows a robust process which is minimally time restrained, the requirement that complexes must be prepared and added to the bioreactor within 5 minutes places significant time restraints on the process itself that are not practical for large scale GMP production.

**Example 2 - Kinetics of complex formation can be modified by preparing the transfection mix in water and varying the PBS concentration used to initiate DNA/PEIPro® complex formation**

The transfection complex was prepared as described above under the subheading "Preparation of PEIPro/DNA complexes" using GFP vector. A bulk transfection mix was prepared in water and divided three ways. A proportion of the transfection complex was spiked with a volume of 10X PBS so that the final concentration of PBS was equal to 1X. A portion of the transfection mix was spiked with 10X PBS so that the final concentration was equal to 0.2X. The remaining transfection mix was left untouched and was not spiked with any PBS. The two preparations that were spiked with PBS were then allowed to incubate at room temperature for up to 2 hours. At defined intervals, approximately 2 mL aliquots were removed from the bulk transfection mix's and used to transfect individual E125 Erlenmeyer flasks seeded with HEK293T cells. The final volume in the shake flasks equalled 40mL so the transfection volume represented 1:20 ratio.

For the 1X spiked transfection mix, 9 shake flasks were transfected at the following time intervals; 1min; 2 mins; 3mins; 4mins; 5mins; 6 mins; 7mins; 15mins; 30mins and 60mins and 120mins. A further 12 shake flasks were transfected with the 0.2X PBS transfection mix at 1 min; 2mins; 3 mins; 4 mins; 6 mins; 10 mins; 15mins; 20mins; 25 mins; 30mins and 40mins. At the end of the GFP vector production process, samples were taken from each shake flask and functional titre was determined using a flow cytometry based transduction assay. The results illustrate that the kinetics of the complex growth can be modified by varying PBS concentration (Figure 2). Figure 2A shows that optimal titre was achieved after 3 minutes' incubation when transfection complex was spiked with 1X PBS. Figure 2B shows that the incubation

time can be extended if the transfection complex is spiked with a lower concentration of PBS. Spiking with 0.2X PBS extends the optimal incubation time to approximately 25 minutes. As a negative control the complex was prepared in water and then used to transfect the cells without spiking with PBS. No vector was produced demonstrating that PBS is required to initiate complex growth.

**Example 3 - Transfection efficiency is stable for up to 12 hours and potentially up to a week following dilution of the transfection mix to reduce the salt concentration (and arrest complex growth)**

The transfection mix was prepared as described above, spiked with 0.2X PBS to initiate complex growth and then incubated for 30 minutes to allow for the optimal size of the complex to be reached. The transfection mix was then diluted using salt-free aqueous solution (i.e. $H_2O$) so that the final concentration of PBS was 0.1X. The transfection mix was then stored at 4°C for up to 1 week. To test the stability of the diluted PEIPro®/DNA complexes, the functional lentiviral vector titre achieved using the complexes after various incubation times post-dilution was evaluated. Aliquots of the transfection mix were then used to generate GFP lentivirus and the resultant functional titre was measured. The results demonstrate that transfection efficiency of the diluted complexes is retained over time (Figure 3) and is stable for at least one week at 4°C. In particular, the graph shows that after 12 hours' incubation, the transfection mix was still efficient to generate lentiviral vectors yielding a comparable functional titre compared to the "fresh" transfection mix.

**Example 4 - Dynamic light scattering analysis (Zetasizer Nanometer ZS Malvern Instrument, Malvern, UK) showing the size over time when the DNA:PEIpro complex is made in $H_2O$.**

DNA/PEIpro complexes were prepared in $H_2O$ as described above and then samples of the transfection mix were removed at 2 minute intervals over a 60-minute period and analysed using the Zetasizer to measure complex size. Figure 4 illustrates that the complex did not increase in size over a period of 60 minutes. This correlates with the data presented in Figure 2B which demonstrated that complexes made up in water alone did not lead to vector production. This data together with the DLS measurements suggest that complexes within the size range of 80-86nm are too small to lead to successful transfection.

**Example 5 - Dilution of the transfection mix by 2-fold halts the growth at the optimal particle size.**

DNA/PEIpro complexes were made up in $H_2O$ as described above and particle growth was initiated by addition of PBS to a final concentration of 0.2X. The complex was incubated for 25 mins at room temperature as this corresponded to the optimal time to achieve the highest functional titre (see Figure 2B). The complex was then diluted 2 fold so that the PBS concentration was 0.1X. The complex size was then measured over time for up to 45mins. The DLS measurements show that dilution of the transfection mix by 2 fold was found to halt the growth of the complex and the size remained between 600-800nm (Figure 5). Correlating this data with the functional data generated in examples 2 and 3 described above, suggests that the optimal complex size that achieves the highest titre, is between 600-800nm.

**Example 6 - DLS analysis illustrates that different concentrations of PBS effect the kinetics of the complex growth.**

DNA/PEIpro complexes were made up in water as described above and particle growth was initiated by addition of three different concentrations of PBS (0.1X; 0.15X and 0.2X) and incubated at room temperature. Samples were then removed every 2 minutes and analysed using the Zetasizer (Zetasizer Nanometer ZS Malvern Instrument, Malvern, UK). The results show that when the transfection mix is spiked with PBS at different concentrations, the rate at which the complex grows to the optimal size for transfection varies depending on the concentration of PBS added. If the concentration of PBS is too low <0.15X PBS, then this is not concentrated enough to initiate complex growth (Figure 6). Table 1 shows the effect of different PBS concentrations on complex growth on time taken to reach optimal size.

Table 1 – Effect of different PBS concentrations on complex growth on time taken to reach optimal size as measured by Zetasizer Nanometer ZS (Malvern Instrument, Malvern, UK)

| PBS concentration | Optimal size* | Time to reach |
|---|---|---|
| 0.1x | 600-800 | Did not initiate growth |
| 0.15x | 600-800 | Minimal growth observed |
| 0.2x | 600-800 | Approximately 25 minutes |

*optimal size determined through time course vector production runs

**Example 7 - Any salt can be used to initiate complex growth**

DNA/PEIpro complexes were prepared in $H_2O$ and then spiked with different concentrations of NaCl or $MgCl_2$. Figure 7A and Table 2 show the impact of spiking the transfection mix with four different concentrations of NaCl (10mM; 20mM; 50mM; 80mM and 100mM). Only concentrations of 80mM and 100mM initiated complex growth. Figure 7B and Table 3 show the impact of spiking the transfection mix with four different concentrations of $MgCl_2$ (10mM; 20mM; 50mM; 80mM; 100mM). As $MgCl_2$ has a higher ionic strength, a lower concentration initiated complex growth and the time taken for the optimal complex size to be reached was quicker than NaCl.

Table 2 – Effect of different NaCl concentrations on complex growth on time taken to reach optimal size as measured by Zetasizer Nanometer ZS (Malvern Instrument, Malvern, UK)

| NaCl concentration (mM) | Optimal size* | Time to reach optimal size |
|---|---|---|
| 10 | 600-800 | Did not initiate growth |
| 20 | 600-800 | Did not initiate growth |
| 50 | 600-800 | Did not initiate growth |
| 80 | 600-800 | 30 minutes |
| 100 | 600-800 | 10 minutes |

*optimal size determined through time course vector production runs

Table 3 – Effect of different $MgCl_2$ concentrations on complex growth on time taken to reach optimal size as measured by Zetasizer Nanometer ZS (Malvern Instrument, Malvern, UK).

| $MgCl_2$ concentration (mM) | Optimal size* | Time to reach optimal size |
|---|---|---|
| 10 | 600-800 | Did not initiate growth |
| 20 | 600-800 | Did not initiate growth |
| 50 | 600-800 | 10-12 minutes |
| 80 | 600-800 | 5 minutes |
| 100 | 600-800 | 5 minutes |

*optimal size determined through time course vector production runs

**Example 8 - The functional titre values obtained at 5L scale using the aqPEIPro transfection method compare favourably with those obtained when the therapeutic vector product is produced using a cationic lipid based transfection method.**

Six 5 L bioreactors were employed in this study, four of the bioreactors were transiently transfected using a cationic lipid based transfection method while the other two bioreactors were transfected using the described aqPEIPro® method. Both bioreactors were transiently transfected with an HIV-1 vector genome encoding 5T4-CAR. This therapeutic vector is for the genetic modification of T-cells enabling them to target and kill tumour cells expressing the antigen 5T4 (Owens, L.G ; Sheard, V.E et al J Immunother. 2018 Apr;41(3):130-140).

Samples at the end of the production process were taken and functional titre was determined through use of transduction assay and flow cytometry analysis.

The functional titre values obtained at 5 L scale in this study compared favourably with those obtained when the product was produced using the cationic lipid based transfection method and the data looked to be more consistent (n = 2). It can be seen that the mean crude harvest values obtained using the aqueous PEIPro method yielded a titre of 4.82.25 $\times$ $10^5$ TU/mL (standard deviation = +/- 6.8 $\times$ $10^4$TU/mL) compared to 3.5375 $\times$ $10^5$ TU/mL (Standard deviation = +/- 1.8 $\times$ $10^5$TU/mL) using the cationic lipid based transfection method (n = 4). This suggests that the aqPEIPro transfection method works equally well to cationic lipid based transfection methods and is scalable to 5L (Figure 8).

All publications mentioned in the above specification are herein incorporated by reference. Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

Preferred features and embodiments of the present invention will now be described with reference to the following numbered paragraphs:

1. An *in vitro* method for producing a solution of polymer/nucleic acid complexes comprising the steps of:

a) mixing a nucleic acid and a cationic polymer in a substantially salt free aqueous solution;
b) adding a salt to the mixture produced in step a) to form a salt solution;
c) optionally incubating the salt solution produced in step b); and/or
d) optionally diluting the salt solution produced in step b) or diluting the salt solution following incubation of step c),

wherein said salt is not a valproic acid salt, isobutyric acid salt or isovaleric acid salt.

2. An *in vitro* method for producing a solution of polymer/nucleic acid complexes comprising the steps of:

a) mixing a nucleic acid and a cationic polymer in a substantially salt free aqueous solution;
b) adding a salt to the mixture produced in step a) to form a salt solution;
c) optionally incubating the salt solution produced in step b); and/or
d) optionally diluting the salt solution produced in step b) or diluting the salt solution following incubation of step c),

wherein said salt is added only prior to contacting the solution of polymer/nucleic acid complexes with a cell.

3. An *in vitro* method for producing a solution of polymer/nucleic acid complexes comprising the steps of:

a) mixing a nucleic acid and a cationic polymer in a substantially salt free aqueous solution;
b) adding a salt to the mixture produced in step a) to form a salt solution;
c) optionally incubating the salt solution produced in step b); and/or
d) optionally diluting the salt solution produced in step b) or diluting the salt solution following incubation of step c),

wherein the final salt concentration in step b) is between about 10 mM to about 100 mM.

4. An *in vitro* method for producing a solution of polymer/nucleic acid complexes comprising the steps of:

a) mixing a nucleic acid and a cationic polymer in a substantially salt free aqueous solution;
b) adding a salt to the mixture produced in step a) to form a salt solution;
c) optionally incubating the salt solution produced in step b); and/or

d) optionally diluting the salt solution produced in step b) or diluting the salt solution following incubation of step c),

wherein said salt has a degree of dissociation of at least 0.95 in the substantially salt free aqueous solution.

5. An *in vitro* method for producing a solution of polymer/nucleic acid complexes comprising the steps of:

a) mixing a nucleic acid and a cationic polymer in a substantially salt free aqueous solution;
b) adding a salt to the mixture produced in step a) to form a salt solution;
c) optionally incubating the salt solution produced in step b); and/or
d) diluting the salt solution produced in step b) or diluting the salt solution following incubation of step c).

6. An *in vitro* method for producing a solution of polymer/nucleic acid complexes comprising the steps of:

a) mixing a nucleic acid and a cationic polymer in a substantially salt free aqueous solution;
b) adding a salt to the mixture produced in step a) to form a salt solution;
c) incubating the salt solution produced in step b); and/or
d) optionally diluting the salt solution produced in step b) or diluting the salt solution following incubation of step c).

7. The method according to any one of the preceding paragraphs, wherein the nucleic acid is selected from DNA, RNA, oligonucleotide molecule, and mixtures thereof.

8. The method according to paragraph 7, wherein the nucleic acid is DNA, preferably plasmid DNA.

9. The method according to any one of the preceding paragraphs, wherein the cationic polymer is a polymer-based transfection reagent.

10. The method according to any one of the preceding paragraphs, wherein the cationic polymer is selected from polyethylenimine (PEI), a dendrimer, DEAE-dextran, polypropyleneimine (PPI), chitosan [poly-(β-1/4)-2-amino-2-deoxy-D-glucopyranose], poly-L-lysine (PLL), poly(lactic-co-glycolic acid) (PLGA), poly(caprolactone) (PCL), and a derivative thereof.

11. The method according to any one of the preceding paragraphs, wherein the cationic polymer is PEI or a derivative thereof, preferably selected from linear PEI, branched PEI, PEGylated PEI, JetPEI, PEIPro®, PEI MAX and PTG1+.

12. The method according to any one of the preceding paragraphs, wherein the salt is selected from a sodium salt, a magnesium salt, a potassium salt, a calcium salt, a phosphate salt and a mixture thereof.

13. The method according to any one of the preceding paragraphs, wherein the salt is phosphate buffered saline (PBS).

14. The method according to any one of paragraphs 1, 2 and 4 to 13, wherein the salt is PBS and PBS is added in step b) to a final concentration of between about 0.1X PBS to about 1.0X PBS, preferably wherein the PBS is added to a final concentration of between about 0.1X PBS to about 0.3X PBS.

15. The method according to any one of paragraphs 1, 2 and 4 to 14, wherein the salt is added in step b) to a final concentration of between about 10 mM to about 100 mM, preferably wherein the salt is added in step b) to a final concentration of between about 20 mM to about 100 mM.

16. The method according to any one of the preceding paragraphs, wherein step a) comprises mixing a plurality of nucleic acid molecules and a plurality of cationic polymer molecules in a substantially salt free aqueous solution.

17. The method according to any one of paragraphs 1 to 5 and 7 to 17, wherein the method comprises the step of incubating the salt solution produced in step b).

18. The method according to paragraph 6 or paragraph 17, wherein the salt solution is incubated for about 1 minute to up to about 2 hours, preferably wherein the salt solution is incubated for about 20 minutes to about 90 minutes.

19. The method according to paragraph 18, wherein the salt solution is incubated for about 60 minutes.

20. The method according to any one of paragraphs 1 to 4 and 6 to 17, wherein the method comprises the step of diluting the salt solution produced in step b) or the salt solution following incubation of step c).

21. The method according to paragraph 5 or paragraph 20, wherein the salt is PBS and the salt solution is diluted to a final concentration of between about 0.05X PBS to about 0.15X PBS, preferably wherein the salt solution is diluted to a final concentration of about 0.1X PBS.

22. The method according to any one of the preceding paragraphs, wherein the nucleic acid encodes a retroviral vector component.

23. The method according to paragraph 22, wherein the retroviral vector is a replication defective retroviral vector.

24. The method according to paragraph 22 or paragraph 23, wherein the retroviral vector is a lentiviral vector, preferably wherein the lentiviral vector is HIV-1, HIV-2, SIV, FIV, BIV, EIAV, CAEV or Visna.

25. The method according to paragraph 24, wherein the vector component is selected from:

a) the RNA genome of the lentiviral vector;
b) env or a functional substitute thereof;
c) gag-pol or a functional substitute thereof; and/or
d) rev or functional substitute thereof.

26. The method according to any one of the preceding paragraphs, wherein the method further comprises the steps of:

e) optionally culturing a mammalian cell in a perfusion culture;
f) transfecting a mammalian cell using the solution of polymer/nucleic acid complexes obtained by the method according to any one of the preceding paragraphs;
g) optionally introducing a nucleic acid that is different to the nucleic acid of the solution of polymer/nucleic acid complexes into the mammalian cell;
h) optionally selecting for a mammalian cell which has the nucleic acid(s) integrated within its genome;
i) optionally culturing the mammalian cell under conditions in which the nucleic acid(s) is (are) expressed;
j) optionally culturing the mammalian cell under conditions in which the retroviral vector is produced; and
k) optionally isolating the retroviral vector.

27. The method according to paragraph 26, wherein the method comprises the step of culturing a mammalian cell in a perfusion culture.

28. The method according to paragraph 26 or paragraph 27, wherein the step of culturing a mammalian cell in a perfusion culture is performed for about 10 hours to about 96 hours.

29. The method according to any one of paragraphs 26 to 28, wherein the method comprises the step of culturing the mammalian cell under conditions in which the nucleic acid(s) is expressed.

30. The method according to any one of paragraphs 26 to 28, wherein the method comprises the step of culturing the mammalian cell under conditions in which the retroviral vector is produced.

31. The method according to any one of paragraphs 26 to 30, wherein the method comprises the step of isolating the retroviral vector.

32. The method according to any one of paragraphs 26 to 31, wherein the mammalian cell is a HEK293T cell.

33. The method according to any one of paragraphs 26 to 32, wherein the mammalian cell is a suspension-adapted mammalian cell.

34. The method according to any one of paragraphs 26 to 33, wherein the transfection, introduction and/or culturing step(s) is (are) performed in suspension in a serum-free medium.

35. The method according to any one of paragraphs 26 to 34, wherein the transfection is a transient transfection.

36. The method according to any one of paragraphs 26 to 35, wherein the transfection, introduction and/or culturing step(s) is (are) carried out in a volume of at least 50 L, preferably wherein the transfection, introduction and culturing steps are carried out in a volume of at least 50 L.

37. The method according to any one of paragraphs 26 to 36, wherein the nucleic acid that is different to the nucleic acid of the solution of polymer/nucleic acid complexes is introduced into the mammalian cell by transfection or by electroporation.

38. The method according to any one of paragraphs 26 to 37, wherein the nucleic acid that is different to the nucleic acid of the solution of polymer/nucleic acid complexes encodes any viral vector components selected from:

a) the RNA genome of the lentiviral vector;
b) env or a functional substitute thereof;
c) gag-pol or a functional substitute thereof; and/or
d) rev or functional substitute thereof;

that are not encoded by the nucleic acid of the solution of polymer/nucleic acid complexes.

39. A solution of polymer/nucleic acid complexes obtained or obtainable by the method according to any one of paragraphs 1-38.

40. The solution of polymer/nucleic acid complexes according to paragraph 39, wherein the solution is obtained or obtainable by the method according to any one of paragraphs 22 to 25.

41. A solution comprising polymer/nucleic acid complexes and a salt, wherein the salt concentration is between about 10 mM to about 100 mM.

42. The solution of polymer/nucleic acid complexes according to paragraph 41, wherein the salt concentration is less than about 90 mM or the salt is PBS and the concentration of PBS is less than about 0.3X PBS.

43. The solution of polymer/nucleic acid complexes according to paragraph 41 or paragraph 42, wherein the nucleic acid encodes a component for producing a retroviral vector, preferably wherein the retroviral vector is a lentiviral vector and the component for producing a lentiviral vector is selected from:

(i) the RNA genome of the lentiviral vector;
(ii) env or a functional substitute thereof;
(iii) gag-pol or a functional substitute thereof; and/or
(iv) rev or functional substitute thereof.

44. The solution of polymer/nucleic acid complexes according to paragraph 43, wherein the retroviral vector is a lentiviral vector, preferably wherein the lentiviral vector is HIV-1, HIV-2, SIV, FIV, BIV, EIAV, CAEV or Visna.

45. Use of a solution of polymer/nucleic acid complexes according to any one of paragraphs 39 to 44 for the transfection of the nucleic acid into cells.

46. Use of a solution of polymer/nucleic acid complexes according to any one of paragraphs 40, 43 or 44 in a method for the production of a retroviral vector.

47. A method for producing a retroviral vector comprising the steps of:

a) optionally culturing a mammalian cell in a perfusion culture;
b) transfecting the mammalian cell using solution of polymer/nucleic acid complexes as defined in any one of paragraphs 22 to 25 or a solution of polymer/nucleic acid complexes according to any one of paragraphs 40, 43 or 44;
c) optionally introducing at least one nucleic acid that is different to the nucleic acid of the solution of polymer/nucleic acid complexes into the mammalian cell;
d) optionally selecting for a mammalian cell which has the nucleic acid sequences encoding the viral vector components integrated within its genome; and

e) culturing the mammalian cell under conditions in which the retroviral vector is produced.

48. The method according to paragraph 47, further comprising the step of isolating the retroviral vector.

49. The method according to paragraph 47 or paragraph 48, wherein the retroviral vector is a replication defective retroviral vector.

50. The method according to any one of paragraphs 47 to 49, wherein the retroviral vector is a lentiviral vector, preferably wherein the lentiviral vector is HIV-1, HIV-2, SIV, FIV, BIV, EIAV, CAEV or Visna.

51. The method according to any one of paragraphs 47 to 50, wherein the mammalian cell is a HEK293T cell.

52. The method according to any one of paragraphs 47 to 51, wherein the mammalian cell is a suspension-adapted cell.

53. The method according to any one of paragraphs 47 to 52, wherein the method is performed in suspension in a serum-free medium.

54. The method according to any one of paragraphs 47 to 53, wherein the method comprises the step of culturing a mammalian cell in a perfusion culture.

55. The method according to any one of paragraphs 47 to 54, wherein the step of culturing a mammalian cell in a perfusion culture is performed for about 10 hours to about 96 hours.

56. The method according to any one of paragraphs 47 to 55, wherein step a), step b), step c) and/or step e) is carried out in a volume of at least 50 L, preferably wherein step a), step b), step c) and step e) are carried out in a volume of at least 50 L.

57. The method according to any one of paragraphs 47 to 56, wherein the nucleic acid is introduced into the cell by transfection or by electroporation in step c).

58. The method according to any one of paragraphs 47 to 57, wherein the transfection is a transient transfection.

59. The method according to any one of paragraphs 47 to 58, wherein the at least one nucleic acid optionally introduced into the mammalian cell in step b) encodes any lentiviral vector components selected from the group consisting of:

(i) the RNA genome of the lentiviral vector;
(ii) env or a functional substitute thereof;
(iii) gag-pol or a functional substitute thereof; and/or
(iv) rev or functional substitute thereof;

that are not encoded by the nucleic acid of the solution of polymer/nucleic acid complexes.

60. A method for transfecting a mammalian cell comprising the steps of:

a) culturing a mammalian cell in a perfusion culture; and

b) transfecting the mammalian cell using solution of polymer/nucleic acid complexes as defined in any one of paragraphs 22 to 25 or a solution of polymer/nucleic acid complexes according to any one of paragraphs 40, 43 or 44.

61. The method according to paragraph 60, wherein the step of culturing a mammalian cell in a perfusion culture is performed for about 10 hours to about 96 hours.

62. The method according to paragraph 60 or paragraph 61, wherein the mammalian cell is a HEK293T cell.

63. The method according to any one of paragraphs 60 to 62, wherein the mammalian cell is a suspension-adapted

cell.

64. The method according to any one of paragraphs 60 to 63, wherein the method is performed in suspension in a serum-free medium.

65. The method according to any one of paragraphs 60 to 64, wherein step a) and/or step b) is carried out in a volume of at least 50 L, preferably wherein step a) and step b) are carried out in a volume of at least 50 L.

66. The method according to any one of paragraphs 60 to 65, wherein the transfection is a transient transfection.

## Claims

**1.** An *in vitro* method for controlling the initiation and/or termination of the maturation of polymer/nucleic acid complexes comprising the steps of:

a) mixing a nucleic acid and a cationic polymer in a substantially salt free aqueous solution;
b) adding a salt to the mixture produced in step a) to form a salt solution;
c)

(i) optionally incubating the salt solution produced in step b); and/or
(ii) optionally diluting the salt solution produced in step b) or diluting the salt solution following incubation of step c)(i).

**2.** The method according to claim 1, wherein:

(i) said salt is not a valproic acid salt, isobutyric acid salt or isovaleric acid salt;
(ii) said salt is added only prior to contacting the solution of polymer/nucleic acid complexes with a cell;
(iii) the final salt concentration in step b) is between about 10 mM to about 100 mM; and/or
(iv) said salt has a degree of dissociation of at least 0.95 in the substantially salt free aqueous solution.

**3.** The method according to any one of the preceding claims, wherein:

(i) the nucleic acid is selected from DNA, RNA, oligonucleotide molecule, and mixtures thereof, preferably wherein the nucleic acid is DNA, preferably plasmid DNA;
(ii) the cationic polymer is a polymer-based transfection reagent;
(iii) the cationic polymer is selected from polyethylenimine (PEI), a dendrimer, DEAE-dextran, polypropyleneimine (PPI), chitosan [poly-(β-1/4)-2-amino-2-deoxy-D-glucopyranose], poly-L-lysine (PLL), poly(lactic-co-glycolic acid) (PLGA), poly(caprolactone) (PCL), and a derivative thereof;
(iv) the cationic polymer is PEI or a derivative thereof, preferably selected from linear PEI, branched PEI, PEGylated PEI, JetPEI, PEIPro®, PEI MAX and PTG1+; and/or
(v) the salt is selected from a sodium salt, a magnesium salt, a potassium salt, a calcium salt, a phosphate salt and a mixture thereof, optionally wherein the salt is phosphate buffered saline (PBS)and PBS is added in step b) to a final concentration of between about 0.1X PBS to about 1.0X PBS, preferably wherein the PBS is added to a final concentration of between about 0.1X PBS to about 0.3X PBS.

**4.** The method according to any one of the preceding claims, wherein the method comprises the step of incubating the salt solution produced in step b), preferably wherein the salt solution is incubated for about 1 minute to up to about 2 hours.

**5.** The method according to any one of the preceding claims, wherein the method comprises the step of diluting the salt solution produced in step b) or the salt solution following incubation of step c), preferably wherein the salt is PBS and the salt solution is diluted to a final concentration of between about 0.05X PBS to about 0.15X PBS.

**6.** The method according to any one of the preceding claims, wherein the nucleic acid encodes a retroviral vector component.

**7.** The method according to any one of the preceding claims, wherein the method further comprises the steps of:

e) optionally culturing a mammalian cell in a perfusion culture;
f) transfecting a mammalian cell using the solution of polymer/nucleic acid complexes obtained by the method according to any one of the preceding claims;
g) optionally introducing a nucleic acid that is different to the nucleic acid of the solution of polymer/nucleic acid complexes into the mammalian cell;
h) optionally selecting for a mammalian cell which has the nucleic acid(s) integrated within its genome;
i) optionally culturing the mammalian cell under conditions in which the nucleic acid(s) is (are) expressed;
j) optionally culturing the mammalian cell under conditions in which the retroviral vector is produced; and
k) optionally isolating the retroviral vector.

**8.** The method according to claim 7, wherein:

(i) the mammalian cell is a HEK293T cell;
(ii) the mammalian cell is a suspension-adapted mammalian cell;
(iii) the transfection, introduction and/or culturing step(s) is (are) performed in suspension in a serum-free medium; and/or
(iv) the transfection is a transient transfection.

**9.** The method according to claim 7 or claim 8, wherein the transfection, introduction and/or culturing step(s) is (are) carried out in a volume of at least 50 L, preferably wherein the transfection, introduction and culturing steps are carried out in a volume of at least 50 L.

**10.** A solution of polymer/nucleic acid complexes obtained or obtainable by the method according to any one of claims 1 to 9, preferably wherein the solution is obtained or obtainable by the method according to claim 6.

**11.** A solution comprising polymer/nucleic acid complexes and a salt, wherein the salt concentration is between about 10 mM to about 100 mM, preferably wherein the salt concentration is less than about 90 mM or the salt is PBS and the concentration of PBS is less than about 0.3X PBS.

**12.** The solution of polymer/nucleic acid complexes according to claim 11, wherein the nucleic acid encodes a component for producing a retroviral vector, preferably wherein the retroviral vector is a lentiviral vector and the component for producing a lentiviral vector is selected from:

(i) the RNA genome of the lentiviral vector;
(ii) env or a functional substitute thereof;
(iii) gag-pol or a functional substitute thereof; and/or
(iv) rev or functional substitute thereof.

**13.** Use of a solution of polymer/nucleic acid complexes according to any one of claims 10 to 12 for the transfection of the nucleic acid into cells.

**14.** Use of a solution of polymer/nucleic acid complexes according to claim 10 or claim 12 in a method for the production of a retroviral vector.

**15.** A method for producing a retroviral vector comprising the steps of:

a) optionally culturing a mammalian cell in a perfusion culture;
b) transfecting the mammalian cell using the solution of polymer/nucleic acid complexes as defined in claim 6 or the solution of polymer/nucleic acid complexes according to claim 10 or claim 12;
c) optionally introducing at least one nucleic acid that is different to the nucleic acid of the solution of polymer/nucleic acid complexes into the mammalian cell;
d) optionally selecting for a mammalian cell which has the nucleic acid sequences encoding the viral vector components integrated within its genome;
e) culturing the mammalian cell under conditions in which the retroviral vector is produced; and
f) optionally isolating the retroviral vector,

preferably wherein step a), step b), step c) and/or step e) is carried out in a volume of at least 50 L, preferably wherein step a), step b), step c) and step e) are carried out in a volume of at least 50 L.

**16.** A method for transfecting a mammalian cell comprising the steps of:

a) culturing a mammalian cell in a perfusion culture; and
b) transfecting the mammalian cell using the solution of polymer/nucleic acid complexes as defined in claim 6 or a solution of polymer/nucleic acid complexes according to claim 10 or claim 12, preferably wherein step a) and/or step b) is carried out in a volume of at least 50 L, preferably wherein step a) and step b) are carried out in a volume of at least 50 L.

**17.** Use of a cationic polymer in an *in vitro* method for controlling the initiation and/or termination of the maturation of polymer/nucleic acid complexes comprising the steps of:

a) mixing a nucleic acid and a cationic polymer in a substantially salt free aqueous solution;
b) adding a salt to the mixture produced in step a) to form a salt solution;
c)

(i) optionally incubating the salt solution produced in step b); and/or
(ii) optionally diluting the salt solution produced in step b) or diluting the salt solution following incubation of step c)(i).

FIGURE 1

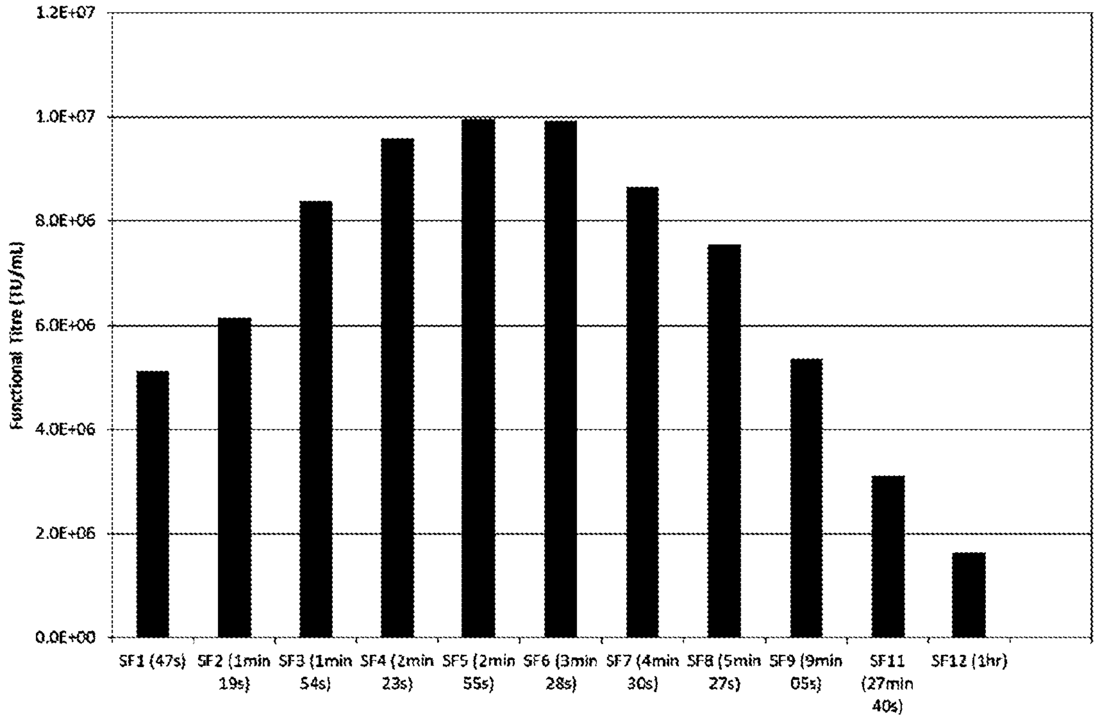

1.2E+07
1.0E+07
8.0E+06
6.0E+06
4.0E+06
2.0E+06
0.0E+00
Functional Titre (TU/mL)
SF1 (47s)
SF2 (1min 19s)
SF3 (1min 54s)
SF4 (2min 23s)
SF5 (2min 55s)
SF6 (3min 28s)
SF7 (4min 30s)
SF8 (5min 27s)
SF9 (9min 05s)
SF11 (27min 40s)
SF12 (1hr)

FIGURE 2

A

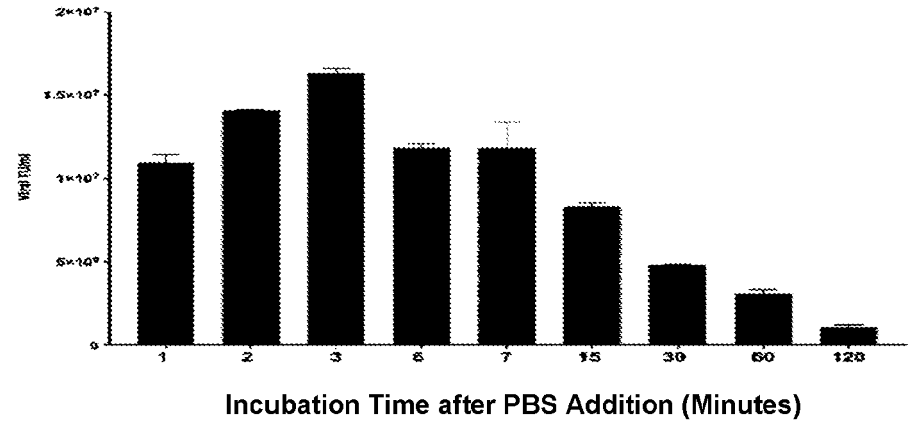
2×10⁷
1.5×10⁷
1×10⁷
5×10⁶
0
Viral TU/ml
1
2
3
6
7
15
30
60
120
Incubation Time after PBS Addition (Minutes)

B

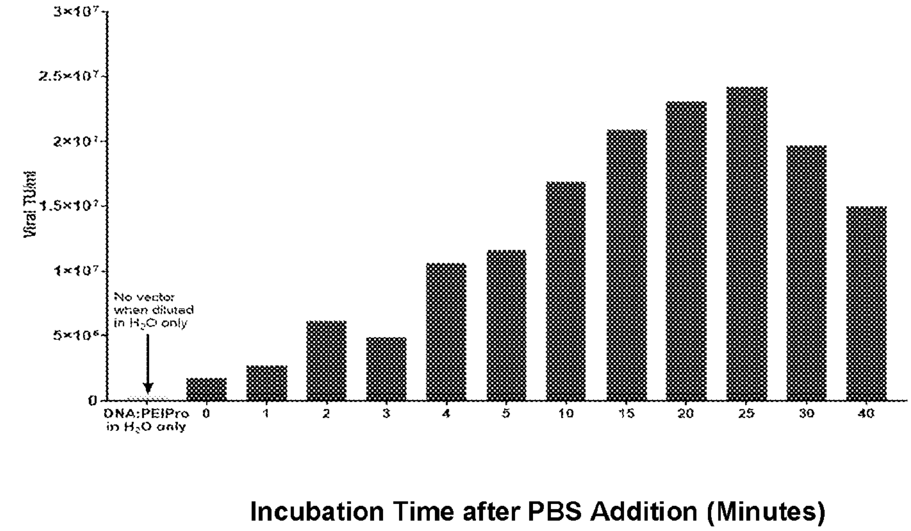
3×10⁷
2.5×10⁷
2×10⁷
1.5×10⁷
1×10⁷
5×10⁶
0
Viral TU/ml
No vector when diluted in H₂O only
DNA:PEIPro in H₂O only
0
1
2
3
4
5
10
15
20
25
30
40
Incubation Time after PBS Addition (Minutes)

FIGURE 3

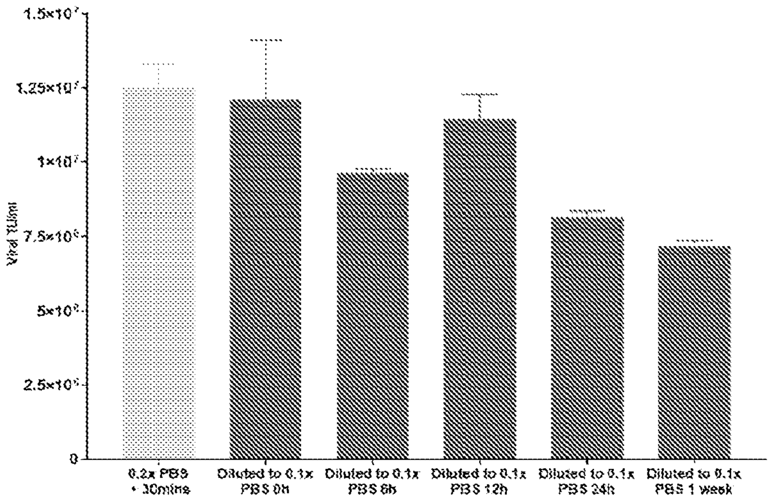
1.5×10^7
1.25×10^7
1×10^7
7.5×10^6
5×10^6
2.5×10^6
0
Viral TU/ml
0.2x PBS + 30mins
Diluted to 0.1x PBS 0h
Diluted to 0.1x PBS 6h
Diluted to 0.1x PBS 12h
Diluted to 0.1x PBS 24h
Diluted to 0.1x PBS 1 week

FIGURE 4

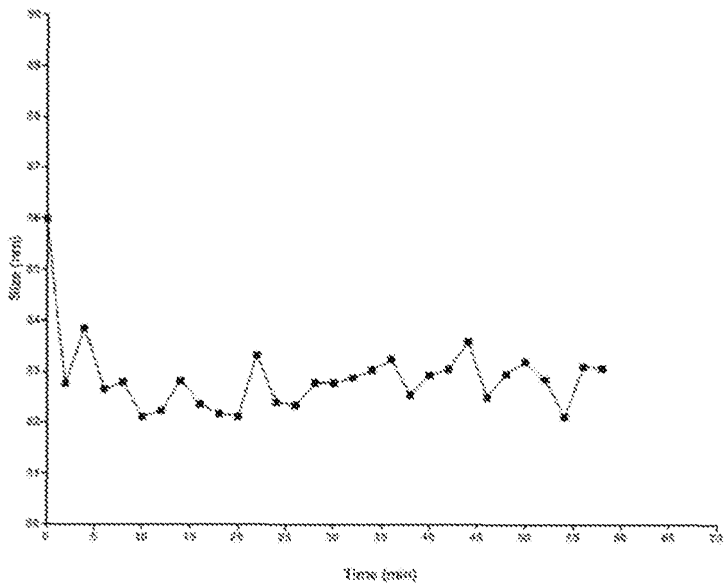

FIGURE 5

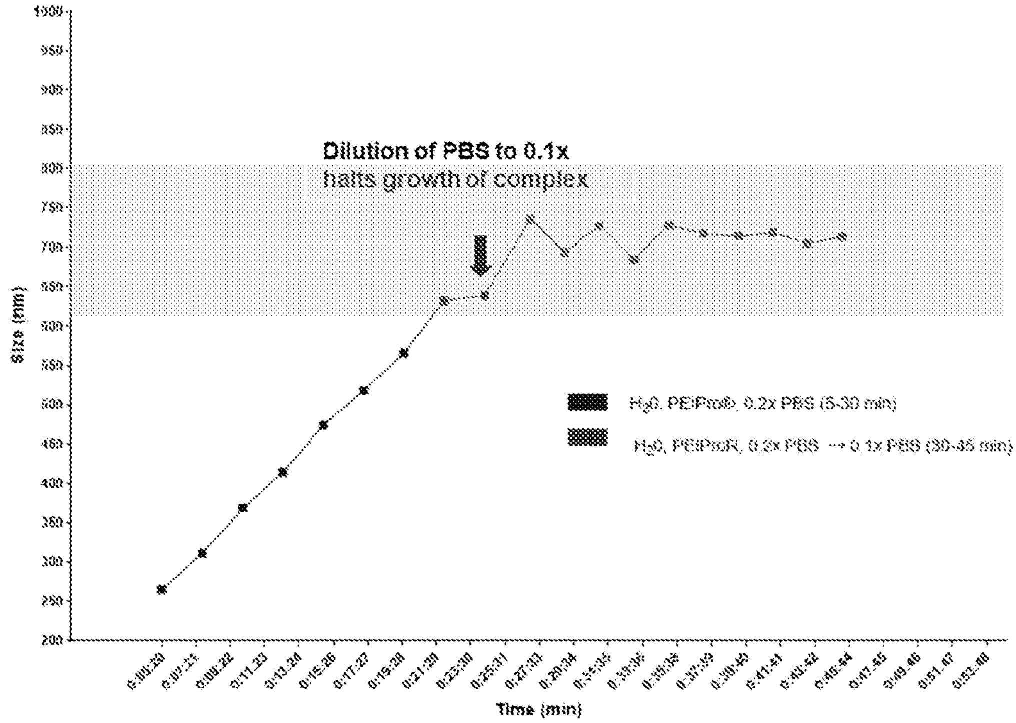
Dilution of PBS to 0.1x
halts growth of complex
Size (nm)
Time (min)

FIGURE 6

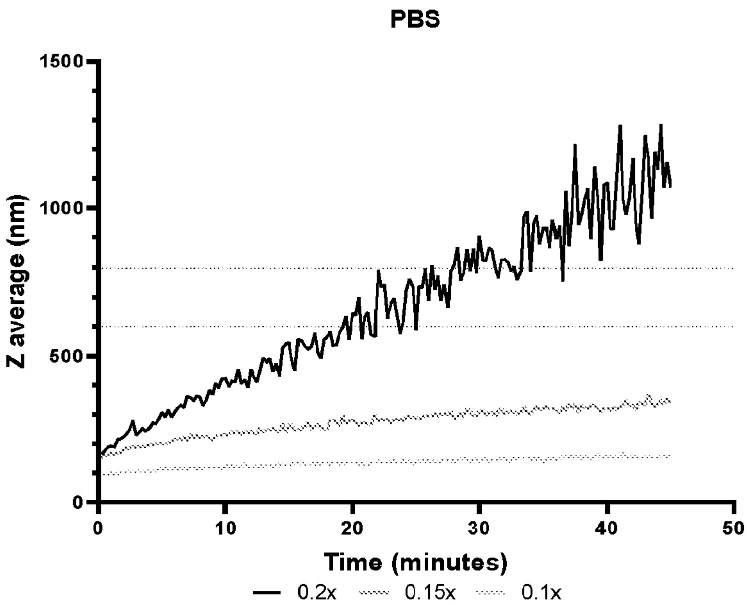
PBS
Z average (nm)
1500
1000
500
0
0
10
20
30
40
50
Time (minutes)
0.2x
0.15x
0.1x

FIGURE 7

A

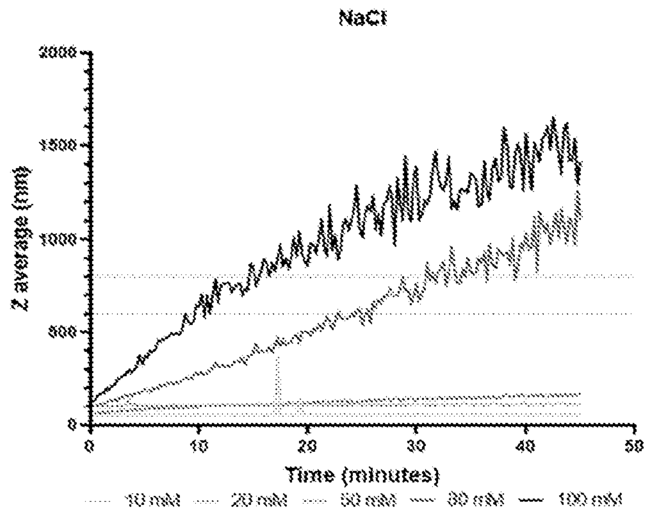
NaCl
Z average (nm)
2000
1500
1000
500
0
0
10
20
30
40
50
Time (minutes)
10 mM
20 mM
50 mM
80 mM
100 mM

B

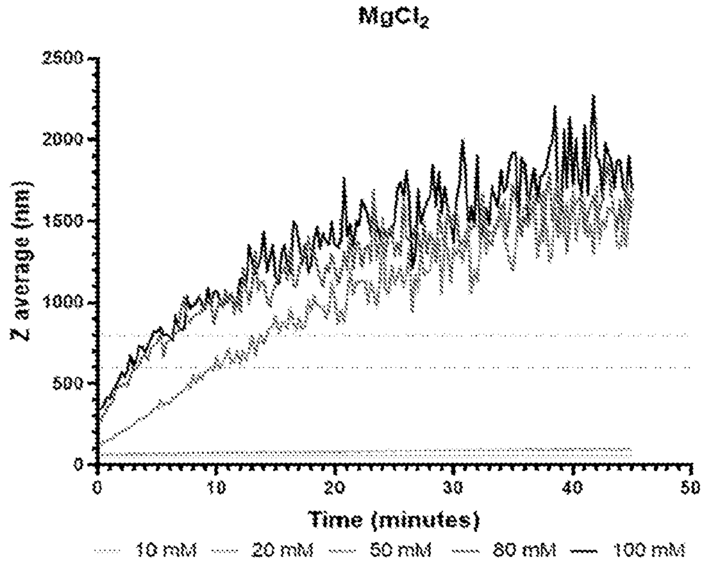
MgCl2
Z average (nm)
2500
2000
1500
1000
500
0
0
10
20
30
40
50
Time (minutes)
10 mM
20 mM
50 mM
80 mM
100 mM

FIGURE 8

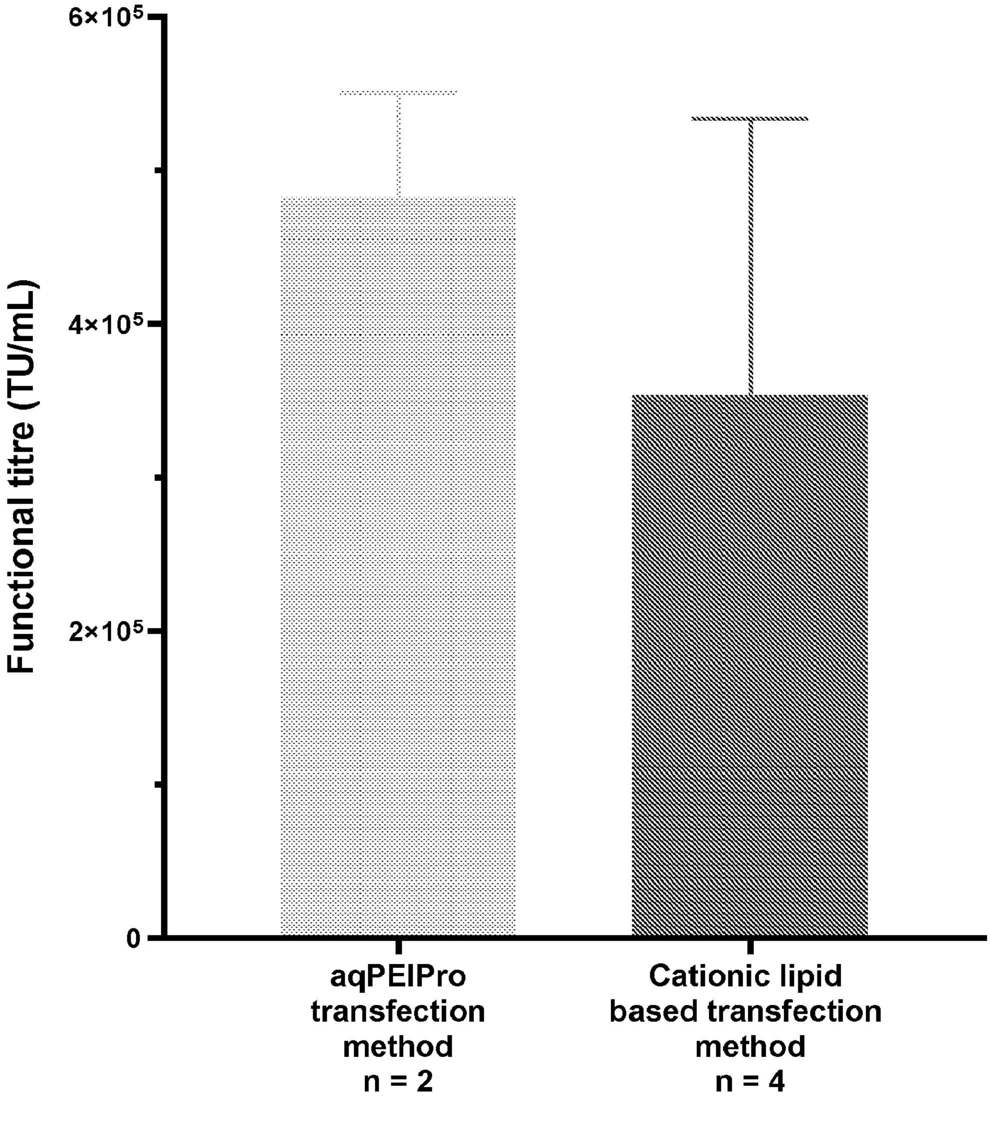
6×10⁵
4×10⁵
2×10⁵
0
Functional titre (TU/mL)
aqPEIPro transfection method n = 2
Cationic lipid based transfection method n = 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 3502260 A
- WO 2004022761 A
- WO 2009153563 A
- WO 2003064665 A
- WO 199817815 A
- WO 9932646 A
- WO 200179518 A
- US 6924123 B
- US 7056699 B
- WO 2006010834 A
- WO 2007071994 A
- WO 2007072056 A
- WO 2015092440 A
- WO 1994294440 A
- WO 200052188 A
- US 5952199 A
- US 6100071 A
- WO 199805635 A
- WO 199807859 A
- WO 199809985 A
- WO 199941397 A


### Non-patent literature cited in the description


- **MERTEN, O-W. et al.** *Pharmaceutical Bioprocessing,* 2014, vol. 2, 183-203
- **MERTEN, O-W et al.** *Pharmaceutical Bioprocessing,* 2014, vol. 2, 237-251
- **VAN DER LOO JCM ; WRIGHT JF.** *Human Molecular Genetics,* 2016, vol. 25 (R1), R42-R52
- **GRAHAM et al.** *Virology,* 1973, vol. 52, 456
- **SAMBROOK et al.** Molecular Cloning, a laboratory manual. Cold Spring Harbor Laboratories, 1989
- **DAVIS et al.** Basic Methods in Molecular Biology. Elsevier, 1986
- **CHU et al.** *Gene,* 1981, vol. 13, 197
- **DR WOLFGANG SCHÄRTL.** Basic Physical Chemistry: A Complete Introduction on Bachelor of Science Level. 2014, 109
- **ATKINS P. ; PAULA J.** Physical Chemistry. W.H.Freeman, 2006, 763
- Tissue Culture. Academic Press, 1973
- **R.I. FRESHNEY.** Culture of animal cells: A manual of basic technique. Wiley- Liss Inc, 2000
- **COFFIN et al.** Retroviruses. Cold Spring Harbour Laboratory Press, 1997, 758-763
- **LEWIS et al.** *EMBO J,* 1992, vol. 11 (8), 3053-3058
- **LEWIS ; EMERMAN.** *J Virol,* 1994, vol. 68 (1), 510-516
- **STEWART HJ ; FONG-WONG L ; STRICKLAND I ; CHIPCHASE D ; KELLEHER M ; STEVENSON L ; THOREE V ; MCCARTHY J ; RALPH GS ; MITROPHANOUS KA.** *Hum Gene Ther,* March 2011, vol. 22 (3), 357-69
- **STEWART, H. J ; M. A. LEROUX-CARLUCCI ; C. J. SION ; K. A. MITROPHANOUS ; P. A. RADCLIFFE.** *Gene Ther,* June 2009, vol. 16 (6), 805-14
- **DERSE ; NEWBOLD.** *Virology,* 1993, vol. 194 (2), 530-536
- **MAURY et al.** *Virology,* 1994, vol. 200 (2), 632-642
- **MARTARANO et al.** *J Virol,* 1994, vol. 68 (5), 3102-3111
- **YU et al.** *PNAS,* 1986, vol. 83, 3194-98
- **MARTY et al.** *Biochimie,* 1990, vol. 72, 885-7
- **NAVIAUX et al.** *J. Virol,* 1996, vol. 70, 5701-5
- **IWAKUMA et al.** *Virol.,* 1999, vol. 261
- **DEGLON et al.** *Human Gene Therapy,* 2000, vol. 11, 179-90
- **YAO F ; SVENSJO T ; WINKLER T ; LU M ; ERIKSSON C ; ERIKSSON E.** *Hum Gene Then,* 1998, vol. 9, 1939-1950
- **MAUNDER et al.** *Nat Commun,* 27 March 2017, vol. 8
- **VERMA ; SOMIA.** *Nature,* 1997, vol. 389 (6648), 239-242
- **VALSESIA-WITTMAN et al.** *J Virol,* 1996, vol. 70, 2056-64
- **NILSON et al.** *Gene Ther,* 1996, vol. 3 (4), 280-286
- **FIELDING et al.** *Blood,* 1998, vol. 91 (5), 1802-1809
- **EMI et al.** *Journal of Virology,* 1991, vol. 65, 1202-1207
- **ABE et al.** *J Virol,* 1998, vol. 72 (8), 6356-6361
- **BURNS et al.** *Proc. Natl. Acad. Sci. USA,* 1993, vol. 90, 8033-7

- **YEE et al.** *Proc. Natl. Acad. Sci. USA,* 1994, vol. 91, 9564-9568
- **AKKINA et al.** *J. Virol.,* 1996, vol. 70, 2581-5
- **KANG et al.** *J. Virol.,* 2002, vol. 76, 9378-9388
- **KUMAR M ; BRADOW BP ; ZIMMERBERG J.** *Hum Gene Ther,* 2003, vol. 14 (1), 67-77
- **KAYE et al.** *J Virol.,* October 1995, vol. 69 (10), 6588-92
- **KOO et al.** *Virology,* 1992, vol. 186, 669-675
- **CHEN et al.** *J. Virol,* 1993, vol. 67, 2142-2148
- **JANG et al.** *Enzyme,* 1990, vol. 44, 292-309
- **MOUNTFORD ; SMITH.** *TIG,* May 1995, vol. 11 (5), 179-184
- **GHATTAS, I.R. et al.** *Mol. Cell. Biol.,* 1991, vol. 11, 5848-5859
- **MACEJAK ; SARNOW.** *Nature,* 1991, vol. 353, 91
- **OH et al.** *Genes & Development,* 1992, vol. 6, 1643-1653
- **PELLETIER ; SONENBERG.** *Nature,* 1988, vol. 334, 320-325
- **MOUNTFORD ; SMITH.** *TIG,* 1985, vol. 11, 179-184
- **ADAM et al.** *J.Virol.,* 1991, vol. 65, 4985
- **YANG J.** *Corces VG,* 2011, vol. 110, 43-76
- **CHUNG J H ; WHITELY M ; FELSENFELD G.** *Cell,* 1993, vol. 74, 505-514
- **FARRELL CM1 ; WEST AG ; FELSENFELD G.** *Mol Cell Biol.,* June 2002, vol. 22 (11), 3820-31
- **J ELLIS et al.** *EMBO J.,* 01 February 1996, vol. 15 (3), 562-568
- **MORIARITY et al.** *Nucleic Acids Res.,* April 2013, vol. 41 (8), e92
- **DICKINS et al.** *Nature Genetics,* 2005, vol. 37, 1289-1295
- **SILVA et al.** *Nature Genetics,* 2005, vol. 37, 1281-1288
- **RALPH et al.** *Nature Medicine,* 2005, vol. 11, 429-433
- **STARK et al.** *Annu Rev Biochem,* 1998, vol. 67, 227-64
- **ELBASHIR et al.** *EMBO J.,* 03 December 2001, vol. 20 (23), 6877-88
- **HUTVAGNER et al.** *Science,* 12 July 2001, vol. 293 (5531), 834-8
- **DEVEREUX et al.** *Nucleic Acids Research,* 1984, vol. 12, 387
- **ATSCHUL et al.** *J. Mol. Biol.,* 1990, 403-410
- *FEMS Microbiol Lett,* 1999, vol. 174 (2), 247-50
- *FEMS Microbiol Lett,* 1999, vol. 177 (1), 187-8
- **OWENS, L.G ; SHEARD, V.E et al.** *J Immunother,* April 2018, vol. 41 (3), 130-140